Europäisches Patentamt

(19) European Patent Office

Office européen des brevets

(11) Publication number: **0 360 750 A2**

(12) **EUROPEAN PATENT APPLICATION**

(21) Application number: **89810685.1**

(22) Date of filing: **13.09.89**

(51) Int. Cl.⁵: **C 12 N 15/29**
C 12 N 15/60, C 12 N 9/88,
C 12 N 5/00

(30) Priority: **22.09.88 US 248000    06.01.89 US 294530**
**08.06.89 US 363663**

(43) Date of publication of application:
**28.03.90  Bulletin  90/13**

(84) Designated Contracting States:
**AT BE CH DE ES FR GB GR IT LI LU NL SE**

(71) Applicant: **CIBA-GEIGY AG**
**Klybeckstrasse 141**
**CH-4002 Basel  (CH)**

(72) Inventor: **Montoya, Alice**
**deceased  (US)**

**Jen, George**
**5704 Earl Road**
**Durham, N.C. 27712  (US)**

**Harms, Christian**
**1412 Gray Bluff Trail**
**Chapel Hill, N.C. 27519  (US)**

**Carswell, Gleta**
**215 West Park Street**
**Cary, N.C. 27511  (US)**

**Armour, Susan**
**4641 Hope Valley Road Apt. E**
**Durham, N.C. 27707  (US)**

**Volrath, Sandra**
**4225 Pine Oak Drive**
**Durham, N.C. 27707  (US)**

The applicant has filed a statement in accordance with Rule 28 (4) EPC (issue of a sample only to an expert). Accession number(s) of the deposit(s): ATCC 53487, 40326, 67420, 67628, 40426, 40529, 40530, 40531, 40532, 40533 and 40534.

(54) **Novel herbicide tolerant plants.**

(57)  Plant cells and plants are described which are tolerant to a herbicidal acetohydroxyacid synthase (AHAS) inhibitor, for example to a sulfonylurea, imidazolinone or triazolopyrimidine herbicide, wherein said tolerance is caused by an increased level of AHAS enzyme. Plant cells of the invention may be obtained by selection procedures or by genetic engineering. Further described are recombinant DNA useful in preparing said plant cells, methods of preparation of said plant cells, plants, seeds and other propagules and methods of controlling weed in a plantation consisting of plants of the invention.

FIG. 1

EP 0 360 750 A2

**Description**

# NOVEL HERBICIDE TOLERANT PLANTS

The present invention relates to plant cells and plants which are tolerant to a herbicidal acetohydroxyacid synthase (AHAS) inhibitor, wherein said tolerance is caused by an increased level of AHAS enzyme, to recombinant DNA useful for the preparation of said tolerant plant cells, and to methods of preparation of said plant cell by selection and bytransformation with recombinant DNA.

Acetohydroxyacid synthase (AHAS, E.C. 4.1.3.18, also referred to as acetolactate synthase) is an enzyme which plays a central role in the biosynthesis of branched chain amino acids, i.e. of valine, leucine and isoleucine. AHAS catalyzes the condensation of two molecules of pyruvate to yield acetolactate and also the condensation of pyruvate with ketobutyrate to yield acetohydroxybutyrate. The catalytic activity requires flavine adenine dinucleotide, thiamine pyrophosphate and a divalent cation. In enteric bacteria, three isozymes have been identified which differ in feedback inhibition by the amino acids formed. Some experimental evidence in plants suggests that plant AHAS may also consist of three or more isozymes. The observation that sulfonylurea herbicides, imidazolinone herbicides and triazolopyrimidine herbicides act by way of inhibition of AHAS has aroused considerable interest in this enzyme.

The discovery that resistance to sulfonylurea herbicides and to imidazolinone herbicides in some enteric bacteria and in yeast is due to altered AHAS has focused research in that direction. For example, in U.S. Patent 4,761,373 maize plants are described which were obtained by selection of plant cell cultures and which are resistant to otherwise herbicidal amounts of imidazolinone AHAS inhibitors. This resistance is conferred to the plants by an altered acetohydroxyacid synthase (AHAS). Deduced from extensive experience with altered AHAS in yeast, European Patent Application 257 993 discloses the nucleotide sequences of several AHAS coding genes and lists seven sub-sequences of these genes wherein nucleic acid changes at the gene level and hence amino acid changes at the enzyme level lead to AHAS resistant to the usual concentrations of sulfonylurea herbicides. This patent application also discloses ways of introducing altered AHAS genes into tobacco by genetic engineering.

Although the approach of selecting for altered AHAS or of introducing altered AHAS by genetic engineering has led to some success so far, it is still open whether this approach will lead to useful crops with inheritable sulfonylurea resistance and further desirable properties. It may be assumed that alterations of the AHAS enzyme in the long run will also alter the biosynthetic pathway leading to branched chain amino acids and hence to plants with unexpected and undesired properties. It is therefore an object of the present invention to describe ways and means to confer to plants tolerance to herbicidal AHAS inhibitors based on manipulations of normal wild-type plant AHAS, for example over-expression or amplification of wild-type AHAS gene.

The mechanism of over-expression of the target enzyme of an antibiotic has been detected in resistant microbes and is extensively documented for all kinds of microbial and mammalian cells. Reports of transient over-expression of enzymes in plants are also available. The International Patent Application WO 86/02097 discloses plant cells resistant to herbicidal glutamine synthetase inhibitors wherein the resistance is caused by increased levels of wild-type glutamine synthetase. However, due to the inherent differences between the glutamine biosynthesis pathway and the branched chain amino acid synthesis pathway, there is no indication whether the concept of over-expression can be applied to confer resistance or tolerance towards herbicidal AHAS inhibitors in plants. Furthermore, results of experiments described in European Patent Application 257 993 wherein the introduction of wild-type tobacco AHAS genes into herbicide sensitive tobacco plant cells failed to confer tolerance to sulfonyurea herbicides to these cells discourage the general application of the principle over-expression to herbicidal AHAS inhibitors.

The invention concerns plant cells which are tolerant to a herbicidal acetohydroxyacid synthase (AHAS) inhibitor, for example to a sulfonylurea, imidazolinone or triazolopyrimidine herbicide, wherein said tolerance is caused by an increased level of AHAS enzyme. Plant cells of the invention may be obtained by selection procedures or by genetic engineering. The invention further concerns plants, seeds and other propagules containing said plant cells, recombinant DNA useful in preparing said plant cells, methods of preparation of said plant cells, plants, seeds and other propagules and methods of controlling weed in a plantation consisting of plants of the invention.

## BRIEF DESCRIPTION OF THE FIGURES

Figure 1 shows a physical map of the 4.0 kbp XbaI insert of pCIB1253. The restriction endonuclease sites EcoRI, HincII, HindIII, NcoI, XbaI, and XhoI are shown. This fragment was cloned from DCIB1200 and shows hybridization to the SacII- BglII Arabidopsis AHAS gene fragment.

Figure 2 shows a physical map of the 7.5 kbp EcoRI insert of pCIB1255. The restriction endonuclease sites BamHI, EcoRI, HindIII, KpnI, PstI, and SalI are shown. This fragment was cloned from DCIB1202 and shows hybridization to the SacII-BglII Arabidopsis AHAS gene fragment.

Figure 3 shows the DNA sequence Clsqb-20.Seq obtained from the 2.1 kbp HindIII-EcoRI fragment of pCIB1253 using the "M13 minus20" primer, 5'GTAAAACGACGGCCAGT3'.

Figure 4 shows the DNA duplex sequence deduced from sequence Clsqb-20.Seq (Clsqb-20.Seq is the top strand), and the amino acid sequence deduced from one reading frame of the strand complementary to the Clsqb-20.Seq sequence. The polypeptide formed from this amino acid sequence should be read from right to left and bottom to top.

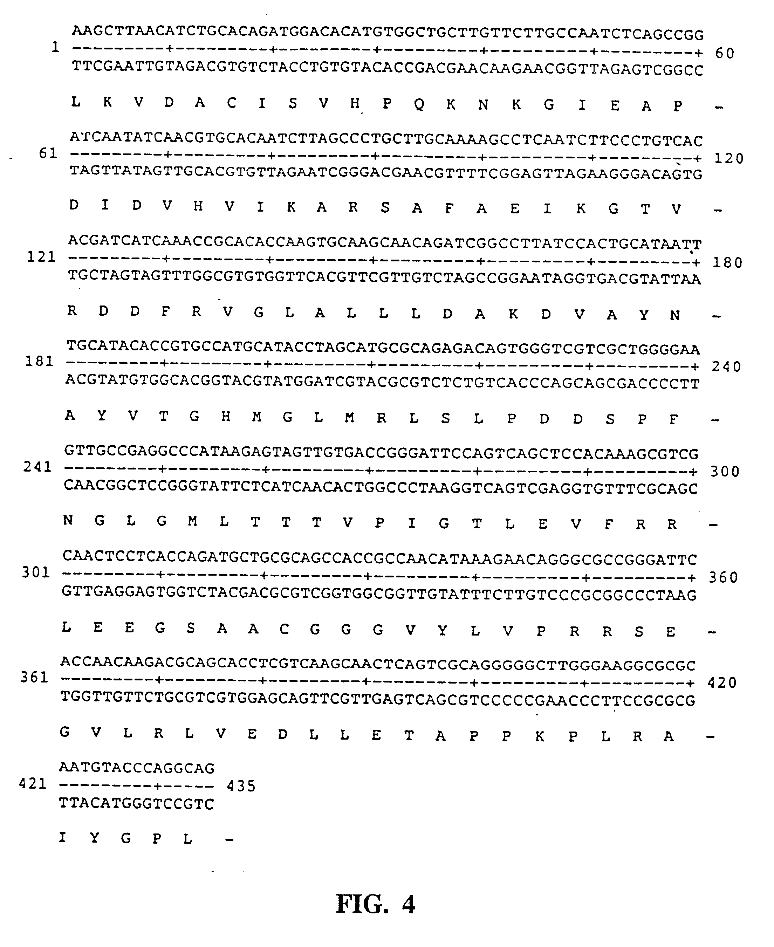

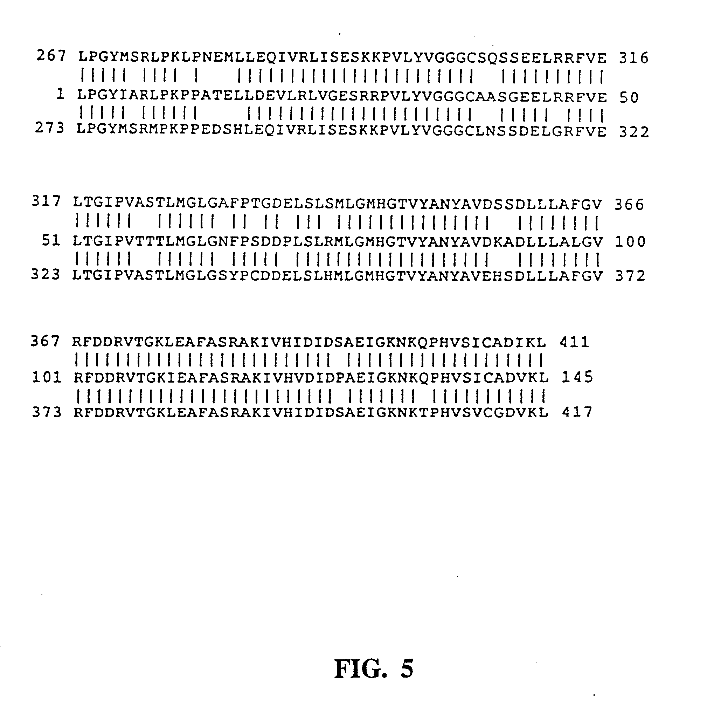

Figure 5 shows a similarity comparison between the polypeptide deduced from Clsqb-20.Seq shown in Figure 4 (middle sequence), the sequence of tobacco SuRB AHAS enzyme from amino acid positions 267 to 411 (top sequence), and the sequence of Arabidopsis AHAS enzyme from amino acid positions 273 to 417 (bottom sequence). The alignments were made by the GAP program of the University of Wisconsin Genetics Computer Group operating on a VAX computer (DEC, Boston, MA). A vertical line is drawn between the maize polypeptide and the tobacco and Arabidopsis sequence wherever they have identical amino acids at the same position in the alignments.

Figure 6 shows the DNA sequence C3sa16seq.Dat obtained from pCIB1255 using a primer made from nucleotide position 1166 to 1182 of the Arabidopsis AHAS gene. Position #1 is the A of the initiator ATG of the Arabidopsis AHAS coding sequence. The primer sequence is 5'TAAGATTGTTCATATTG3'.

Figure 7 shows the DNA duplex sequence deduced from the sequence C3sa16seq.Dat (C3sa16seq.Dat is the top strand), and the amino acid sequence deduced from one reading frame of the C3sa16.seq.Dat sequence. The polypeptide formed from this amino acid sequence should be read from left to right and top to bottom.

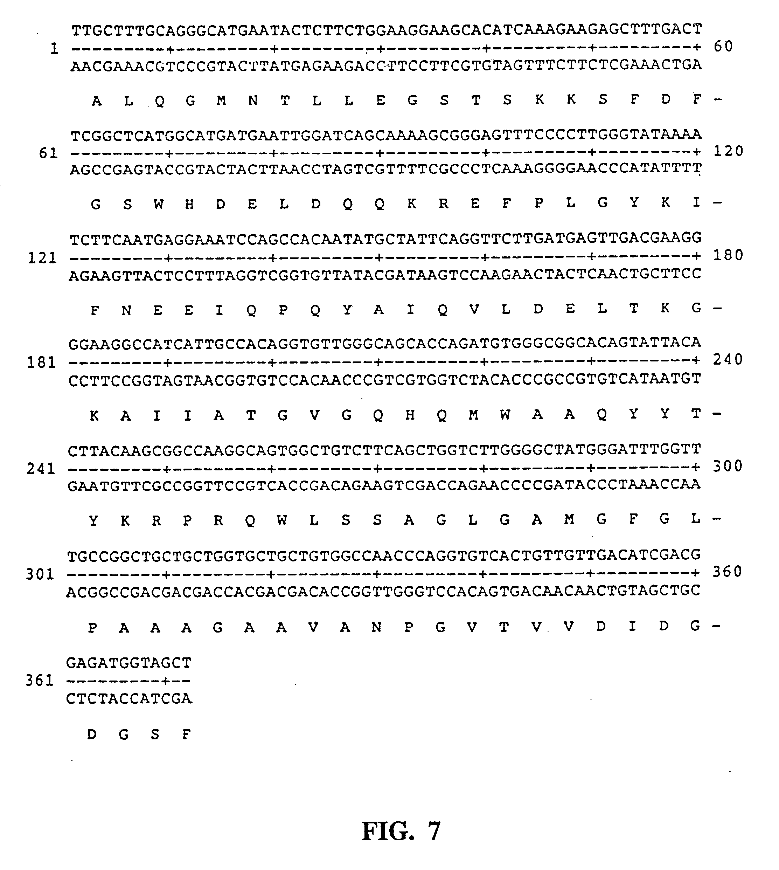

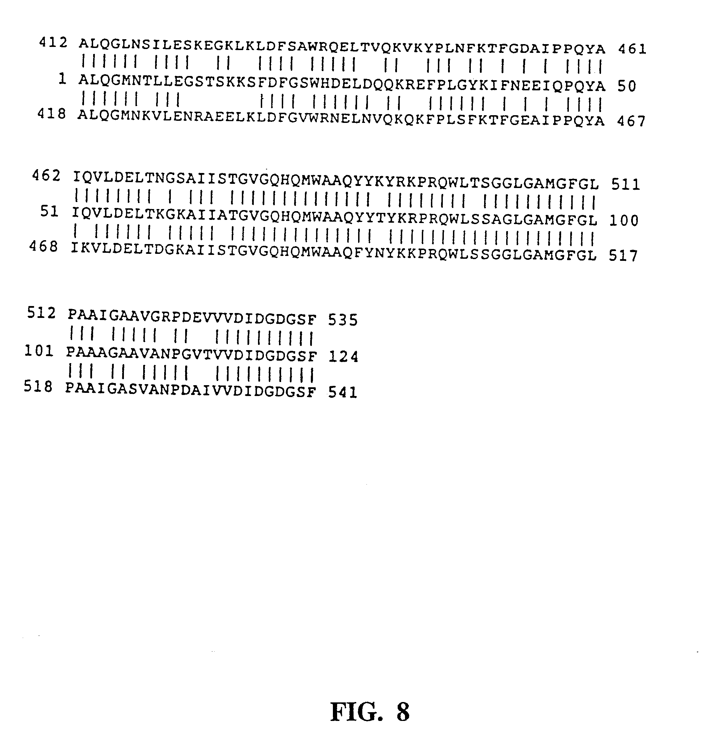

Figure 8 shows a similarity comparison between the polypeptide deduced from C3sa16seq.Dat shown in Figure 7 (middle sequence), the sequence of tobacco SuRB AHAS enzyme from amino acid positions 412 to 535 (top sequence), and the sequence of Arabidopsis AHAS enzyme from amino acid positions 418 to 541 (bottom sequence). The alignments were made by the GAP program of the University of Wisconsin Genetics Computer Group operating on a VAX computer (DEC, Boston, MA). A vertical line is drawn between the maize polypeptide and the tobacco and Arabidopsis AHAS sequence wherever they have identical amino acids at the same position in the alignments.

Figure 9 shows the DNA sequence of the complete maize C1 AHAS gene and flanking DNA. The deduced amino acid of maize C1 AHAS is depicted below the DNA sequence.

Figure 10 shows the DNA sequence of the complete maize C3 AHAS gene and flanking DNA. The deduced amino acid of maize C3 AHAS is depicted below the DNA sequence.

Figure 11 shows the genomic DNA sequence of the 2 kb pair fragment between the XhoI and BglII sites of the tobacco PR-1a gene. The arrow at about nucleotide 903 indicates the transcriptional start site, and the vertical arrows marked 207 and 313 identify the ends of two independent cDNA clones. The polypeptide sequence encoded by the coding portion of the gene is also disclosed.

## DEPOSITS AT THE AMERICAN TYPE CULTURE COLLECTION

| | |
|---|---|
| 53487 Agrobaceterium tumefaciens A281 | deposited May 14, 1986 |
| 40326 Zea mays suspension culture | May 20, 1987 |
| 67420 E. coli HB101 with pE16/8-c4 plasmid | May 29, 1987 |
| 67628 E. coli HB101 with pTJS75-Km | Feb. 12, 1988 |
| 40426 Plasmid pBS-PR1013Cla | Feb. 12, 1988 |
| 40529 Plasmid DNA pCIB 1253 | Dec. 29, 1988 |
| 40530 Phage lambda DNA DCIB 1200 | Dec. 29, 1988 |
| 40531 Lambda phage LCIB 1200 | Dec. 29. 1988 |
| 40532 Plasmid DNA pClb 1255 | Dec. 29, 1988 |
| 40533 Phage lambda DNA DCIB 1202 | Dec. 29, 1988 |
| 40534 Lambda phage LCIB 1202 | Dec. 29, 1988 |

Plant cells

The invention concerns plant cells which are tolerant to a herbicidal acetohydroxyacid synthase (AHAS) inhibitor, wherein said tolerance is caused by an increased level of AHAS enzyme.

Tolerant means that the said plant cells survive at a concentration of said herbicidal AHAS inhibitor which suffices to kill at least 95 %, for example 99 %, 99.9 % or more, of corresponding plant cells of the same genus and strain with a normal level of AHAS enzyme. An increased level of AHAS enzyme is a level which is substantially higher than the average level of AHAS enzyme in corresponding plant cells of the same genus and strain with normal level of AHAS enzyme, for example a twofold or higher level, such as a tenfold level.

The increased AHAS level in the plant cells of the invention may be due to an increased amount of the same AHAS enzyme as is present in corresponding normal, i.e. wild-type, cells of the same genus and strain, but it may also be due to an increased amount of an AHAS enzyme derived from wild-type cells of another strain or of another genus, or to an increased amount of a mutated AHAS enzyme derived from natural enzyme by mutation with suitable chemicals or high energy irradiation, or by chemical synthesis. Sources of natural or mutated enzymes may be any plant, but also microbes such as bacteria and yeast. An AHAS enzyme from a wild-type cell is usually sensitive to a herbicidal AHAS inhibitor mentioned below, but may show variable degree of tolerance to such a herbicide.

Plant cells of the invention displaying increased levels of AHAS enzyme are obtained by selection processes or by genetic engineering. The preferred selection processes are those used with any kind of plant cell cultures from which whole plants can be regenerated, such as those described in more detail below, for example single cell, plant cell cultures, embryogenic plant tissue cell cultures, protoplast cultures, callus cultures, or the like. Selection procedures are also appli cable to whole plants or seeds, but these are much less effective. The preferred selection procedures may be applied to cells that have undergone treatment which favors mutation processes, such as treatment with mutagenic chemicals, for example N-ethyl-N-nitroso-urea (NEU), N-methyl-N'-nitro-N-nitroso-guanidine (MNNG), ethyl methane-sulfonate (EMS), or sodium azide (NaN$_3$), or treatment with high energy irradiation, for example short wave ultraviolet (UV) light, gamma or X-rays. However, the natural frequency of mutation usually suffices to produce plant cells with the desired properties which then can be selected with procedures of the invention.

The increased levels of AHAS enzyme in plant cells of the invention obtained by selection procedures may be due to different causes, for example, to multiple copies of the corresponding endogenous AHAS gene or multiple occurrence of the AHAS coding sequence within the corresponding AHAS gene, i.e. gene amplification, or to increased expression of an endogenous AHAS coding sequence as a consequence of a mutation in the non-coding regulatory sequence of the endogenous AHAS gene, i.e. over-expression.

Several different approaches may be used with genetic engineering techniques to obtain increased levels of AHAS enzyme. Genetic engineering techniques may be used to effect the results obtainable by selection procedures as detailed above. For example, multiple copies of an endogenous AHAS gene may be introduced and stably integrated into the genome of the plant cells of the invention, or an AHAS gene may be constructed and introduced in the genome wherein multiple copies of the endogenous AHAS coding sequence are under control of the endogenous promoter in the correct reading frame. Furthermore, an endogenous AHAS gene may be manipulated in the non-coding regulatory region by introducing, replacing or excising base sequences in order to increase expression of the endogenous AHAS coding sequences, or the endogenous AHAS coding sequences may be put under the control of a different endogenous promoter known to provide high levels of expression.

Genetic engineering techniques can also be used according to the invention to prepare plant cells with increased levels of AHAS enzyme by introducing exogenous AHAS genes, exogenous AHAS coding sequences and/or exogenous promoters and enhancers. The said exogenous AHAS gene may code for an AHAS of a different strain or genus that essentially has the properties of a wild-type AHAS. An example of such an exogenous AHAS gene is a gene from a plant which is sensitive to a herbicidal AHAS inhibitor. In spite of this the plant cell containing an exogenous gene coding for a sensitive AHAS may be tolerant to a herbicidal AHAS inhibitor because of increased expression of that gene in the different surroundings. In a further aspect of the invention, the plant cells contain stably incorporated chimeric DNA constructs comprising a DNA sequence coding for an AHAS functional in said plant cell operably linked to a promoter providing increased expression of said AHAS. The nature of the different exogenous promoters to be used will be described in more detail below. The AHAS coding sequence operably linked may be the endogenous AHAS coding sequence or an AHAS coding sequence of another strain or genus, in particular a wild-type coding sequence which is derived from a plant cell sensitive to a herbicidal AHAS inhibitor. Such DNA constructs may contain further coding sequences, for example a coding sequence for a transit peptide which leads to a fusion protein which is processed by the plant cell of the invention in such a manner that the AHAS enzymes are transported into the usual site of occurrence and action, for example into the chloroplasts or mitochondria.

The plant cells of the invention may be cells of a monocotyledonous or dicotyledonous plant. Any plant is considered, in particular crop plants of economic interest, for example plants grown for human or animal nutrition, plants grown for their content of useful secondary metabolites, plants grown for their content of fibers, trees, plants of ornamental interest, and the like. Examples, which do not imply any limitation as to the scope of the present invention, are corn (maize), wheat, barley, rice, sorghum, sugarcane, sugarbeet, soybean, Brassica, sunflower, carrot, tobacco, lettuce, cucumber, tomato, potato, cotton, Arabidopsis, Lolium, Festuca, Dactylis, or poplar.

In particular, the invention concerns plant cells wherein the tolerance to a herbicidal AHAS inhibitor is caused by an increased level of AHAS enzyme and the said AHAS essentially has the properties of a wild-type AHAS. Preferably, the said AHAS is derived from a plant cell that is sensitive to the herbicidal AHAS inhibitors mentioned below.

Preferred are plant cells wherein said increased level of AHAS enzyme is at least twofold, in particular at least tenfold, higher than the level of AHAS enzyme in otherwise identical, naturally occurring plant cells sensitive to herbicidal AHAS inhibitors.

Further preferred are plant cells wherein the increased level of AHAS enzyme is due to increased expression of an endogenous AHAS coding sequence, such as caused by a mutation in the non-coding sequence of an endogenous AHAS gene, or to multiple copies of an endogenous AHAS gene.

In another preferred aspect of the invention the plant cells display an increased level of AHAS enzyme due to an exogenous AHAS gene, preferably due to an exogenous AHAS gene which codes for an AHAS which essentially has the properties of a wild-type AHAS, for example due to an exogenous AHAS gene derived from a plant cell that is sensitive to a herbicidal AHAS inhibitor. In particular, such preferred plant cells have stably incorporated a chimeric DNA construct comprising a DNA sequence coding for an AHAS functional in said plant cell operably linked to a promoter providing increased expression of said AHAS, or a chimeric DNA construct comprising a DNA sequence coding for an AHAS functional in said plant cell and a DNA sequence coding for a transit peptide, and the said coding sequences being operably linked to a promoter providing increased expression of said AHAS. A preferred promoter is a chemically regulatable promoter.

The preferred plant cells are corn (maize), wheat, barley, rice, sorghum, sugarcane, sugarbeet, soybean, Brassica, sunflower, carrot, tobacco, lettuce, cucumber, tomato, potato, cotton, Arabidopsis, Lolium, Festuca, Dactylis, or poplar cells, in particular corn, wheat, soybean and tobacco cells.

## Herbicidal AHAS inhibitors

Herbicidal AHAS inhibitors are known in the art. In particular, they belong to the classes of sulfonylurea herbicides, imidazolinone herbicides or triazolopyrimidine herbicides.

Sulfonylurea herbicides are, for example, ortho-substituted N-arylsulfonyl-N'-pyrimidinyl- or triazinyl-ureas and derivatives thereof, for example 2-chloro-N-[(4-methoxy-6-methyl-1,3,5-triazin-2-yl)-aminocarbonyl]-benzenesulfonamide (chlorsulfuron), 2-methoxycarbonyl-N-[(4,6-dimethylpyrimidin-2-yl)-aminocarbonyl]-benezensulfonamide (sulfometuronmethyl), 2-methoxycarbonyl-N-[(4-chloro-6-methoxypyrimidin-2-yl)-aminocarbonyl]-benzenesulfonamide, methyl 2-{N-[(4,6-dimethoxy-pyrimidin-2-yl)-aminocarbonyl]aminosulfonylmethyl}-benzoate, 2-(2-chloroethoxy)-N-[(4-methoxy-6-methyl-1,3,5-triazin-2-yl)-aminocarbonyl]benzenesulfonamide (triasulfuron), 2-methoxycarbonyl-N-[(4-methoxy-6-methyl-1,3,5-triazin-2-yl)-aminocarbonyl]-3-thiophene sulfonamide (thiameturon methyl), 2-methoxycarbonyl-N-[(4,6-bis-(difluoromethoxy}-pyrimidin-2-yl)-amino-carbonyl]-benzenesulfonamide, or 2-(2-methoxyethoxy)-N-[4,6-dimethoxy-1,3,5-triazin-2-yl)-aminocarbonyl]-benzenesulfonamide. The preferred sulfonylurea herbicides are N-o-methoxy-or ethoxycarbonylphenylsulfonyl-N'-(difluoromethoxy substituted pyrimidinyl)-ureas such as 2-methoxycarbonyl-N-[(4,6-bis{difluoromethoxy}-pyrimidin-2-yl)-aminocarbonyl]benzenesulfonamide called SU872, and N-o-(2-methoxy- or 2-ethoxy-ethoxy)-phenylsulfonyl-N'-(methyl and/or methoxy substituted triazinyl)-ureas such as 2-(2-methoxyethoxy)-N-[(4,6-dimethoxy-1,3,5-triazin-2-yl)-aminocarbonyl]benzenesulfonamide called SU464.

Imidazolinone herbicides are, for example, 2-(2-imidazolinyl)-pyridines or -quinolines and derivatives thereof, for example 2-(5-isopropyl-5-methyl-4-oxo-2-imidazolinyl)nicotinic acid (imazapyr), 2-(5-isorpopyl-5-methyl-4-oxo-2-imidzaolinyl)-3-quinolinecarboxylic acid (imazaquin), or 5-ethyl-2-(5-isopropyl-5-methyl-4-oxo-2-imidazolinyl)nicotinic acid (imazalin), and acid addition salts thereof.

A triazolopyrimidine herbicide is for example N-(2,6-dichlorophenyl)-5,7-dimethyl-1,2,4-triazolo[1,5-a]-pyrimidine-2-sulfonamide or N-(2-methyl-6-nitrophenyl)-5,7-dimethyl-1,2,4-triazolo[1,5-a] pyrimidine-2-sulfonamide.

Preferred are plant cells which are tolerant to a sulfonyl urea herbicide, in particular to a N-o-methoxy- or ethoxycarbonylphenylsulfonyl-N'-(difluoromethoxy substituted pyrimdinyl)-urea herbicide, for example to SU872, to a N-o-(2-methoxy- or 2-ethoxy-ethoxy)-phenylsulfonyl-N'-(methyl and/or methoxy substituted triazinyl)-urea herbicide, for example to SU464, or to a N-o-(2-chloroethoxy)-phenylsulfonyl-N'-(methyl and/or methoxy substituted triazinyl)-urea herbicide, for example to triasulfuron.

Plant cells tolerant to an imidazolinone herbicide or to a triazolopyrimidine herbicide are likewise preferred.

## Plants, seeds and other propagules

The invention further concerns plants, seeds and other proagules which are tolerant to a herbicidal acetohydroxyacid synthase (AHAS) inhibitor. In particular the invention concerns plants, seeds and other propagules which contain plant cells tolerant to a herbicidal acetohydroxyacid synthase (AHAS) inhibitor, wherein said tolerance is caused by an increased level of AHAS enzyme in said plant cells.

Plant means any kind of organized and differentiated form of a plant, including plantlets, seedlings, plant embryos and like forms, also part of a plant, for example leaves, roots, stems, flowers, flower buds, fruits and the like. Propagules comprise generative or vegetative plant organs or tissues which can be used to propagate the plant, for example seeds, tubers, stem or root cuttings, buds and the like. Propagules should also be understood to mean any cells or tissues which can be propagated in vitro and from which plants can be regenerated by in vitro culture and micropropagation methods.

The plants, seeds and other propagules of the invention contain plant cells tolerant to a herbicidal AHAS inhibitor as defined above. The plants, seeds and other propagules may consist completely of the mentioned tolerant plant cells or just a limited number of such plant cells which suffices to confer to the plant, seed or other propagules a tolerance towards herbicidal AHAS inhibitor at one time or other. Because plants, seeds and other propagules are highly differentiated and consist of plant cells with different performances and properties, it is by no means required that all these cells be tolerant to herbicidal AHAS inhibitors in order to confer herbicide tolerance to the whole plant, seed or other propagule.

The plants of the invention may be monocotyledonous or dictoyledonous. Any plant is considered, in particular crop plants of economic interest, for example plants grown for human or animal nutrition, plants grown for their content of useful secondary metabolites, plants grown for their content of fibers, trees, plants of ornamental interest, and the like. Examples, which do not imply any limitation as to the scope of the present invention, are corn (maize), wheat, barley, rice, sorghum, sugarcane, sugarbeet, soybean, Brassica, sunflower, carrot, tobacco, lettuce, cucumber, tomato, potato, cotton, Arabidopsis, Lolium, Festuca, Dactylis, or poplar.

The preferred plants, seeds and other propagules of the invention are those containing preferred plant cells as defined above.

In particular, the invention concerns plants, seeds and other propagules containing plant cells wherein the tolerance to a herbicidal AHAS inhibitor is caused by an increased level of AHAS enzyme, and the said AHAS essentially has the properties of a wild-type AHAS, for example an AHAS derived from a plant cell which is sensitive to the herbicidal AHAS inhibitors mentioned above. The increased level of AHAS enzyme in the plant cells is preferably at least twofold, in particular at least tenfold. Further preferred are plants, seeds and other propagules containing plant cells wherein the increased level of AHAS enzyme is due to increased expression of an endogenous AHAS coding sqeuence, to multiple copies of an endogenous AHAS gene, or to an exogenous AHAS gene, preferably to an exogenous AHAS gene which codes for an AHAS which essentially has the properties of a wild-type AHAS. In particular, such preferred plants, seeds or other propagules contain plant cells having stably incorporated a chimeric DNA construct comprising a DNA sequence coding for an AHAS functional in said plant cell operably linked to a promoter providing increased expression of said AHAS, or a chimeric DNA construct comprising a DNA sequence coding for an AHAS functional in said plant cell and a DNA sequence coding for a transit peptide, said transit peptide coding sequences being operably linked to a promoter providing increased expression of said AHAS. A preferred promoter is a chemically regulatable promoter.

## DNA constructs

The invention further concerns recombinant DNA useful in the preparation of plant cells as defined above by genetic engineering. In particular, the ivention concerns chimeric DNA constructs comprising a DNA sequence coding for a wild-type AHAS operably linked to a promoter, wherein said promoter is different from the promoter linked to said AHAS in the wild-type gene.

Recombinant DNA is DNA excised from a naturally occurring gene or genome, or DNA of a chimeric DNA construct as defined below, isolated from its natural surroundings, introduced into a suitable cloning vector, and multiplied in a host. Techniques of isolation, suitable cloning vectors, and hosts will be described below in more detail.

A chimeric DNA construct is a DNA sequence composed of different DNA fragments not naturally occurring in this combination. The DNA fragments combined in the chimeric DNA construct may originate from the same species or from different species. For example, a DNA fragment coding for an AHAS may be operably linked to a DNA fragment representing a promoter from another gene of the same species that provides increased expression of the AHAS coding sequence. Preferably, however, a DNA fragment coding for an AHAS is operably linked to a DNA fragment containing a promoter from another species, for example, from another plant species, from a fungus, yeast, or from a plant virus, or a synthetically produced promoter. A synthetically produced promoter is either a promoter synthesized chemically from nucleotides de novo or a hybrid promoter spliced together by combining two or more nucleotide sequences from synthetic or natural promoters which are not present in this combined form in any organism. The promoter has to be functional in the plant cell to be tranformed with the chimeric DNA construct.

The promoter derived from the same or from a different speices, instead of being down-regulated by branched chain amino acids as may be the case with the wild-type AHAS gene promoter, may provide for constant expression of AHAS or may be regulated, in particular positively regulated, by internal or external factors other than those provided by the branched chain amino acid biosynthesis cycle. External factors for the regulation of promoters are, for example, light, heat, or chemicals, for example inorganic salts such as chlorides, nitrites, nitrates, or ammonium salts, in particular sodium or ammonium nitrite, heavy metals, or organic compounds, for example organic acids, in particular salicylic acid, acetylsalicyclic acid, benzoic acid, naphthoic acids, phenyl- or naphthalene-acetic acids, benzothiadiazolecarboxylic acids or other aromatic and heteroaromatic acids, or polyacrylic acid, and derivatives of the mentioned acids, in particular salts, for example alkali, earth alkali or ammonium salts, and esters, for example lower alkyl esters such as methyl and ethyl esters.

Examples of promoters to be included in chimeric DNA constructs are cauliflower mosaic virus (CaMV) 195 or 35S promoters and double promoters, nopaline synthase promoters, pathogenesis-related (PR) protein promoters, small subunit of ribulose bisphosphonate carboxylase (ssuRUBISCO) promoters, and the like.

The chimeric DNA construct of the invention may contain multiple copies of a promoter and/or multiple copies of the DNA coding sequences. In addition the construct may include coding sequences for markers and coding sequences for other peptides such as signal or transit peptides.

Useful markers are peptides providing antibiotic or drug resistance, for example resistance to hygromycin, kanamycin, G418, gentamycin, lincomycin, methotrexate, glyphosate, or the like. These markers can be used to select cells transformed with the chimeric DNA constructs of the invention from untransformed cells. Other useful markers are peptidic enzymes which can be easily detected by a visible reaction, for example a color reaction, for example luciferase, β-1,3-glucuronidase, or β-galactosidase.

Signal or transit peptides provide the AHAS formed on expression of the chimeric DNA constructs of the invention with the ability to be transported to the desired site of action. Examples for transit peptides of the invention are chloroplast transit peptide or mitochondria transit peptide.

The AHAS coding sequence in the chimeric DNa constructs of the invention may be derived from a plant, for example from tobacco, corn (maize) or Arabidopsis, or from a microorganism, for example from E. coli or yeast. Such a DNA sequence may code for an AHAS which is tolerant or sensitive to a herbicidal AHAS inhibitor as described above. Preferably, however, the DNA sequence codes for a sensitive, wild-type AHAS. In chimeric DNA constructs containing coding sequences for transit peptides, these sequences are usually derived from a plant, for example from tobacco, corn or Arabidopsis. Preferably, transit peptide and AHAS coding sequences are derived from the same plant.

In the preferred chimeric DNA constructs of the invention the promoter is functional in plant cells. In particular such a chimeric DNA construct comprises a DNA sequence coding for a wild-type AHAS and a DNA sequence coding for a transit peptide operably linked to a promoter, wherein said promoter is different from the promoter linked to said AHAS in the wild-type gene, but functional in plant cells.

Particularly preferred are chimeric DNA constructs wherein the promoter is regulatable, for example regulatable by heat, light or chemicals, for example a pathogenesis related (PR) protein promoter. Further preferred chimeric DNA constructs comprise a cauliflower mosaic virus (CaMV) 35S promoter.

Preferred chimeric DNA constructs are also constructs containing a coding sequence for a wild-type AHAS derived from tobacco or from Arabidopsis which is sensitive to a herbicidal AHAS inhibitor as described above. Likewise preferred are constructs containing a coding sequence for a wild-type AHAS derived from corn (maize).

Most preferred are chimeric DNA constructs comprising a DNA sequence coding for a wild-type AHAS and a DNA sequence coding for a chloroplast transit peptide, for example constructs wherein said DNA sequences are derived from tobacco or from corn (maize) which is sensitive to a herbicidal AHAS inhibitor, and chimeric DNA constructs comprising a DNA sequence coding for a selectable marker, for example coding for hygromycin resistance.

In another embodiment the invention concerns a recombinant DNA comprising multiple copies of a wild-type AHAS coding sequence, for example multiple copies of a wild-type AHAS gene. The AHAS coding sequence may be derived from a microorganism, for example from E. coli or yeast, or from a plant, for example from any plant metnioned above, such as Arabidopsis, tobacco, or corn (maize). The recombinant DNA may code for an AHAS which is sensitive to a herbicidal AHAS inhibitor or for a wild-type AHAS which shows some degree of tolerance towards the herbicidal AHAS inhibitors of the invention.

The recombinant DNA of the invention, for example recombinant DNA comprising multiple copies of a wild-type gene or recombinant DNA containing chimeric DNA constructs as described above, may be in the form of a cloning vector, for example a plasmid, a phage DNA, a cosmid, or other DNA able to replicate in a host cell. Such a cloning vector usually contains a single or a small number of recognition sites for restriction endonucleases which allows one to cut the vector at determined site(s) in orderto handle the coding sequences and other DNA-encoded information in a predictable manner. In one aspect of the invention the recombinant DNA is a vector for the transformation of plant cells, which may or may not comprise an origin of replication functional in a microorganism, for example in E. coli and/or in Agrobacterium. The recombinant DNA may also contain a plant selectable marker, for example a coding sequence for kanamycin, hygromycin, G418, gentamycin, lincomycin, or methotrexate resistance.

Preferred is a recombinant DNA comprising multiple copies or a wild-type AHAS coding sequence, such as a gene, for example derived from tobacco or corn (maize) which is sensitive to a herbicidal AHAS inhibitor. Further preferred is a recombinant DNA comprising a DNA sequence coding for a plant selectable marker, for example coding for hygromycin resistance.

Particularly preferred is a recombinant DNA comprising a chimeric DNA construct as described above which is a vector for the transformation of plant cells, optionally containing an origin of replication functional in a microorganism, for example functional in E. coli and/or Agrobacterium.

The invention further concerns recombinant DNA comprising a DNA sequence coding for a wild-type AHAS from corn, Zea mays.

Also comprised by the present invention are processes for producing a chimeric DNA construct capable of conferring tolerance to a herbicidal acetohydroxyacid synthase (AHAS) inhibitor to a plant, which process comprises the following steps:

    (a) isolating a first DNA component sequence coding for a wild-type AHAS from its natural source or from a genomic or cDNA library; and

    (b) operably linking the DNA component sequence of step (a) to a promoter which is different from the promoter linked to said AHAS in the wild-type gene but is functional in plants, by ligating them concurrently or consecutively.

The instant invention further relates to a process for producing a chimeric DNA construct capable of conferring tolerance to a herbicidal acetohydroxyacid synthase (AHAS) inhibitor to a plant, which process

7

comprises the following steps:

(a) isolating a first DNA component sequence coding for a wild-type AHAS from its natural source or from a genomic or cDNA library;

(b) isolating a second DNA component sequence conding for a transit peptide;

(c) ligating the component sequences of step (a) and (b)

(d) operably linking the resulting DNA sequence of step (c) to a promoter which is different from the promoter linkedto said AHAS in the wild-type gene but is functional in plants, by ligating them concurrently or consecutively.

## Selection of herbicide tolerant plant cells in cell culture

A further aspect of the invention is a method of preparation of a plant cell as described above wherein

(a) wild-type plant cells are cultured in the presence of increasing amounts of a herbicidal AHAS inhibitor,

(b) surviving plant cells from step (a) are subcultured in the presence of said herbicidal AHAS inhibitor,

(c) the plant cells obtained in step (b) are analyzed for the presence of AHAS having essentially wild-type AHAS characteristics and for increased level of AHAS enzyme, and

(d) the plant cells having the desired properties mentioned in step (c) are selected and cultured.

Cells may be cultured in suspension culture or in solid media. The culture media are media known in the art and usually contain mineral salts, buffers, vitamins, sugars, and, if desired, further growth regulating compounds, for example hormones, or additional nutritients, such as amino acids. Preferred media are those mentioned in the EXAMPLES, for example those described by T. Murashige et al., Physiol. Plant. 15: 473-497 (1962), K.N. Kao et al., Planta 126: 105-110 (1975) and C-C. Chu et al., Scientia Sinica 18: 659 (1975).

As an example, for a suspension culture 25-50 ml medium are used with 100 ml Erlenmeyer flasks, or 50-100 ml medium with 250 ml flasks. Flasks are inoculated with 1-25 ml of a cell culture previously grown for 1-10 days. Cells are incubated at 20-30°C either in the dark or in the light, preferably on an orbital shaker at 50-200 rpm (2-5 cm throw). Cells are subcultured at 3-10 day intervals (usually 7 days) by inoculating an aliquot sample into fresh medium, by decanting or pipetting off between 50 and 95 % of the cell suspension followed by replenishing fresh medium to give the desired volume of suspension.

The culturing conditions have to be adapted to the particular plant genus and species. For example, methods for the production of embryogenic tissue cultures of corn (maize) are known in the art and can be used accordingly. However, it has been found that different corn genotypes differ widely in their culture response and in their specific culture requirements. Therefore it is necessary to determine the conditions for effective culturing for each corn genotype. This is particularly true in the case of elite corn genotypes, such as elite corn inbred lines.

For example, embryogenic callus is produced from corn scutellum tissue by harvesting cobs from self pollinated plants about 10-16 days post pollination containing embryos about 1.5-2.5 mm long, removing the embryos from the kernels and placing them, embryo axis downwards, onto solidified culture medium. Either field grown or greenhouse grown plants are used. The medium may be one of several known in the art suited for the induction of embryogenic callus, for example MS (T. Murashige et al., supra), R.U. Schenk et al., Canad. J. Botany 50: 199-204, 1972), KM (K.N. Kao et al., supra), N6 (C-C. Chu et al., in: Proceedings of Symposium on Plant Tissue Culture, pp. 43-50, Science Press, Beijing, 1978), D (Duncan et al., Planta 165: 322-332, 1985) or others known in the art, or modifications thereof, may be used. The medium contains one or more auxin-type growth regulators, for example 2,4-dichlorophenoxyacetic acid (2,4-D), 3,6-dichloro-2-methoxybenzoic acid (dicamba), 4-amino-3,5,6-trichloro-2-pyridinecarboxylic acid (picloram), p-chlorophenoxyacetic acid (pCPA), 2,4,5-trichlorophenoxyacetic acid (2,4,5-T), 3-amino-2,5-dichlorobenzoic acid (chloramben), or 2-methyl-4-chlorophenoxyacetic acid (MCPA) in a concentration suitable to induce embryogenic callus, for example 0.01 to 10 mg/l, preferably about 2 mg/l. The preferred growth regulator depends on the genotype used. For elite inbreds 211D, 8N810, 2717, 5N984 (Funk Seeds International), Greensboro, NC) the preferred growth regulator is 2,4-D. For elite inbred 273Y (Funk Seeds International) the preferred growth regulator is PCPA. For elite inbred 274 the preferred growth regulator is dicamba. The medium is solidified with a suitable gelling agent, for example Noble agar, Difco Bacto agar, Difco purified agar, Phytagar or Gelrite®. The carbohydrate concentration of the medium may be in the range of 2 to 12 % depending upon the requirements of the genotype. The preferred carbohydrate is sucrose. The amount and ratio of reduced vs. oxidized nitrogen may be altered depending upon the genotype used. In addition to major and minor salts, Fe-EDTA and vitamins, the medium may contain other promotive supplements, such as, for example, amino acids, for example casamino acids (100-200 mg/l) or proline (10-150 mM) to promote embryogenic callus formation. Such supplements and modifications are used as required for each genotype cultured.

For the preparation of a corn suspension culture, immature ears containing embryos of Zea mays plants, for example of self-pollinated plants of the inbred line Funk 2717 (Funk Seeds International), are sterilized and plated on a suitable initiation medium known in the art for the formation of callus. Callus may be subcultured on a different type of medium of induce formation of the desired type of callus which is identified by its characteristic morphology, i.e. a non-mucilaginous, granular and very friable type of callus which is separated into small individual cell aggregates upon placing into liquid medium. Such callus is subcultured for extended periods of time, usually 3 months or more, for example around 6 months. After subculture in suspension, preferably at around room temperature, for example 26°C, in the dark on a gyratory shaker or a similar device, only those cultures are retained which do not show large numbers of expanded cells. The preferred tissue consists of densely cytoplasmic dividing cell aggregates which have a characteristically smoother surface than the usual type of cell aggregates and have a rapid growth rate, with a doubling time of less than one week. The lines are maintained by always choosing for subculture those cells which exhibit the best morphology. Such a preferred Zea mays suspension culture is deposited with the American Type Culture Collection, see above.

Other culturing conditions are known in the art and may be used accordingly.

In order to obtain cells tolerant to otherwise lethal concen trations of a herbicidal AHAS inhibitor cells are cultured in the presence of such inhibitor sufficient to inhibit growth of most of the sensitive wild-type cells.

Usually cultured cells are diluted with fresh culture medium to give a cell density suitable for cell plating. The culture medium used for dilution may contain the inhibitor in the desired concentration. For plating, cells in a small volume of medium are evenly spread on top of solidified culture medium containing a suitable concentration of the inhibitor. The suitable inhibitor concentration is determined from a dose-response curve and is at least twofold higher than the $IC_{50}$ (50 % inhibition concentration) of sensitive wild-type cells.

Culture plates containing cells spread onto selection medium are incubated under suitable growth conditions, for example 18-32°C, preferably 25-28°C, either in the dark or in the light until cell colonies are formed. Emerging cell colonies are transferred to fresh medium, liquid or solidified, containing the inhibitor in the desired concentration.

Pregrown suspension cultured cells may first be spread in a small volume of liquid medium on top of the solidified medium. An about equal amount of warm liquid agar or agarose medium (1.2-1.6 % agar or agarose) or other suitable solidifying agents such as Gelrite®, kept molten at around 38-44°C (depending on solidifying temperature of the gelling agent), is added and the plate gently but immediately swirled to spread the cells evenly over the medium surface and to mix cells and agar medium before the medium solidifies.

Alternatively, the cells are mixed with the molten agar medium prior to spreading on top of the selection medium. The medium may contain the inhibitor in the desired concen. tration. This method has the advantage that the cells are embedded and immobilized in a thin layer of solidified medium on top of the selection medium. This method allows for better aeration of the cells as compared to embedding cells in the whole volume of 20-30 ml per 10 cm Petri dish.

As a further alternative, herbicide tolerant cells may be selected in a liquid selection medium. Precultured cells are inoculated at a suitable cell density into liquid medium containing the desired concentration of a herbicidal AHAS inhibitor. Cells are subcultured, as appropriate depending on the rate of growth, using fresh medium containing the desired inhibitor concentration after a period of 2 days to 6 weeks, preferably 7-10 days.

The liquid or the solid medium or any medium used to prepare a cell suspension may contain the herbicidal AHAS inhibitor in the desired concentration, i.e. the concentration determined from the dose-response curve, for example 20 ppb, 50 ppb, 100 ppb, 500 ppb, 1000 ppb or the like.

The herbicidal AHAS inhibitor may be anyone of the herbicides mentioned above, for example a sulfonylurea herbicide, an imidazolinone herbicide or a triazolopyrimidine herbicide, in particular one of the mentioned sulfonylureas, for example SU872, SU464 or triasulfuron.

Depending on the inhibitor concentration used, cell growth will be considerably slower than in the absence of inhibitor.

In order to obtain cell cultures which have tolerance to enhanced levels of AHAS inhibitor herbicides, cell colonies emerging from plated cells or suspensions grown in the presence of a herbicide are transferred, in a step-wise manner, to increasingly higher concentrations of herbicides, for example sulfonylurea herbicides, in particular the mentioned sulfonyureas, for example SU872, SU464 or tria sulfuron. The increase in herbicide concentration at each step may be two- to fivefold. The cells are then grown for 1-10 subcultures on the elevated herbicide concentration before they are transferred to the next higher concentration. The process of transferring cell colonies to ever increasingly higher concentrations of herbicides is repeated 1-10 times so that cells are obtained which grow well at very high herbicide concentrations.

Further, herbicide tolerant cell cultures with a broad spectrum of tolerance may be obtained by selection with two, three or more different herbicidal AHAS inhibitors. The different herbicides may be used as a mixture. Preferentially, however, the cells are first grown in medium containing one single herbicide and are then transferred to medium containing a second herbicide, for example first in medium containing SU464 and then in medium containing SU872 at the concentrations mentioned above. In particular, the cells are grown in the presence of one herbicide, transferred in a step-wise manner to media containing increasingly higher concentrations of this same herbicide, next transferred to media containing low concentrations of a different herbicide and finally grown in the presence of increasingly higher concentrations of this second herbicide. The cells may be cultured for 1-10 subcultures before transfer to the next herbicide concentration and/or the next type of herbicide.

Particular procedures are used to select corn (maize) cell cultures tolerant to herbicidal AHAS inhibitors. Which procedure is used depends on the culture characteristics and specific requirements of the corn genotypes used. Preferably, inbred lines of elite corn germplasm are used, but the procedures are equally applicable to corn varieties or hybrids. The media and culture conditions are determined for each corn inbred cultured and are used in accord with the specific cultural requirements of that genotype. Suitable examples of media and culture conditions are mentioned above.

For example, the selection may be performed on embryogenic callus. This procedure is used, for example,

9

with elite inbreds 8N810, 2717, 5N984 and 6N615 (Funk Seeds International). It uses embryogenic callus of either type 1 or type II morphology as the starting material for selection. Type 1 and type II embryogenic callus morphologies are known in the art (C.E. Green et al., in: K. Downey et al. (eds.), Advances in gene technology: Molecular genetics of plants and animals, Miami Winter Symposium 20: 147-157, Academic Press, New York, 1983). Embryogenic callus is initiated from immature zygotic embryos as described above, or from other suitable explants, for example tassel primordia or leaf base tissue, and propagated to produce sufficient amounts of callus for selection.

For selection embryogenic callus is transferred to callus maintenance medium containing suitable amounts of growth regulators, for example an auxin-type growth regulator such as 2,4-D and containing a suitable concentration of a herbicidal AHAS inhibitor. The composition of the callus maintenance medium is chosen in accord with the special growth requirements of the corn genotype used. The inhibitor concentration useful for selection is determined by culturing embryogenic callus on medium containing various amounts of the inhibitor and by measuring the growth response. Useful inhibitor concentrations are between 1 and 100 ppb of a herbicidal AHAS inhibitor as described above, for example of a herbicidal sulfonyurea, for example SU872, SU464 or triasulfuron. The preferred inhibitor is SU872. A useful inhibitor concentration is one which inhibits the growth of embryogenic callus by 70-90 percent. Callus capable of growing at a rate exceeding that of most cultures is transferred to fresh medium of the same composition. Only cultures with a desired embryogenic morphology are subcultured.

Cultures are propagated through one to several subcultures onto fresh medium containing the inhibitor at weekly or bi-weekly intervals whereby only the fastest growing cultures are subcultured. The callus may be grown for one or several subcultures on medium containing one level of herbicide before it is transferred to a medium containing a higher concentration of herbicide. The concentration of the herbicide is increased usually by a factor of two to three upon transfer. When the cultures are growing well on this herbicide concentration, they are again transferred to the next higher concentration. This stepwise transfer to a higher selection level is repeated several times until a desired level of tolerance is achieved. When sufficient callus has been produced it is transferred to a medium suitable for embryo maturation and plant regeneration as described below.

It is possible to perform selection using embryogenic corn suspension cultures with corn genotypes which form regener. able suspension cultures. This procedure is used, for example, with elite corn inbreds 2717 and 5N984 (Funk Seeds International). Embryogenic suspension cultures are established as described in the EXAMPLES, and they are maintained by subculturing at weekly intervals to fresh medium. The medium contains growth regulating components suitable for maintaining the desirable friable embryogenic culture morphology. Preferred media are MS or N6 based liquid media containing 1-100 mg/l 2,4-D or a related compound as mentioned above as a growth regulator.

For selection, a suitable amount of herbicidal AHAS inhibitor is added to the medium. The suitable herbicide concentration is determined by culturing the cells in media supplemented with various amounts of herbicide and by measuring the growth response of the cultures. A suitable herbicide concentration is one which reduces the growth of the cultures by at least 50 percent. In accord with this procedure, 0.1 to 100 ppb of a herbicidal AHAS inhibitor as mentioned above, for example of a herbicidal sulfonylurea, for example SU872 are used for selection. The suitable herbicide concentration used with embryogenic suspension cultures may be different from the concentration used with embryogenic callus of the same corn genotype, as the different culture types may differ in sensitivity to the inhibitor. Cultures are grown in the medium containing the inhibiting herbicide for one to several subcultures. When cultures appear to grown well in the presence of herbicide, they are transferred to medium containing a higher herbicide concentration. This concentration is usually two to three fold higher than the one previously used. Cultures growing well on the higher herbicide concentration are subcultured for one to several times in the presence of this higher concentration of herbicide and are then transferred to the next higher level of herbicide. When the cultures are growing well on this herbicide concentration, they are transferred to the next higher concentration. This stepwise transfer to a higher selection level is repeated several times until a desired level of tolerance is achieved. When a sufficient cell mass has been produced cells are plated onto a solidified medium suitable for embryo maturation and plant regeneration as described below.

In order to assess the effectiveness of the selection method a dose-response curve of the growth of cells at different concentrations of herbicides is determined. Suspension culture cells of herbicide sensitive wild-type and herbicide tolerant selected cells are inoculated into appropriate amounts of liquid medium of desired herbicide concentration contained in a suitable flask, care being taken to inoculate the same amount of cells into each flask. Each flask contains an equal volume of medium. Several replicate flasks are inoculated per herbicide concentration. The herbicide concentrations are selected from zero ( = control) up to a concentration at which the tolerant cells do not grow any more. Usually, herbicide concentrations are used over several orders of magnitude. To bridge a wide range of concentrations, a logartithmic scale is used, for example, 0.1-0.3-1.0-3.0-10-30-100-300-1,000-3,000-10,000 ppb. Several samples of inoculated cells are also taken at the time of inoculation to determine the mass of cells inoculated per flask. Cells are then incubated for growth under controlled conditions of temperature and light for several days. Cells are harvested and the fresh mass of cells is weighed. Cell growth is determined and expressed as gain of cell fresh mass within a predetermined amount of time, for example 10 days, and expressed as a percentage relative to the mass of cells grown in the absence of herbicide. $IC_{50}$ values, the herbicide concentration at which cell growth is 50 % of control growth, are determined from graphs of plotted data of relative cell mass vs. herbicide concentration.

Dose-dpendent growth may also be measured using pieces of callus derived from a herbicide resistant cell culture transferred to solidified culture medium containing the different herbicide concentrations. Relative growth is determined after a suitable culture period, for example 2-6 weeks, by weighing the callus pieces and comparing to a control culture grown in medium without herbicide.

The method using cell suspension cultures is preferred for its greater accurancy.

In order to determine the extent at which cells show cross tolerance to analogous or completely different herbicides, the dose-response curves are obtained by growing cells in increasing concentrations of each of the chosen herbicides. The relative growth of the cells and their $IC_{50}$ value is determined for each herbicide for comparison. Again the cross tolerance may be determined on callus culture, but the method using cell suspension culture is preferred for its greater accuracy.

In order to determine whether the herbicide tolerant phenotype of a cell culture is maintained over time, cells are transferred from herbicide containing medium to medium without herbicide. Cells are grown in the absence of herbicide for a period of one week to several months, for example 3-18 months, employing regular subculturing at suitable intervals, for example 7-10 days for suspension cultures or 3-6 weeks for callus cultures. At a given point in time, a known quantity of cells is transferred back to herbicide containing medium and cultured for a suitable period, for example 10 days (suspension cultures) or 4 weeks (callus cultures). Relative growth is determined as described above. This procedure is repeated with cultures grown for different periods of time, for example 3 to 18 months, in the absence of the herbicidal AHAS inhibitor.

Transformation of plants

The invention further concerns a method of preparation of a plant cell as described above wherein any kind of plant material is transformed with a recombinant DNA.

Plants may be transformed by Agrobacterium transfection of plant material, by direct gene transfer into protoplasts, by transformation of plant cells by microprojectile bombardment or by injection of DNA. These methods are known in the art and are applied accordingly.

For example, isolated plant protoplasts may be co-cultivated with Agrobacteria containing the foreign DNA sequences as described by L. Marton in Cell Culture and Somatic Cell Genetics of Plants, Vol. 1: 514-521 (1984). Transgenic cell colonies and callus are selected, and transgenic plants are obtained following established procedures known in the art. The appropriate procedure may be chosen in accordance with the plant species used.

Alternatively, the desired exogenous DNA may be introduced into protoplasts in accordance with the methods decribed in the following publications: J. Paszkowski et al., EMBO J. 3:2717 (1984); British patent application GB 2 159 173; European Patent Application EP 129 668; R.D. Shillito et al., Bio/Technology 3: 1099 (1985); I. Potrykus et al., Mol. Gen. Genet. 199: 183 (1985).

For the direct gene transfer into protoplasts of cereal and grass species the methods described in the following references may be used: H. Lörz et al., Mol. Gen. Genet. 199: 178 (1985); M.E. Fromm et al., Nature 319: 719 (1986); British Patent Application GB 2 140 822; and I. Negrutiu et al., Plant Mol. Biol. 8: 363 (1987).

The exogenous DNA may be added to the protoplasts in any form such as, for example, naked linear, circular or supercoiled DNA, DNA encapsulated in liposomes, DNA in spheroplasts, DNA in other plant protoplasts, DNA complexed with salts, and the like.

Another method to introduce foreign DNA sequences into plant cells comprises the attachment of said DNA to particles which are then forced into plant cells by means of an acceleration device (T.M. Klein et al., supra; D.E. McCabe et al., Biotechnology 6: 923-926, 1988). Any plant tissue or plant organ may be used as the target for this procedure, including but not limited to embryos, apical and other meristems, buds, somatic and sexual tissues in vivo and in vitro. Transgenic cells and callus are selected following established procedures known in the art. Targeted tissues are induced to form somatic embryos or regenerate shoots to give transgenic plants according to established procedures known in the art. The appropriate procedure may be chosen in accordance with the plant species used.

Transfer of DNA into plant cells is also achieved by injection into isolated protoplasts, cultured cells and tissues as described in U.S. Patent 4,743,548 and by T.J. Reich et al., Bio/Technology 4: 1001-1004 (1986); Can. J. Bot. 64; 1259-1267 (1986), and injection into meristematic tissues of seedlings and plants as described by A. De La Pena et al., Nature 325: 274-276 (1987), A.C. Graves et. al., Plant Mol. Biol. 7: 43-50 (1986), G.M.S. Hooykaas-Van Slogteren et al., Nature 311: 763-764 (1984), Grimsley et al., Biotechnology 6: 185 (1988), and Grimsely et al., Nature 325: 177 (1987). Transgenic plants and progeny therefrom are obtained by conventional methods known in the art.

The regenerated plant may be chimeric with respect to the incorporated foreign DNA. If the cells containing the foreign DNA develop into either micro- or macrospores, the integrated foreign DNA will be transmitted to sexual progeny. If the cells containing the foreign DNA are somatic cells of the plant, non-chimeric transgenic plants are produced by conventional methods of vegetative propagation either in vivo, i.e. from buds or stem cuttings, or in vitro following established procedures known in the art. Such procedures may be chosen in accordance with the plant species used.

In particular, tobacco leaf disks may be transformed with Agrobacterium in the following way: The different genotypes of Agrobacterium tumefaciens containing the desired genes to be tranformed into tobacco are grown on Agrobacterium minimal medium known in the art. 5-7 mm leaf discs are punched aseptically from in vitro cultured tobacco and are dipped into the bacterial suspension for several minutes essentially as

11

described by R. Horsch et al., Science 227: 1229-1231 (1985). Leaf disks are then transferred to filter paper on a medium inducing shoot formation which contains a suitable antibiotic to kill the Agrobacterium, for example carbenicillin, kanamycin, hygromycin, vancomycin, mefoxin or others, or combinations of the mentioned antibiotics. The leaf disk is further transferred to a liquid or preferably solid shoot inducing growth medium additionally containing a herbicidal amount of an AHAS inhibitor, for example a sulfonyurea herbicide, such as triasulfuron, SU464 or SU872. The concentration of the herbicidal AHAS inhibitor is in the useful range as determined by the dose-response curves as described above, for example between 10 and 100 ppb SU872. The concentration of the antibiotic is chosen so as to kill essentially all the Agrobacterium, for example for carbenicillin, vancomycin or mefoxin it may be between 100 and 5000 mg/l, preferably around 500 mg/l, for kanamycin in the range of 10 to 1000 mg/l, preferably around 100 mg/l, and for hygromycin between 5 and 100 mg/l, preferably around 20 mg/l.

Alternatively, the transgenic callus is selected using a selection agent other than the herbicidal AHAS inhibitor. In this method, the choice of selection agent is dictated by the resistance gene in the vector construct contained in the Agrobacterium used in the transformation. For example, if the vector construct comprises a kanamycin resistance gene, the desired transgenic callus and shoots are selected using a medium containing between about 50 and 200 mg/l kanamycin. If the vector construct comprises a hygromycin resistance gene, a medium containing between about 20 and 100 mg/l hygromycin is used. The expression of the tolerance towards the herbicidal AHAS inhibitor is later tested on tissue selected previously by the mentioned selection agent.

Shoots that arise from the cultured tissue on any of the mentioned selection media are transferred to medium which induces plantlet development. Preferably this medium also contains the herbicidal AHAS inhibitor in useful concentrations as mentioned above, for example around 100 ppb SU872, and if desired, the mentioned antibiotics to kill Agrobacterium, preferably in the mentioned concentrations. Plantlet development is allowed to continue for at least three weeks. Plantlets may be divided to give replicate cuttings which are allowed to develop and root. Rooted plantlets are then transplanted to a soil-vermiculite mixture and moved to the greenhouse and full plants grown.

The desired genes are introduced into Agrobacterium used for transformation by methods known in the art. For example suitable shuttle vectors may be transferred from E. coli SM17 (R. Simon et al., Bio/Technology 1: 74-75, 1983) to an Agrobacterium tumefaciens strain by mating. Alternatively, the vectors may be transformed into Agrobacterium tumefaciens strain by mating. Alternatively, the vectors may be transformed into Agrobacterium tumefaciens by the method of M. Holsters et al., supra. A suitable Agrobacterium tumefaciens strain is for example a derivative of strain EHA101 (E. Hood et al., J. Bacteriol. 168: 1291-1301, 1986) in which the kanamycin marker of the plasmid has been replaced by the spectinomycin/streptomycin portion of Tn7.

For the transformation of corn (maize), the following method is preferred: Corn protoplasts are prepared from a cell suspension culture, for example from a suspension culture as described above. This suspension culture is incubated in a suitable enzyme mixture digesting the cell walls. Such enzyme mixtures are known in the art. Digestion is carried out depending on the type and amount of enzyme, for example at around 30°C on a slow rocking table for a period of several hours. The preparation is preferably monitored under an inverted microscope for protoplast release. The protoplasts are collected preferably by filtration through sieves of suitable porosity, for example a 100 μm and/or a 50 μm mesh sieve, washed and conditioned in a salt solution, preferably a buffered solution of potassium, magnesium and calcium chloride, then finally suspended in a buffered magnesium salt solution for transformation.

Transformation of corn protoplasts by electroporation may be performed by treating the protoplasts with a heat shock of around 45°C for a few, for example five minutes, followed by cooling to room temperature on ice. The heat shock treatment is preferred, but not essential. The linearized plasmid containing the desired AHAS gene constructs and, if desired, carrier DNA, for example calf thymus DNA, are added to the protoplast suspension. An aqueous polyethylene glycol solution of a concentration between 10 and 40 % and a molecular weight between 2000 and 10,000 containing a magnesium salt and optionally other additives such as sugars or sugar alcohols, for example mannitol, is added to the protoplasts. The mixture is mixed well but gently, and incubated for a few minutes, for example for 10 minutes. However, the incubation with polyethylene glycol solution before electroporation may be omitted. The sample is transferred to the chamber of an electroporator and samples pulsed two to five times at intervals of several seconds, for example three times at 10 or 3 seconds intervals, at initial voltages of between 1000 and 2800 Vcm$^{-1}$, and an exponential decay time of around 10 μsec. Optionally the protoplasts are cooled to a temperature below room temperature after electroporation, for example to around 16°C. The protoplasts may then be cultured in the usual media, for example in a solidified complete medium containing all essential mineral salts, vitamins, organic acids, sugars, sugar alcohols, nucleotides and amino acids, optionally containing a low amount of a growth regulator such as 2,4-dichlorophenoxyacetic acid. The protoplasts may be washed beforehand, for example with a buffered solution of potassium, calcium and magnesium salts, or a buffered solution of sodium, potassium and calcium salts, each optionally containing a sugar, for example glucose. In a preferred method, the protoplasts are cultured on filters on top of nurse cultures, prepared for example from the embryogenic corn suspension culture described above or from Black Mexican Sweet corn suspension cultures. Any protoplast culture medium may contain a herbicidal amount of an AHAS inhibitor, for example a sulfonylurea herbicide such as those mentioned above, in particular SU872, in order to effect selection already at this stage.

Transformation of corn protoplasts in the presence of polyethylene glycol is an alternative. The protoplasts

EP 0 360 750 A2

are treated with a heat shock of around 45°C for a few, for example five minutes, followed by cooling to room temperature on ice. This heat shock treatment is not essential and may be omitted. The linearized plasmid containing the desired gene constructs and, if desired, carrier DNA, for example calf thymus DNA, are added to the protoplast suspension. A slightly alkaline aqueous solution of a pH between 8 and 9 containing polyethylene glycol of a molecular weight between 2000 and 10,000, preferably around 6000, and a calcium salt, for example calcium nitrate at a concentration around 0.1 M, and optionally other additives such as sugars or sugar alcohols, for example mannitol, is added to the protoplasts to give a polyethylene concentration of between 12 and 25 %, preferably around 20 %. The mixture is incubated for 10 to 60 minutes, preferably around 30 minutes, with occasional gentle shaking, then stepwise diluted with a salt solution, for example a solution containing potassium and calcium salts, preferably a solution containing sodium, potassium and calcium salts and a sugar such as glucose, for example by adding aliquots at two- to three-minutes intervals up to ten times, for example five times. The protoplasts may then be cultured in the usual media, for example in a solidified complete medium containing all essential mineral salts, vitamins, organic acids, sugars, sugar alcohols, nucleotides and amino acids, optionally containing a low amount of a growth regulator such as 2,4-dichlorophenoxyacetic acid.

Transformed protoplasts that have undergone electroporation or polyethylene glycol treatment are cultured in the mentioned complete medium preferably in the dark at about room temperature, for example around 26°C. Within 14 days, colonies arise from the protoplasts. These colonies are subcultured in the mentioned complete media or other callus-sustaining media known in the art for extended periods of time, for example a few weeks. Preferably the media for subculture contain a herbicidal amount of an AHAS inhibitor to select for transgenic cells containing the desired AHAS gene construct. For example, the medium contains a herbicidal amount of a sulfonylurea herbicide as determined in the dose-response curve method mentioned above. Typically, the medium contains between 50 and 500 ppb, for example 100 ppb, of the herbicide SU872. The media may contain also an antibiotic to which the DNA constructs used in the transformation contain a resistance marker, for example hygromycin. If the transformed cells are expected to contain a hygromycin resistance gene, this antibiotic is added to the medium for callus subculture in a concentration of between 20 and 200 mg/l, preferably around 50 mg/l.


Protoplast fusion and plant regeneration

In order to obtain a plant from a non-regenerable cell culture cell which is herbicide tolerant, the method of protoplast fusion and somatic hybridization is used. The herbicide tolerant non-regenerable cell culture serves as one fusion parent. The second fusion parent are protoplasts isolated from a regenerable tissue or a regenerable cell culture of the same plant species.

Protoplasts are isolated from the herbicide tolerant cultured cells by incubating the cells in an osmotically buffered enzyme solution comprising cellulolytic and pectinolytic enzymes, for example Cellulase Onozuka® R10 or RS, Macerozyme®, Pectolyase®, Rhozyme®, Pectinase®, Macerase®, Driselase®, or the like, until protoplasts are obtained, for example 1-5 hours at room temperature, depending on the enzymes and their concentration used. The protoplast-enzyme mixture is poured through a suitable filter, for example a 50 μm or 100 μm stainless steel screen. Protoplasts in the filtrate are washed free of enzyme by repeated centrifugation or flotation on sucrose of suitable concentration or Percoll® followed by resuspending in solution.

In parallel, protoplasts are also isolated from leaf tissue, hypocotyl or cotyledon tissue of in vitro grown or normally soil-grown wild-type plants by incubating pieces of tissue in a suitable osmotically buffered enzyme solution. The protoplasts from herbicide-tolerant cell culture and from wild-type leaves are mixed in about equal numbers. To induce fusion an equal amount of 20-50 % aqueous polyethylene glycol of a molecular weight between 1500 and 6000 is added to the mixed protoplast suspension. After incubation for 5-30 min, a suitable diluent is added over a period of 15-60 min. Suitable diluents are aqueous solutions containing calcium salts, for example calcium chloride or calcium nitrate, and if desired, sugar or sugar alcohols, such as mannitol. Alternatively, the protoplasts may also be fused by the application of an electric pulse, for example as described by U. Zimmermann et al., Planta 151: 26, 1981. The obtained fusion products are cultured in the usual protoplast culture media known in the art, for example in a medium containing salts, vitamins, sugar or sugar alcohols. The protoplasts may be cultured in liquid medium or in a solidified medium in the dark or under low light at about room temperature or slightly below or above.

In order to obtain hybrid cell colonies following fusion of protoplasts a selection system is used: The addition of a herbicidal amount of an AHAS inhibitor will kill all wild-type cells derived from the sensitive fusion parent, i.e. the leaf, hypocotyl or cotyledon protoplasts. Herbicide resistant cells derived from the herbicide tolerant fusion parent, i.e. the tolerant cell culture protoplasts, will grow under these conditions. Hybrid cells formed by fusion of a herbicide tolerant cell culture protoplast and a herbicide sensitive protoplast will also be able to grow because of dominance of the herbicide tolerance. Further selection is obtained when the culture medium contains growth regulators stimulating shoot formation. Cells derived from protoplasts of the herbicide tolerant cell culture cells will not form shoots because of their inherent defect which renders them non-regenerable. However, hybrid cells comprised of a protoplast of the non-regenerable cell culture and a regenerable leaf, hypocotyl or cotyledon protoplast will be able to form shoots under shoot inducing conditions, because regenerability generally behaves as a dominant trait. The combination of the two selection steps either applied one after the other or concurrently allows one to obtain regenerated hybrid plants which are herbicide tolerant.

13

EP 0 360 750 A2

In particular, when small cell colonies have formed from the cultured protoplast fusion mixture, usually after 2-4 weeks, cells are washed and transferred to selection medium containing a herbicidal amount of the AHAS inhibitor, for example a sulfonylurea herbicide, preferably one of the sulfonylureas mentioned above, for example SU872, SU464 or triasulfuron, which is sufficient to inhibit growth of cells of the sensitive wild-type fusion parent. The amount of herbicide which is sufficient to inhibit growth of wild-type sensitive cells is determined as described above. For example 10, 30 or 100 ppb of SU872 are used. Cells are grown in the selection medium for one to several subcultures. The cells growing in this herbicide containing medium are subsequently grown in a medium suitable to promote shoot and plant regeneration. Such regeneration media are known in the art and may be chosen according to the plant species used. The regeneration medium may also contain 10 to 100 ppb of a herbicidal AHAS inhibitor, for example one of the mentioned sulfonylureas, for example SU872. The cells are incubated in the light until green shoots are formed, usually for 3-6 weeks.

In the preferred single-step selection method, the cell colonies derived from the protoplast fusion mixture are cultured in the absence of herbicide for about 4-6 weeks. Then the colonies are plated on top of solidified culture medium which is suitable to induce shoot formation and which contains a herbicidal amount of sulfonylurea herbicide, for example SU872 sufficient to inhibit the growth of sensitive wild-type cells. Colonies are grown on this medium until green shoots are formed.

In a further modification, the mixed protoplast suspension obtained after fusion as described above is cultured by embedding in agar or agarose containing protoplast culture medium. When small cell colonies arise from the plated cells, usually after 2-4 weeks, the agarose medium is divided into several sectors. One of the sectors is transferred to about 10-50 ml of liquid culture medium to serve as a control to determine the extend of colony formation (plating efficiency). The other sectors are transferred to the same volume of liquid culture medium containing a herbicidal amount of a sulfonylurea herbicide, an imidazolinone herbicide or a triazolopyrimidine herbicide, preferably containing 10 to 100 ppb SU872, sufficient to inhibit growth of sensitive wild-type cells. These cultures are incubated either in the dark or in the light on a shaker under gentle agitation until prominent cell colonies are formed. The liquid medium is changed at suitable intervals, usually 1 week. Prominent colonies capable of growing in the presence of the herbicide are then cut out of he solid medium sector and transferred to fresh solidified medium containing the herbicidal amount of AHAS inhibitor. Pieces of growing callus are then transferred to medium suitable to promote shoot and plantlet regeneration and incubated in the light. Such regeneration media are known in the art and may be chosen according to the plant species used. When green shoots are formed and are sufficiently well developed (1-3 cm tall), they are transferred to a fresh medium to promote further growth, shoot elongation and rooting. Suitable media are known in the art. When plantlets are developed sufficiently well, they are transferred to a suitable potting compost mixture and acclimatized to growing in soil. The medium may contain a herbicidal amount of a AHAS inhibitor, for example 10 to 100 ppb of one of the mentioned sulfonylurea herbicides, for example triasulfuron, SU464 or SU872. Plants or parts thereof are analyzed for their herbicide tolerance properties according to the methods described hereinafter.

Progeny is obtained by using standard methods of open or controlled pollination.

Plants are regenerated from embryogenic corn callus cultures by allowing the somatic embryos to mature and germinate into small plantlets which are transferred to soil and grown to maturity. Embryo maturation is promoted by transferring embryogenic callus to fresh culture medium containing a reduced concentration, for example 0.01 to 0.5 mg/l, or none, or the auxin-type growth regulator(s) as described above. A small amount, in the range of 0.01 to 20 mg/l, of a cytokinin, for example 6-furfurylaminopurine (kinetin), trans-zeatin, zeatin riboside, isopentyladenine (2-iP) or 6-benzylaminopurine (6-BAP) may be included in the medium to promote shoot growth. The medium used to promote regeneration may contain an altered, for example reduced, amount of salts, vitamins or carbohydrate source. Further the medium may contain a small amount, for example 0.1-30 ppb, of the herbicidal AHAS inhibitor used for selection. The conditions best suited for regeneration will depend upon the corn genotype used. To promote root formation, the small plantlets may be transferred to medium containing a small amount, for example 0.01 to 1 mg/l, of a weak auxin, for example α-napthaleneacetic acid (NAA), indole-3-acetic acid (IAA) or indole-3-butyric acid (IBA).

Once roots are sufficiently developed, plantlets are trans. planted to a suitable potting mix and transferred to the greenhouse. In order to facilitate acclimatization, plantlets may be covered for some days with a clear plastic cup to retain humidity and with shading. Plantlets may also be cultured for some time on a misting bench for acclimatization. Once established in soil plants are grown essentially like normal corn plants until maturity. Upon flowering plants are hand pollinated, preferably self pollinated. Seeds are harvested and stored as known in the art.

Cell mass obtained in a selection procedure using suspension cultures is regenerated by the methods described above for embryogenic corn callus or any other method known in the art. The cells are plated onto a solidified culture medium suitable for embryo maturation and then treated as described above.

Other methods of plant regeneration are known in the art. They are adapted to the particular plant species and are used accordingly.

Evaluation of herbicide tolerance

In order to determine the extent of tolerance to a herbicidal AHAS inhibitor in plants or tissues derived from such plants, the following tests may be used:

Pieces of tissue are explanted from transgenic, mutant or somatic hybrid plants containing the desirable

14

AHAS gene according to this invention and are placed in a medium suitable to promote cell proliferation. The medium used may be a liquid or a gel so as to produce a cell suspension or a callus culture. Such media are known in he art and may be chosen according to the plant species used. The cell culture obtained is subcultured as described above until a suitable cell mass is obtained. These cells are tested for the herbicide tolerance as described above.

Further, the herbicidal AHAS inhibitor may be applied directly to the plant resembling the methods used in the field. In pre-emergent application, an inhibitor solution of suitable concentration is applied to the substrate into which seeds of the test plant have been sown. In post-emergent application, plants are grown in seed flats, pots or in soil in a greenhouse, phytotron or in a field. An inhibitor solution of suitable concentration is sprayed to cover the shoots. A wetting agent is usually included for better contact. A defined volume of inhibitor solution is used or the plants are sprayed to run-off. In post-emergent application to soil, after plants are established in their substrate, a known volume of inhibitor solution of a suitable concentration is applied to the soil. Damage from the inhibitor is evaluated using standard methods of scoring, for example by determining the number of non-germinated plants or determining damaged leaf area or number of killed plants.

The seed plating method is suitable when samples of seed need to be evaluated for inhibitor tolerance in relatively small scale under carefully controlled conditions. This method is also useful to test the genetic segregation of traits in the progeny of inhibitor tolerant plants. Seeds are sterilized by a standard procedure and then plated onto solidified germination medium which contains a suitable concentration of inhibitor. Seed germination and inhibitor damage, for example bleaching or kill, are evaluated after a suitable period of time, usually 2-6 weeks.

A further useful method is the seedling pouch test. This method is suitable for testing the inhibitor tolerance of seed, for example of corn seed, to determine the level of tolerance (dose-response curve), the cross tolerance of seeds from a particular herbicide tolerant line to other herbicidal AHAS inhibitors and to determine segregation of inhibitor tolerance in progeny of inhibitor tolerant plants. Seeds are placed on germination paper, and the paper is wetted with a salt solution containing the desired concentration of herbicidal AHAS inhibitor, for example one of the mentioned sulfonylurea herbicides, for example SU872. A second paper is applied on top and the two papers rolled to form a wick, placed in germination solution, and incubated at around room temperature in the dark or under low light at high relative humidity for 5 to 30 days. The germination solution may contain a fungicide to reduce fungal contamination. The procedure may be performed under aseptic conditions. Root and shoot length are measured of the seedlings in order to determine the amount of herbicide tolerance.

Determination of AHAS specific activity

The determination of AHAS specific activity without herbicide or in the presence of herbicidal amounts of AHAS inhibitors is used to judge whether herbicide tolerant cells contain an altered AHAS enzyme, which is known in the art, or whether these cells express an increased amount of the wild-type enzyme or of an AHAS enzyme with essentially wild-type AHAS characteristics.

Suitable conditions for assaying AHAS activity are known in the art. An improved protocol is used and detailed in the EXAMPLES in order to assure correct results. In particular this protocol includes the use of glycerol, flavine adenine dinucleotide and magnesium salts which stabilize the enzyme activity. A further stabilizing effect is provided by thiamine pyrophosphate. Preferentially a protease inhibitor, for example phenylmethylsulfonyl fluoride, is included during tissue extraction and purification to ensure stability of the enzyme. A linear activity is found under the assaying conditions at 30°C for up to 2.5 hours. The AHAS activity is found to be unstable to freezing and storage at below 0°C temperatures, but is stable for up to 4 days at 5°C. However, the plant tissue or cells can be frozen in liquid nitrogen, stored at -80°C or lower and thawed again without loss of AHAS activity.

As described in the art the plant AHAS enzyme seems to consist of multiple isozymes which show different sensitivity to freezing, different patterns of sensitivity to inhibition by herbicides, and different feed-back inhibition by the amino acids valine, leucine and isoleucine.

The AHAS enzyme assay proves that, following the selection procedures detailed above and in the EXAMPLES, plant cells can be found which are tolerant to otherwise herbicidal amounts of an AHAS inhibitor, contain increased AHAS enzyme levels when compared to corresponding wild-type cells, but contain an AHAS enzyme which is comparable to wild-type AHAS enzyme with respect to inhibition by herbicides and feed-back inhibition by the mentioned amino acids. In particular, out of 21 herbicide tolerant tobacco cell lines prepared byselection using increasing amounts of the preferred herbicidal AHAS inhibitor SU464, 5 cell lines displayed AHAS activity at least two times higher than in correspondingwild-type cells, but contained an AHAS sensitive to inhibition similar to the wild-type cells. Likewise, out of 108 herbicide tolerant tobacco cell lines prepared byselection using increasing amounts of the preferred herbicidal AHAS inhibitor SU872, 22 cell lines displayed AHAS activity at least two times higher than in corresponding wild-type cells, but contained an AHAS sensitive to inhibition similar to the wild-type cells.

Use

A particular use envisaged by this invention is a method of controlling weed in a plantation. Plants of the invention as described above are used in such a plantation, and all plants in said plantation are contacted with a plant controlling amount of a herbicidal AHAS inhibitor. The plants of the invention will survive such a

15

treatment because they are tolerant to the herbicidal AHAS inhibitor, but the weed plants will be killed.

Suitable modes of application of a herbicidal AHAS inhibitor to such a plantation are well known in the art and are chosen according to the circumstances such as type of crop plant of the invention, type of weed generally encountered in such a plantation, directions for use of the herbicidal AHAS inhibitors and environmental conditions such as type of soil, expected weather and the like. Principal modes of application are pre-emergent application, i.e. application of the herbicidal AHAS inhibitor to the soil into which seeds of the plant of the invention have been sown, and post-emergent application, i.e. application to all plants or to the soil after the plants of the invention have grown for some time. The concentration and amount of the herbicidal AHAS inhibitor to be used is so chosen as to control weed in the plantation, avoiding any excess in order to minimize or prevent environmental damage.

In a preferred aspect of the invention, crop plants are used in which the tolerance to the herbicidal AHAS inhibitor is regulatable by a chemical regulator. All plants in the plantation are contacted with said chemical regulator and simultaneously or consecutively with a weed controlling amount of the herbicidal AHAS inhibitor. Again the worker skilled in the art will use the appropriate mode of application as mentioned above, and appropriate concentrations and amounts of chemical regulator and herbicidal AHAS inhibitor.

Further uses of the plant cells and plants of the invention are envisaged. For example the tolerance to a herbicidal AHAS inhibitor as described above may be used for selection of somatic hybrid plants and of transgenic plants derived from plants of the invention. If the tolerance to the herbicidal AHAS inhibitor is conferred to plant cells by a recombinant DNA containing an AHAS gene, this property may be used in a method of selecting plant cells representing a desired genotype. Any mixture of plant cells comprising cells of the desired genotype are cultured in the presence of a herbicidal AHAS inhibitor and surviving cells are isolated. In particular, any of he preferred recombinant DNA and/or chimeric DNA constructs as described above may be used as a selectable marker conferring tolerance to an AHAS inhibitor herbicide. Furthermore the increased levels of AHAS enzyme in the plant cells of the invention make these cells a convenient source for the isolation, purification and detailed characterization of the enzyme. Plant cells of the invention can also be used conveniently in screening methods for the detection of novel and more selective AHAS inhibitor herbicides.

EXAMPLES

Standard recombinant DNA and molecular cloning techniques used here are described by T. Maniatis, E.F. Fritsch and J. Sambrook, Molecular Cloning: A Laboratory Manual, Cold Spring Harbor Laboratory, Cold Spring Harbor, NY (1982) and by T.J. Silhavy, M.L. Berman, and L.W. Enquist, Experiments With Gene Fusions, Cold Spring Harbor Laboratory, Cold Spring Harbor, NY (1984).

Abbreviations:
ATP adenosine triphosphate
BSA bovine serum albumin
BMS Black Mexican Sweet corn
2,4-D 2,4-dichlorophenoxyacetic acid
DTT dithiothreitol
DMSO dimethyl sulfoxide
EDTA ethylene diamine tetraacetic acid
FAD flavine adenine dinucleotide
FW fresh weight
kbp kilo-basepairs
MES 2-morpholinoethanesulfonic acid
MW molecular weight
PCV packed cell volume
PBS phosphate buffered saline
PEG polyethylene glycol
PMSF phenylmethylsulfonyl fluoride
SDS sodium dodecyl sulfate
TPP thiamine pyrophosphate
Tris tris(hydroxymethyl)aminomethane

Buffers:

TE buffer
10 mM Tris-HCl, pH 8.0, 1 mM EDTA

TENP buffer
100 mM Tris-Hcl, pH 8.0, 10 mM EDTA, 1 % (v/v) NP-40 (Sigma Chemicals)

TBE buffer
89 mM Tris borate, 89 mM boric acid, 2 mM EDTA

LS hybridization buffer
25 % v/v formamide (Ultra pure grade, Bethesda Research Laboratory, Gaithersburg, MD), 5 X SSC, 10 % dextran sulfate (Pharmacia LKB, Uppsala, Sweden), 1 % SDS (BDH Chemicals LTD., Poole, England), 5 X Denhardt's solution, 200 μg/ml sheared and denatured salmon sperm DNA (Sigma Chemicals)

Denhart's solution
0.02 % Ficoll (type 400, Sigma Chemicals, St. Louis, MO), 0.02 % polyvinylpyrrolidone (PVP360, Sigma Chemicals, St. Louis, MO), 0.02 % BSA (Fraction V, Sigma Chemicals)

SSC
0.15 M NaCl, 0.015 M sodium citrate, pH 7.0

Media:

MX1
This medium consists of Murashige and Skoog ("MS", T. Murashige et al., Physiol. Plant. 15: 473-497, 1962) major salts, minor salts and Fe-EDTA (Gibco # 500-1117; 4.3 g/l), 100 mg/l myo-inositol, 1 mg/l nicotinic acid, 1 mg/l pyridoxine-HCl, 10 mg thiamine-HCl, 2-3 g/l sucrose, 0.4 mg/l 2,4-dichlorophenoxyacetic acid, and 0.04 mg/l kinetin, pH 5.8. The medium is sterilized by autoclaving.

Tobacco callus medium
consist of MS major and minor salts and Fe-EDTA (Gibco # 500-1117; 4.3 g/l), MS vitamins, 100 mg/l myo-inositol, 20 g/l sucrose, 2 mg/l α-naphtaleneacetic acid and 0.3 mg/l kinetin.

Tobacco protoplast culture medium
consists of MS major and minor salts, MS vitamins, 100 mg/l myo-inositol, 2 % sucrose, 0,45 M mannitol, 2 mg/l p-chloro-phenoxyacetic acid, and 0.5 mg/l kinetin, pH 5.8.

MSBN
This medium comprises MS major and minor salts (KC Biologicals), 6-benzyladenine (1 mg/l) α-naphtaleneacetic acid (0.1 mg/l), myo-inositol (100 mg/l), nicotinic acid (1 mg/l), pyridoxine (1 mg/l), thiamine-HCl (10 mg/l), and sucrose (30 g/l). The pH is adjusted to 5.8 prior to autoclaving. The medium is usually used solidified with 0.8 % agar or 0.24 % Gelrite®.

OMS
This medium comprises MS major and minor salts and Fe-EDTA (Gibco # 500-1117; 4.3 g/l), B 5 vitamins (O.L. Gamborg et al., Experimental Cell Research 50: 151-158, 1968), 100 mg/l myo-inositol, and 30 g/l sucrose, pH 5.8. The medium is usually used solidified with 0,8 % agar or 0.24 % Gelrite®.

KM
This medium comprises macroelements, microelements and Fe-EDTA as described by K.N. Kao et al., Planta 126: 105-110 (1975), and the following organic compounds: Biotin (0.01 mg/l), pyridoxine-HCl (1 mg/l), thiamine-HCl (10 mg/l), nicotinamide (1 mg/l), nicotinic acid (0.1 mg/l), folic acid (0.4 mg/l), D-calcium-pantothenate (1 mg/l), p-aminobenzoic acid (0.02 mg/l), choline chloride (1 mg/l), riboflavine (0.2 mg/l), vitamin B12 (0.02 mg/l), glycine (0.1 mg/l), sucrose (0.25 g/l), glucose (68.4 g/l), mannitol (0.25 g/l), sorbitol (0.25 g/l), cellobiose (0.25 g/l), fructose (0.25 g/l), mannose (0.25 g/l), rhamnose (0.25 g/l), ribose (0.25 g/l), xylose (0.25 g/l), myo-inositol (0.1 g/l), citric acid (40 mg/l), fumaric acid (40 mg/l), malic acid (40 mg/l), sodium pyruvate (20 mg/l), adenine (0.1 mg/l), guanine (0.03 mg/l), thymidine (0.03 mg/l), uracil (0.03 mg/l), hypoxanthine (0.03 mg/l), cytosine (0.03 mg/l), glutamine (5.6 mg/l , alanine (0.6 mg/l), glutamic acid (0.6 mg/l), cysteine (0.2 mg/l), asparagine, aspartic acid, cystine, histidine, isoleucine, leucine, lysine, methionine, phenylalanine, proline, serine, threonine, tryptophan, tyrosine and valine (each 0.1 mg/l). The solution is filter sterilized. The final pH is 5.8.

Remarks: Macroelements are made up as a 10 X concentrated stock solution, and microelements as a 1000 X concentrated stock solution. Citric, fumaric and malic acid and sodium pyruvate are prepared as a 100 X concentrated stock solution, adjusted to pH 6.5 with $NH_4OH$. Adenine, guanine, thimidine, uracil, hypoxanthine and cytosine are prepared as a 1000 X concentrated stock solution, adjusted to pH 6.5 with $NH_4OH$. The amino acids are added using a 10 X stock solution (pH 6.5 with $NH_4OH$) to yield the given final concentrations. Vitamin stock solution is normally prepared 100 X concentrated.

N6
This medium comprises macroelements, microelements and Fe-EDTA as described by C-C. Chu et al., Scientia Sinica 18: 659 (1975), and the following organic compounds: Pyridoxine-HCl (0.5 mg/l), thiamine-HCl (0.1 mg/l), nicotinic acid (0.5 mg/l), glycine (2.0 mg/l), and sucrose (30.0 g/l). The solution is autoclaved. The final pH is 5.6. The medium is usually used solidified with 0.24 % Gelrite®.

Remarks: Macroelements are made up as a 10 X concentrated stock solution, and microelements as a 1000 X concentrated stock solution. Vitamin stock solution is normally prepared 100 X concentrated.

EXAMPE 1: Growth of tobacco cell suspension cultures

Suspension cultured cells of Nicotiana tabacum, line S3, are grown in liquid culture medium MX1. 100 ml Erlenmeyer flasks containing 25 ml medium MX1 are inoculated with 10 ml of a cell culture previously grown for 7 days. Cells are incubated at 25°C in the dark on an orbital shaker at 100 rpm (2 cm throw). Cells are subcultured at 7 day intervals by inoculating an aliquot sample into fresh medium, by decanting or pipetting off approximately 90 % of the cell suspension followed by replenishing fresh medium to give the desired volume of suspension. Typically, 5-8 g of fresh weight cell mass are produced within 10 days of growth from an inoculum of 250-350 mg cells.

EXAMPLE 2: Production of tobacco cell cultures tolerant to herbicidal AHAS inhibitors by plating cells on solidified selection medium

Cells are pregrown as in EXAMPLE 1. Cells are harvested by allowing cells to sediment, or by brief centrifugation at 500 x g, and the spent culture medium is removed. Cells are then diluted with fresh culture medium to give a cell density suitable for cell plating, about 10,000 colony forming units per ml. For plating, cells in a small volume of medium (approx. 1 ml) are evenly spread on top of solidified culture medium (MX1, 0.8 % agar) containing the desired concentration of the inhibitor. About 20-30 ml of medium are used per 10 cm Petri plate. The suitable inhibitor concentration is determined from a dose-response curve (EXAMPLE 5), and is at least twofold higher than the $IC_{50}$ of sensitive wild-type cells.

Culture plates containing cells spread onto selection medium are incubated under normal grwoth conditions at 25-28°C in the dark until cell colonies are formed. Emerging cell colonies are transferred to fresh medium containing the inhibitor in the desired concentration.

In a preferred modification of the described method the pregrown suspension of cultured cells is first spread in a small volume of liquid medium on top of the solidified medium. An equal amount of warm liquid agar medium (1.2-1.6 % agar) kept molten at arount 40°C is added and the plate gently but immediately swirled to spread the cells evenly over the medium surface and to mix cells and agar medium, before the medium solidifies.

Alternatively, the cells are mixed with the molten agar medium prior to spreading on top of the selection medium. This method has the advantage that the cells are embedded and immobilized in a thin layer of solidified medium on top of the selection medium. It allows for better aeration of the cells as compared to embedding cells in the whole volume of 20-30 ml.

The following herbicidal AHAS inhibitors are used in concentrations of 20 ppb, 50 ppb, 100 ppb, 500 ppb, and 1000 ppb: SU872 and SU 464.

EXAMPLE 4: Production of tobacco cell cultures tolerant to a herbicidal AHAS inhibitor by growing cells in liquid selection medium

Cells cultured as in EXAMPLE 1 are inoculated at a suitable cell density into liquid medium MX1 containing the desired concentration of a herbicidal AHAS inhibitor. Cells are incubated and grown as in EXAMPLE 1. Cells are subcultured, as appropriate depending on the rate of growth, using fresh medium containing the desired inhibitor concentration after a period of 7-10 days.   ·

Depending on the inhibitor concentration used, cell growth may be slower than in the absence of inhibitor.

The following herbicidal AHAS inhibitors are used in concen trations of 20 ppb, 50 ppb, 100 ppb, 500 ppb, and 1000 ppb: SU872 and SU 464.

EXAMPLE 4: Production of tobacco cells with enhanced levels of AHAS enzyme

In order to obtain cell cultures or callus with enhanced levels of AHAS enzyme, suspension cultures or callus are transferred, in a step-wise manner, to increasingly higher concentrations of herbicidal AHAS inhibitor. In particular, the following steps are performed:

Cell colonies emerging from plated cells of EXAMPLE 2 are transferred to liquid MX1 medium containing the same concentration of AHAS inhibitor as used in the selection according to EXAMPLE 2 in order to form suspension cultures. Alternatively, selected cell suspension cultures of EXAMPLE 3 are subcultured in liquid MX1 medium containing the same concentration of AHAS inhibitor as used for selection according to EXAMPLE 3.

Cultures are subcultured 1-10 times at weekly intervals and are then subcultured into MX1 medium containing the next higher herbicide concentration. The cells are cultured for 1-10 subcultures in medium containing this higher concentration of herbicide. The cells are then transferred to MX1 medium containing the next higher concentration of herbicide. By repeating this procedure, cells are moved, in a stepwise manner, from 50 to 100 ppb of SU464, then to 100 ppb of SU872 and subsequently to 500, then to 1000, and then to 5000 ppb of SU872.

Alternatively, pieces of selected callus of EXAMPLE 2 are transferred to solidified MX1 medium supplemented with the desired herbicide concentration. Transfer to higher herbicide concentrations follows the procedure outlined in the preceeding paragraph except that solidified medium is used.

EXAMPLE 5: Measuring herbicide dose-dependent growth of cells in suspension cultures

In order to obtain a dose-response curve the growth of cells at different concentrations of herbicide is determined. Suspension culture cells of herbicidal AHAS inhibitor sensitive wild-type tobacco cells 53 and herbicide tolerant selected or transgenic cells are pregrown in liquid medium as in EXAMPLE 1 at a high cell density for 2-4 days. The cells are washed free of spent medium and fresh medium without herbicide is added to give the desired cell density (about 150 mg FW cells per ml of suspension). A sample of 2.5 ml of cell suspension, containing approx. 250-300 mg FW cells, is then inoculated into approx. 30 ml of liquid medium of desired herbicide concentration contained in a 100 ml Erlenmeyer flask. Care is taken to inoculate the same amount of cells into each flask. Each flask contains an equal volume of medium. 3-6 replicate flasks are inoculated per herbicide concentration. The herbicide concentration is selected from zero (= control), 0.1 ppb, 0.3 ppb, 1 ppb, 3 ppb, 10 ppb, 30 ppb, 100 ppb, 300 ppb, 1000 ppb, 3000 ppb, and 10,000 ppb. Several samples of inoculated cells are also taken at the time of inoculation to determine the mass of cells inoculated per flask.

Cells are then incubated for growth under controlled conditions at 28°C in the dark for 10 days. The cells are harvested by pouring the contens of each flask onto a filter paper disk attached to a vacuum suction device to remove all liquid and to obtain a mass of reasonably dry fresh cells. The fresh mass of cells is weighed. The dry weight of the samples may be obtained after drying.

Cell growth is determined and expressed as cell gain within 10 days and expressed as a percentage relative to cells grown in the absence of herbicide according to the formula: (final mass of herbicide-grown cells minus inoculum mass x 100 divided by final mass of cells grown without herbicide minus inoculum mass). $IC_{50}$ values are determined from graphs of plotted data (relative cell mass vs. herbicide concentration). $IC_{50}$ denotes the herbicide concentration at which cell growth is 50 % of control growth (cells grown in the absence of herbicide).

In a modification of the method several pieces of callus derived from a herbicide resistant cell culture, as obtained in EXAMPLES 2 and 4, are transferred to solidified callus culture medium containing the different herbicide concen. trations. Relative growth is determined after a culture period of 2-6 weeks by weighing callus pieces and comparing to a control culture grown in medium without herbicide.

However, the suspension method is preferred for its greater accuracy.

EXAMPLE 6: Determination of cross tolerance

In order to determine the extent at which cells show tolerance to analogous or other herbicides, EXAMPLE 5 is repeated by growing cells in increasing concentrations of chosen herbicides. The relative growth of the cells and their $IC_{50}$ value is determined for each herbicide for comparison.

EXAMPLE 7: Determining the stability of the herbicide tolerance phenotype over time

In order to determine whether the herbicide tolerant pheno-type of a cell culture is maintained over time, cells are transferred from herbicide containing medium to medium without herbicide. Cells are grown, as described in EXAMPLE 1, in the absence of herbicide for a period of 3 months, employing regular subculturing at suitable intervals (7-10 days for suspension cultures; 3-6 weeks for callus cultures). A known quantity of cells is then transferred back to herbicide containing medium and cultured for 10 days (suspension cultures) or 4 weeks (callus cultures). Relative growth is determined as in EXAMPLE 5.

EXAMPLE 8: Isolation and fusion of tobacco protoplasts

In order to obtain a plant from a non-regenerable cell culture cell which is herbicide tolerant, the method of protplast fusion and somatic hybridization is used. The herbicide tolerant non-regenerable cell culture serves as one fusion parent. The second fusion parent are protoplasts isolated from a regenerable tissue or a regenerable cell culture of the same plant species.

a) Protoplast isolation from herbicide-tolerant tobacco cell culture:

Protoplasts are isolated from herbicide tolerant cultured cells by incubating the cells in an osmotically buffered enzyme solution consisting of 1 % Macerozyme and 1 % Cellulase R10 in 0.5 M mannitol, 10 mM $CaCl_2$, and 5 mM MES, pH 5.8. The protoplast-enzyme mixture is poured through a 100 µm stainless steel screen. Protoplasts in the filtrate are washed free of enzyme by repeated flotation or centrifugation on sucrose (0.5 M) followed by resuspending in a wash solution containing 0.5 M mannitol, 10 mM $CaCl_2$, and 5 mM MES, pH 5.8. Protoplast are counted in a hemocytometer and diluted to a density of about $0.5 \times 10^{-6}$ per ml.

b) Protoplast isolation from tobacco leaf tissue:

Protoplasts are isolated from leaf tissue of in vitro grown tobacco plants by incubating pieces of leaves in the enzyme solution of EXAMPLE 8a. Protoplasts are washed, counted and resuspended as in EXAMPLE 8a.

c) Protoplast fusion:

After purification, the protoplasts from EXAMPLE 8a and 8b are mixed in equal numbers. To induce fusion, about 100 µl of the mixed protoplast suspension is placed on a glass cover slip and an equal amount of 40 % (w/v) polyethylene glycol (PEG, molecular weight 4000) is added. After incubation for 10 min, a wash solution (0.4 M mannitol, 50 mM CaCl2, pH 8) is added in steps of 100 μl at 5 min intervals over a period of 30 min. The protoplast fusion mixture is then washed in wash solution to remove the PEG and finally resuspended in protoplast culture medium.

d) Protoplast culture:
Protoplasts are diluted to a density of 0.4-1 x $10^5$ per ml and are cultured in a thin layer of liquid protoplast culture medium in the dark at about 28°C. After about 2 weeks, when cell colonies have reformed from the protoplasts, liquid culture medium of reduced osmolality (0.2 M mannitol) is added. As colonies grow rapidly cultures are further diluted with liquid MX1 culture medium.

Alternatively, protoplasts of step c) are mixed, at a final protoplast density of 0.4-1 x $10^5$ per ml, with an equal volume of warm protoplast medium containing 1.2 % molten agarose or 1.4 % agar as described by R.D. Shillito et al., Plant Cell Reports 2: 244-247 (1983). The mixture is poured in a layer, about 2 mm thick, into a 6 cm Petri dish, gently swirled for mixing and allowed to gel. The embedded proto plasts are incubated in the dark at about 28°C until small colonies are formed.

EXAMPLE 9: Selection of herbicide tolerant hybrid colonies and regeneration of somatic hybrid tobacco plant shoots
The selection of herbicide tolerant hybrid plants is accomplished in either a one-step or a two-step selection procedure.

a) Two-step procedure:
In the first step, a selection for herbicide tolerance is made. Cells and small cell colonies formed from the cultured protoplast fusion mixture of EXAMPLE 8 after 2-4 weeks of culture are washed with and transferred to selection medium MX1 containing 30 ppb of SUB72 which is sufficient to inhibit growth of the sensitive wild-type cells derived from sensitive tobacco protoplasts of EXAMPLE 8b. The suspended cells are spread as a thin layer in a Petri dish and are cultured in the dark at about 28°C for about 2 weeks. Alternatively, cells are cultured in a larger volume, 20-100 ml, in a flask under agitation.

In the next step, cells and cell colonies growing in the herbicide containing medium MX1 are washed with liquid MSBN medium and then plated onto solidified MSBN medium. The MSBN medium contains 30 or 100 ppb of SU872. Alternatively no herbicide is added to the medium. Cultures are incubated for 3-6 weeks in the light (approx. 3000 lux from cool white fluroescent tubes) at about 28°C until green shoots are formed.

b) One-step procedure:
The protoplast fusion mixture of EXAMPLE 8 is cultured for about 4 weeks until cell colonies of at least 1 mm diameter are formed. The colonies are washed with liquid MSBN medium and plated on top of solidified MSBN medium which contains 30 -100 ppb of SU872. Cultures are incubated for 3-6 weeks at about 28°C in the light (approx. 3000 lux from cool white fluorescent tubes) until green shoots are formed.

In a further modification, cultured protoplast fusion mixtures embedded in solidified medium according to EXAMPLE 8d are used for selection. When small cell colonies arise from the embedded cells after 2-4 weeks of culture, the agar or agarose medium is cut into several sectors. One ofthe sectors is transferred to about 30 ml of liquid culture medium MX1 to serve as a control to determine the extent of colony formation (plating efficiency). The other sectors are transferred to 30 ml culture medium MX1 containing 30 ppb SU872. These cultures are incubated in the dark on a shaker under gentle agitation until prominent cell colonies are formed. The liquid medium is changed at weekly intervals. Prominent colonies capable of growing in the presence of SU872 are cut out of the solidified medium sector and transferred to fresh solidified MX1 medium containing 30 or 100 ppb of SU872 to grow callus. Pieces of callus (about 100-200 mg FW) are then transferred to solidified MSBN medium containing 30 or 100 ppb of SU872. Cultures are incubated for 3-6 weeks at about 28°C in the light (approx. 3000 lux from cool white fluorescent tubes) until green shoots are formed.

EXAMPLE 10: Regeneration of hybrid tobacco plants
Green shoots formed according to EXAMPLE 9 sufficiently well developed (1-3 cm tall) are transferred to OMS medium to promote further growth, shoot elongation and rooting. When plantlets are developed sufficiently well, they are transferrred to a potting compost mixture and acclimatized to growing in soil.

Plants or parts thereof are analyzed for their herbicidal tolerance properties according to EXAMPLE 11. Progeny is obtained by using standard methods of open or controlled pollination.

EXAMPLE 11: Determining herbicide tolerance of plant cells and plants

a) Leaf disc method:
Leaf discs of 4-10 mm from in vitro grown plantlets or from soil-grown plants of EXAMPLE 10, 23 or 24 are placed on MSBN medium containing a pre-selected amount (for example zero ( = control), 10 ppb, 100 ppb, 1000 ppb, or 10,000 ppb) of the herbicidal AHAS inhibitor to be tested. After 3-6 weeks of growth in light, the cultured tissue pieces are evaluated for their ability to form green shoots in the presence of the inhibitor. In analogy, sections from stems, petioles, roots, cotyledons, and hypocotyls are used.

20

b) Cell culture method:

Pieces of tissue are explanted from the plants of EXAMPLE 10, 23 or 24 and placed in MX1 medium. After a callus has formed, it is subcultured using either liquid or solidified medium MX1 for 3-6 weeks. These cells are tested according to the method of EXAMPLES 5 and 6.

c) Application of inhibitor to plants:

1) pre-emergent application: An inhibitor solution of pre-selected concentration is applied to the substrate into which seeds of the test plant have been sown.

2) post-emergent application: Plants are grown in seed flats, pots or in soil in a greenhouse, phytotron or in a field. An inhibitor solution of pre-selected concentration is sprayed to cover the shoots. A wetting agent is included for better contact. A defined volume of inhibitor solution is used or the plants are sprayed to run-off.

3) post-emergent application to soil: After plants are established in their substrate, a known volume of inhibitor solution of a pre-selected concentration is applied to the soil.

Damage from the inhibitor is evaluated using standard methods of scooring, by determining the number of non-germinated plants or determining damaged leaf area or number of killed plants.

d) Seed plating method:

This method is the preferred one to test the genetic segregation of traits in the progeny of inhibitor tolerant plants. Seeds are sterilized by a standard procedure and then plated onto solidified OMS culture medium which contains a pre-selected concentration of inhibitor. Seed germination, bleaching or kill are evaluated after a period of 2-6 weeks.

e) Seedling pouch test:

This method is particularly suitable for testing the inhibitor tolerance of corn seed, to determine the level of tolerance (dose-response curve), the cross tolerance and to determine segregation of inhibitor tolerance in progeny of inhibitor tolerant plants.

Seeds are placed on germination paper (25x38 cm), about 2.5 cm from the top and 2.5 cm apart, ten in a row. The paper is wetted with a solution of 1 mM $Ca(SO_4)_2$ containing the desired concentration of herbicidal AHAS inibitor. A second paper is applied on top and the two papers rolled to form a wick such that the seeds are about 2.5 cm from the top of the wick. At least 2 replicate wicks are prepared per seed lot to be tested. At least two rolls are prepared with germination solution not containing a herbicidal AHAS inhibitor in order to serve as control. The paper rolls are placed upright into 4 l palstic buckets containing 1.5 l of the germination solution. 2.25 g Captan® fungicide (Dragon Chemicals, Roanoke, Virginia) is added to the germination medium to reduce fungal contamination. A plastic bag is strapped over the bucket to maintain humidity. The bucket is incubated at 28°C under low light (16/8 h photoperiod) at 85 % relative humidity for 7 days. Root and shoot length are measured of the seedlings.

EXAMPLE 12: Induction and culture of embryogenic callus from corn scutellum tissue

Ears are harvested from self pollinated corn plants of the inbred line Funk 2717 (Funk Seeds International, Greensboro, NC) 12-14 days post pollination. Husks are removed and the ears are sterilized for about 15 minutes by shaking in a 20 % solution of commercial chlorox bleach with some drops of detergent added for better wetting. Ears are then rinsed several times with sterile water. All further steps are performed aseptically in a sterile air flow hood. Embryos of 1.5-2.5 mm length are removed from the kernels with spatula and placed, embryo axis downwards, onto MS culture medium containing 2 mg/l 2,4-dichlorophenoxyacetic acid (2,4-D) and 3 % sucrose, solidified with 0.24 % Gelrite®.

Embryogenic callus forms on the scutellum tissue of the embryos within 2-4 weeks of culture at about 28°C in the dark. The callus is removed from the explant and transferred to fresh solidified MS medium containing 2 mg/l 2,4-D. The subculture of embryogenic callus is repeated at weekly intervals. Only callus portions having an embryogenic morphology are subcultured.

EXAMPLE 13: Selection of corn cell cultures tolerant to herbicidal AHAS inhibitors

a) Selection using embryogenic callus:

Embryogenic callus of EXAMPLE 12 is transferred to callus maintenance medium consisting of N6 medium containing 2 mg/l 2,4-D, 3 % sucrose and 30 ppb SU872, and solidified with 0.24 % Gelrite®. Cultures are incubated at 28°C in the dark. After 14 days growing callus is transferred to fresh medium of the same composition. Only cultures with the desired embryogenic morphology known as friable embryogenic callus of type II morphology are subcultured. Cultures are propagated by subculturing at weekly intervals for two to ten subcultures on fresh medium whereby only the fastest growing cultures are subcultured. The fast growing callus is then transferred to callus maintenance medium containing 100 ppb SU872. When callus grows well on this herbicide concentration, ususally after about five to ten weekly subcultures, the callus is transferred to callus maintenance medium containing 300 ppb SU872 and subcultured until a well growing culture is

21

obtained. This process is repeated using medium containing 1000 ppb of SU872, and again with medium containing 2000 and 4000 ppb.

When sufficient callus has been produced it is transferred to regeneration medium suitable for embryo maturation and plant regeneration as described in EXAMPLE 14. Embryogenic callus growing on each of the herbicide concentrations used is transferred to regeneration medium.

b) Selection using embryogenic suspension cultures:

Embryogenic suspension cultures of corn Funk inbred line 2717 are established according to EXAMPLE 24 and maintained by subculturing 2 mg/l 2,4-D. For selection, the cultures are transferred to liquid N6 medium containing 2 mg/l 2,4-D and 10 ppb of SU872. Cultures are grown on a shaker at 120 rpm at 28°C in the dark. At weekly intervals, the medium is removed and fresh medium added. The cultures are diluted with culture medium in accord with their growth to maintain about 10 ml of packed cell volume per 50 ml of medium. At each subculture, cultures are inspected and only fast growing cultures withthe desired friable embryogenic morphology are retained for further subculture. After two to ten subcultrues in N6 medium containing 10 ppb of SU872, cultures are increasing in growth rate at least two- to threefold per weekly subculture. Cultures are then transferred to N6 medium containing 2 mg/l 2,4-D and of 30 ppb SU872. Growing cultures are repeatedly subcultured in this medium for another two to ten subcultures as described above. Fast growing cultures withthe desired friable embryogenic morphology are selected for further subculture. Fast growing cultures are then transferred to N6 medium containing 2 mg 2,4-D and 100 ppb of SU872 and the process of subculturing growing cultures with the desired friable embryogenic morpholgy is repeated for two to ten subcultures until fast growing cultures are obtained. These cultures are then transferred to N6 medium containing 2 mg/l 2,4-D and 300 ppb of SU872.

For regeneration of plants from each embryogenic suspension culture selected with the mentioned herbicide concentration level, the cultures are first transferred onto N6 medium solidified with 0.24 % Gelrite® and containing 2 mg/l 2,4-D and, optionally, the concentration of SU872 in which the cultures have been growing, to produce embryogenic callus. The embryogenic callus is subcultured onto fresh callus maintenance medium until a sufficient amount of callus is obtained for regeneration. Only cultures with the desired embryogenic morphology are subcultured.

EXAMPLE 14: Regeneration of corn plants form selected callus or suspension culture

Plants are regenerated from the selected embryogenic callus cultures of EXAMPLE 13 by transferring to fresh regeneration medium. Regeneration media used are: 0N6 medium consiting of N6 medium lacking, 2,4-D, or N61 consisting of N6 medium containing 0.25 mg/l 2,4-D and 10 mg/l kinetin (6-furfurylaminopurine), or N62 consisting of N6 medium containing 0.1 mg/l 2,4-D and 1 mg/l kinetin, all solidified with 0.24 % Gelrite®. Cultures are grown at 28°C in the light (16 h per day of 10-100 $\mu$Einsteins/m$^2$sec from white fluorescent lamps). The cultures are subcultured every two weeks onto fresh medium. Plantlets develop within 3 to 8 weeks. Plantlets at least 2 cm tall are removed from adhering callus and transferred to root promoting medium. Different root promoting media are used. The media consist of N6 or MS medium lacking vitamins with either the usual amount of salts or with salts reduced to one half, sucrose reduced to 1 g/l, and further either lakcing growth regulating compounds or containing 0.1 mg/l $\alpha$-naphthaleneacetic acid. Once roots are sufficiently developed, plantlets are transplanted to a potting mixture consisting of vermiculite, peat moss and garden soil. At transplanting all remaining callus is trimmed away, all agar is rinsed off and the leaves are clipped about half. Plantlets are grown in the greenhouse initially covered for some days with an inverted clear plastic cup to retain humidity and grown with shading. After acclimatization plants are repotted and grown to maturity. Fertilizer Peters 20-20-20 is used to ensure healthy plant development. Upon flowering plants are pollinated, preferably self pollinated.

EXAMPLE 15: Isolation of the wild-type Arabidopsis AHAS gene

a) Isolation of Arabidopsis plant DNA:

Total plant DNA is isolated from 5 weeks old Arabidopsis thaliana ecotype Columbia with the following procedure: 40 g of leaf and stalk tissues are harvested into liquid nitrogen. The frozen tissue is ground to a fine powder with mortar and pestle chilled on dry-ice. The powder is resuspended in 200 ml of an extraction buffer (50 mM Tris-HCl, pH 8, 50 mM EDTA, 50 mM NaCl, 2 % sodium sarkosinate, 100 $\mu$g/ml proteinase K). The slurry is thawed, mixed and incubated at 55°C for 1 hour, then centrifuged at 10,000 xg for 15 minutes. The supernatant is mixed with 2 volume of ethanol, and centrifuged at 10,000 x g for 15 minutes. The DNA-containing pellet is dissolved in 64.4 ml of 10 X TE buffer. To this solution 62.2 g CsCl and 2.9 ml of 10 mg/ml ethidium bromide are added, and the mixture is heated in a 55°C waterbath for 5 minutes to dissolve the CsCl. The CsCl solution is clarified by centrifugation at 8,000 x g for 45 minutes, loaded into VTi50 "Quick-seal" centrifuge tube, and centrifuged at 45,000 rpm in Beckman VTi50 rotor at 20°C for 18 hours. The fluorescent DNA band is pulled from the tube with a syringe tipped with a 16 gauge needle, and extracted 5 times with isopropanol saturated with 20 X SSC to remove the ethidium bromide. The resulting solution is diluted with 2 volumes of water and 0.1 volumes of 3 M sodium acetate, pH 4.8, and mixed with 2 volumes of ethanol. The DNA fiber is spooled out of the solution with the tip of a Pasteur pipette, rinsed in 70 % ethanol, air dried, dissolved in 0.5 ml of TE buffer, and stored at 4°C.

22

b) Isolation of Xbal insert DNA:

250 μg of Arabidopsis DNA is digested with 4000 units of the restriction enzyme Xbal for 4 hours. The digest is fractionated by electrophoresis on a 0.8 % agarose gel. The DNA fragments between 6.5 kbp and 4.36kbp (based upon HindIII-digested lambda DNA size markers) are isolated from the gel using NA-45 DEAE membrane (Schleicher and Schuell, Keene, NH) following the manufacturer's recommended procedures. The DNA is dissolved in 20 μl of TE buffer, and the concentration of the solution is determined with UV spectrophotometry.

c) Construction of the lambda library:

Xbal digested and phosphatase-treated lambda ongC (longC) arm preparation is obtained from Stratagene Cloning Systems (La Jolla, CA). 0.1 μg of the Arabidopsis Xbal insert DNA is ligated to 1.0 μg of the longC arms according to procedures recommended by Stratagene. 2.5 μl of the ligation reaction is packaged in lambda phage heads using the Gigapack Plus kit (Stratagene Cloning Systems) following procedures recommended by the manufacturer. The activity of the library is titered on E. coli strain VCS257 (Stratagene Cloning Systems).

d) Preparation of the yeast AHAS probe:

50 μg of pE16/8-c4 plasmid DNA (J. Polaina, Carlsberg Res. Comm. 49: 577-584, 1984, deposited at ATCC, see above) are digested with EcoRI, and the digest products are resolved on a 0.8 % agarose gel. The 2 kbp EcoRI fragment containing the coding sequence for the yeast AHAS gene is isolated from the gel using NA-45 DEAE membrane following procedures recommended by the manufacturer.

Radiolabeled probes to the 2 kbp fragment are synthesized on the day of the hybridization reaction with the random priming reaction using Prime Time kit (International Biotechnologies Inc., New Haven, CT), following procedures recommended by the manufacturer. The labeling reaction is scaled up to 125 μl and contains 0.25 μg of the fragment and 125 μCi of 3000 Ci/mMol $\alpha$-$^{32}$P dCTP. 50 μg of yeast tRNA are added to the reaction as carrier and the DNA is precipitated with 125 μl of 5 M ammonium acetate and 500 μl of ethanol. The reaction mixture is frozen in dry ice, thawed, and centrifuged for 15 min at top speed in a microfuge (Brinkman). The pellet is dissolved in 200 μl of TE buffer, and the radiolabeled DNA probe is further cleaned of unincorporated nucleotides with a run over a G50 Sephadex Quick Spin column (Boehringer Mannheim Biochemicals, Indianapolis, IN) following the manufacturer's recommended procedures. The DNA probe is denatured by heating for 5 minutes in a boiling waterbath, chilled, and added to the hybridization solution.

e) Screening of the lambda library:

250,000 recombinant phages are used to infect E. coli strain VCS257 and plated at a density of 50,000 phages per 150 mm Petri plate on 5 plates. Duplicate membrane lifts of the phage plaques are made using Colony/Plaque Screen membranes (NEN Research Products, Du Pont, Boston, MA) following procedures recommended by the manufaturer. The membranes are incubated overnight in wash buffer (5 X SSC, 2 % SDS). The membranes are then incubated at 42°C with gentle shaking in 150 ml of LS hybridization buffer. After 16 hours the membranes are transferred to 150 ml of fresh LS hybridization buffer containing 0.25 μg of $^{32}$P-labeled yeast AHAS gene probe prepared as described above. The membranes are incubated for 16 hours at 42°C with gentle shaking, washed twice at room temperature with 2 X SSC for 15 minutes per wash, washed three times at 42°C in LS wash buffer (25 % formamide, 5 X SSC, 1 % SDS) for 2 hours per wash, and washed twice in room temprature rinse buffer (0.1 X SSC, 0.1 % SDS) for 30 minutes per wash. The membranes are mounted and fluorographed with Cronex Lightening Plus Screens (Du Pont, Wilminton, DE) and XAR-5 film (Kodak, Rochester, NY). The plaques from regions of the plates that gave positive film signals on both membrane lifts are picked and replated at a low density of approximately 300 plaques per 150 mm plate, and the screening process is repeated until single hybridizing plaques are isolated.

f) Subcloning of the Arabidopsis AHAS gene onto plasmid vectors:

Miniprep of phage DNA from the plaque-purified phage is carried out following procedures accompanying the LambdaSorb Phage Adsorbent kit (Promega, Madison, WI). The phage DNA is cut with restriction enzyme Xbal and the 5.8 kbp insert fragment is cloned into the Xbal site of pBS(-) plasmid (Stratagene Cloning Systems). The identity of the cloned fragment with the Xbal fragment containing the Arabidopsis AHAS gene is verified by comparing its restriction map and DNA sequence with those published for the Arabidopsis AHAS gene by G. Haughn et al., Mol. Gen. Genet. 211: 266-271 (1988), and B. Mazur et al., Plant Physiol. 85: 1110-1117 (1987). This plasmid is designated pCIB1207.

EXAMPLE 16: Construction of a CaMV 35S wild-type Arabidopsis AHAS chimeric gene

a) Source and modification of the CaMV 35S promoter cassette:

The CaMV 35S promoter fusion site in the PCIB710 plant expression vector (S. Rothstein et al., Gene 53: 153-161, 1987) is converted from BamHI to Ncol as follows: The Ncol site in the fragment containing the CaMV 35S promoter is destroyed by cutting the PCIB710 plasmid DNA with Ncol, filling in the protruding ends with the Klenow fragment of E. coli DNA polymerase (Klenow DNA polymerase), and recircularizing the plasmid with T4 DNA ligase. The resulting plasmid is cut with BamHI. The protruding ends are filled in with Klenow DNA polymerase, and NcoI linkers (New England Biolabs, Beverly, MA) are ligated to the ends with T4 DNA ligase to form pCIB1211.

b) Insertion of the Arabidopsis AHAS coding sequence into the CaMV 35S promoter cassette and construction of a binary vector:

The XbaI site 1.3 kbp downstream of the Arabidopsis AHAS coding sequence in pCIB1207 is converted into a NcoI site by cutting with XbaI, filling in the protruding ends with Klenow DNA polymerase, and adding in NcoI linkers (New England Biolabs) with T4 DNA ligase. This is followed by cutting with NcoI to release the 3.3 kbp NcoI fragment containing the AHAS coding sequence from the plasmid sequence. The digest is ligated with NcoI-cut pCIB1211 DNA and transformed into E. coli strain JM109 (C. Yanisch et al., Gene 33: 103-119, 1985) cells. Miniprep plasmid DNA is prepared from colonies showing ampicillin resistance and digested with XbaI and SacII restriction endonucleases. The digest products are analyzed by gel electrophoresis to identify a clone containing a recombinant plasmid that has fused the 5' end of the AHAS coding sequence directly downstream of the pCIB1211 CaMV 35S promoter expression site. This plasmid is designated pCIB1212.

The 35S promoter AHAS chimeric gene is excised from pCIB1212 by cutting with XbaI and KpnI restriction enzymes, and ligated into XbaI and KpnI cut PCIB200. This gives the binary vector pCIB1213 containing the chimeric CaMV 35S driven Arabidopsis AHAS gene.

d) Construction of PCIB200:

TJS7SKan is first created by digestion of PTJS75 (Schmidhauser and Helinski, J. Bacteriol. 164: 446-455, 1985) with NarI to excise the tetracylcine gene, followed by insertion of an AccI fragment from pUC4K (J. Messing and J. Vierra, Gene 19: 259-268, 1982) carrying a NptI gene. pCIB200 is then made by ligating XhoI linkers to the EcoRV fragment of pCIB7 (containing the left and right T-DNA borders, a plant selectable nos/nptII chimeric gene and the pUC polylinker, see S.J. Rothstein et al., Gene 53: 153-161, 1987) and cloning XhoI digested fragment into SalI digested TJS7SKan.

d) Conjugal transfer of the binary vector from E. coli to A. tumefaciens:

The binary vector pCIB1213 is transferred from E. coli to A. tumefaciens by the triparental mating procedure of Ditra et al., Proc. Nat. Acad. Sci. USA 77: 7347-7351, 1980. Stationary cultures of E. coli strain HB101 (E. Releigh and G. Wilson, Proc. Nat. Acad. Sci. USA 83: 9070-9074, 1986) harboring pCIB1213 (50 μl), E. coli strain HB101 harboring the mobilization helper plasmid pRK2013 (ATCC 37159) (50 μl), and A. tumefaciens strain A136 (B. Watson et al., J. Bacteriol. 123: 255-264, 1975) harboring pCIBS42 virulence helper plasmid (100 μl) are mixed and allowed to grow on a LB agar plate for 2 days at 30°C. A. tumefaciens A136 trans-conjugants containing pCIBS42 and pCIB1213 are selected for and purified by three cycles of streaking on MAB minimal agar plates (M. Chilton et al., Proc. Nat. Acad. Sci. USA 71: 3672-3676, 1974) containing spectinomycin and kanamycin both at 50 mg/l. This strain of Agrobacterium tumefaciens is designated CGA1402.

EXAMPLE 17: Isolation of the wild-type tobacco AHAS gene

a) Production of tobacco protoplasts:

100 ml of a 2 days old tobacco cell suspension culture of EXAMPLE 1 is mixed with an equal volume of double strength enzyme solution. Enzyme solution contains 10 g/l Cellulase R.10 Onozuka® (Yakult Honsha, Tokyo, Japan), 2.5 g/l Macerase® (pectinase from Rhizopus sp., Behring Diagnostics, La Jolla, CA), 1 g/l Pectolyase Y-23® (Seishin Pharm. Co., Tokyo, Japan), 1.45 g/l $CaNO_3 \cdot 4H_2O$, 0.5 g/l $MgSO_4 \cdot 7H_2O$, 0.23 g/l $NH_4H_2PO_4$, 1.2 g/l $KNO_3$, 0.3 g/l KCl, and 73 g/l D-mannitol, pH 5.70, centrifuged and filtered through a 0.2 μm filter. The mixture is incubated at room temperature on a gently-rotating platform shaker (40 rpm) for 5 hours. At intervals during this digestion, small samples are removed for microscopic examination. The digestion is continued until approximately 80 % of the cells have been converted to spherical protoplasts. The flasks are removed from the shaker and the cell/protoplast suspensions are allowed to settle. The top halves of the medium in the flasks, substantially free of cells and protoplasts, are aspirated off with a pipette and discarded. The remaining digestion mixtures are transferred to sterile 50 ml centrifuge tubes and centrifuged for 10 minutes at 500 rpm in a clinical centrifuge (Model HN-SII, IEC). Pellets containing the protoplasts are resuspended in Rinse I solution and centrifuged for 10 minutes at 1,000 rpm in the clinical centrifuge. Rinse I solution consists of sucrose (154 g/l), MES (0.59 g/l), $KNO_3$ (250 mg/l), $NH_4NO_3$ (25 mg/l), $NaH_2PO_4 \cdot H_2O$ (15 mg/l), $CaCl_2 \cdot 2H_2O$ (90 mg/l), $MgSO_4 \cdot 7H_2O$ (25 mg/l), and $(NH_4)_2SO_4$ (13.4 mg/l). The protoplasts band at the top of centrifuge tube, while debris and intact cell are pelleted at the bottom of the tube. The protoplast fraction is collected and the centrifugation in Rinse 1 solution is repeated. The protoplasts fraction is transferred to centrifuge tubes and stored on ice until use.

b) Isolation of tobacco DNA:

100 ml of ice-cold protoplast suspension are mixed with 400 ml of ice-cold TENP buffer to lyse the protoplasts. Nuclei are pelleted from the lysate with a 10 min 2,000 rpm spin in an IEC clinical centrifuge. The nuclear pellet is resuspended in 500 ml of ice-cold TENP and pelleted once more as described above. Eight CsCl gradient tubes are prepared, each by suspending the nuclear pellet from approximately 12.5 ml of protoplast suspension to 26 ml with 10 X TE buffer, adding 5 ml of 20 % (w/v) sodium lauroylsarkosine to lyse the nuclei, adding 32.2 g of CsCl and 2.89 ml of 10 mg/ml ethidium bromide (EtBr) to each lysate, and gently mixing to dissolve the CsCl. The lysates are loaded into polyallomer tubes (Beckman VTi50, 39 ml tubes) and spun at 45,000 rpm for 16 hours at 20°C in a VTi50 rotor (Beckman). UV fluorescent bands are pulled from the gradients using 3 ml syringes with 16 gauge needles and pooled. The DNA is further purified by repeating the CsCl/EtBr equilibrium centrifugation in VTi50. The UV fluorescent bands are pulled from the gradient and cleared of EtBr by 6 cycles of extraction with an equal volume of isopropanol saturated with 20 X SSC. The DNA is precipitated from the cleared gradient solution by adding in succession 2 volumes of distilled water, 0.1 volumes of 3.0 M sodium acetate, pH 5.4, and 2 volumes of ethanol. The DNA fiber is spooled out of solution with a Pasteur pipette and washed in 70 % ethanol, then dissolved in 30 ml of TE buffer and extracted with an equal volume of 1:1 phenol:chloroform mix. The aqueous phase is extracted further with an equal volume of chloroform and the DNA precipitated with additions of 0.1 volumes of 3 M sodium acetate, pH 5.4, and 2.0 volumes of ethanol. The DNA fiber is spooled out, washed in 70 % ethanol and air dried for 5 min, then dissolved in a total of 7 ml of TE buffer and stored at 4°C until use.

c) Construction of lambda genomic bank of tobacco:

The tobacco DNA is partially digested with Sau3A endonuclease, and sedimented through a 10-40 % sucrose gradient in a SW41 rotor (Beckman) at 20,000 rpm for 20 hours. Fractions from the gradient are analyzed on a 0.5 % agarose gel in TBE buffer. Fractions containing DNA fragments in the 12 to 22 kbp size range are pooled. The DNA is precipitated with 1/10 volume of 3 M sodium acetate (pH 4.8) and 2 volumes of ethanol and ligated with BamHI-digested, phosphatase-treated lambda EMBL3 DNA from Stratagene (La Jolla, CA). The ligation is done following the procedures and conditions recommended by Stratagene using 5 µl reactions each containing 1 µg of lambda vector DNA and 0.1 µg of insert DNA. The ligation reaction product is packaged into lambda phage heads using the Gigapack Plus kit (Stratagene) following the instructions of the manufacturer. The phage yield titered on E. coli strain CES201 (DNA cloning, volume 1: a practical approach, D.M. Glover ed., IRL Press, Oxford and Washington DC, p. 33) is approximately 2 x 10⁶ phage per µg of insert DNA.

d) Preparation of radiolabeled probe from the Arabidopsis AHAS coding sequence and screening of the tobacco gene library:

A radioactive probe is prepared from a DNA fragment containing internal coding sequences of the Arabidopsis AHAS gene following the approaches and procedures described in EXAMPLE 15 for the production of radiolabeled probes from the yeast AHAS gene. For this experiment, the DNA fragment spanning SacII to BglII restriction sites within the Arabidopsis AHAS coding region (G. Haughn et al., Mol. Gen. Genet. 211: 266-271, 1988) is isolated from pCIB1207 and used in the production of the radiolabeled probe. 0.5 µg of the Arabidopsis AHAS fragment is labeled with the random priming procedure described in EXAMPLE 15 to a specific activity of 7 x 10⁸ cpm per µg used in the screening of the tobacco lambda library.

A lambda library yielding approximately 1 million plaques on E. coli strain CES201 is plated out and screened with the radiolabeled probe. The procedures and conditions used for the library screening and purification of positive clones are those described for isolating the lambda clones of the Arabidopsis AHAS gene in EXAMPLE 15, except that the volumes of the prehybridization and hybridization reactions used are adjusted proportionally to the number of membrane lifts being screened.

e) Radiolabeling of AHAS gene specific oligonucleotides:

Oligonucleotides with sequences specific to tobacco SuRA and SuRB genes (K. Lee et al., EMBO J. 7: 1241-1248, 1988) are synthesized on the Applied Biosystems Model 380A DNA Synthesizer (Foster City, CA) following standard protocols. The SuRA gene specific oligonucleotide is of the following sequence: 5'TTTTTAGCTTAGTAGTGCTCTCATGTTCTCA3'. The SuRB gene specific oligonucleotide is of the following sequence: 5'TTCAGTTTCGTGGCGCTCTCGTCTTCTCAGA3'. These oligonucleotides are radiolabeled at their 5'-hydroxyl termini with T4 poly nucleotide kinase in the presence of γ-³²P ATP (3000 Ci/mMol) following procedures described by Maniatis et al. (supra, p. 122-123). 0.15 µg of oligonucleotides are treated in a 50 µl reaction containing 100 µCi of ATP label and 20 units of T4 polynucleotide kinase at 37°C for 40 minutes. The radio-labeled oligonucleotides are separated from unicorporated ATP by gel-exclusion chromatography with Sephadex G25 Quick Spin columns (Boehringer Mannheim) and show specific activity of greater than 10⁹ cpm per µg.

f) Identification of the SuRA and SuRB AHAS gene clones:

Membrane lifts of plaques on 100 mm Petri plates are made with 10 lambda clones isolated from the above experiment using procedures described in EXAMPLE 15. The membranes are incubated in 50 ml of ON hybridization buffer (1 M NaCl, 1 % SDS, 10 % w/v dextran sulfate) at 55°C with gentle shaking. After an overnight incubation, 30 ng of end-labeled tobacco SuRA AHAS gene-specific oligonucleotide and 1.7 ml of sheared and heat-denatured salmon sperm DNA are added to the hybridization reaction containing the membranes. The reaction is incubated overnight at 55°C with gentle shaking. The membranes are rinsed with room temperature 2 X SSC for 30 minutes, washed with 2 litres of ON wash buffer (2 X SSC, 1 % SDS) at 55°C for 90 minutes, and rinsed in room temperature 2 X SSC for 15 minutes. The membranes are fluorographed

25

with Cronex Lightening Plus screen (Du Pont) and XAR-5 film (Kodak). After 1 day and 3 day exposures, the same membranes are striped of their annealed radioactive probes by boiling for 15 minutes in 5 buffer (0.1 X SSC and 0.1 % SDS), and the hybridization procedure described above is repeated using the radiolabeled tobacco SuRB AHAS gene probe (see above). Lambda clones containing the tobacco SuRA AHAS gene are identified as those that show strong hybridization signals with SuRA probe and weak or no signals with the SuRB signals, while clones containing the SuRB gene are identified as those showing the inverse. For a verification of the identities assigned by the hybridization experiment, minipreps of phages DNA are prepared from each lambda clone using procedures described in EXAMPLE 15 and analyzed by diestions with BamHI, EcoRI, Ncol, Patl, and Xhol restriction enzymes. The SuRA and SuRB clones are shown to contain the diagnostic restriction fragments characteristics of these genes based upon the published sequences of K. Lee et al., EMBO J. 7: 1241-1248, 1988.

g) Subcloning of the tobacco SuRA and SuRB AHAS genes:
Miniprep DNA of a SuRA lambda clone shown by the above restriction digest analysis to contain the entirety of the SuRA AHAS gene is cut with Xhol, and the 6.5 kbp Xhol fragment containing the AHAS gene is cloned into the Xhol site of plasmid vector pBS(-) (Strategene). Similarly, miniprep DNA of a SuRB lambda clone shown by the above restriction digest analysis to contain the entirety of the SuRB AHAS gene is cut with BamHI and PstI restriction enzymes, and the 6.2 kbp BamHI-PstI fragment is cloned into the BamHI/PstI site of pBS(-) plasmid. The correctness of these clones is verified by diagnostic restriction digests to show the presence of expected restriction fragments. The pBS(-) plasmids containing the wild-type tobacco SuRA and SuRB AHAS gene are designated pCIB1209 and pCIB1210, respectively.

EXAMPLE 18: Construction of a CaMV 355 wild-type tobacco SuRA AHAS chimeric gene:
The Sspl and Stul sites adjacent to the coding sequence of the tobacco SuRA AHAS gene are used for constructing a transcriptional fusion to the CaMV 35S promoter of pCIB710. The sspi site is located 75 bp 5' upstream of the initiator ATG codon of the SuRA coding sequence, and the Stul site is located 30 bp 3, downstream of the TGA termination codon of the SuRA coding sequence (K. Lee et al., EMBO J. 7: 1241-1248, 1988). pCIB1209 is cut with Sspl and Stul, and BamHI linkers (NEB) are attached to the ends of the fragments with T4 DNA ligase. The fragments containing the linkers are digested with BamHI and ligated with BamHI digested pCIB710. The ligation reaction product is transformed into E. coli strain JM 109. Minipreps of plasmid DNA are prepared from kanamycin resistant transformants and digested with BamHI to identify clones containing the 2.1 kbp SuRA AHAS gene insert. The DNA of these clones is further digested with Xbal, which cuts just 5' upstream of the CaMV 35S promoter, and Ncol, which cuts asymmetrically within the insert DNA, in order to identify clones containing the SuRA gene in the orientation that allows for its correct transcription by the CaMV 355 promoter. One such plasmid is designated pCIB1214.

This plasmid is digested with Xbal and Kpnl restriction enzymes, and the Xbal/Kpnl fragment containing the chimeric gene is ligated into Xbal and Kpnl digested pCIB200. The ligation reaction is transformed into E. coli strain HB101 and miniprep plasmid DNA prepared from kanamycin-resistant transformants. The miniprep DNA is digested with diagnostic restriction enzymes to identify colonies with pCIB200 containing the chimeric gene insert. One such plasmid is designated pCIB1215. This plasmid is transferred to Agrobacterium tumefaciens following the procedure described in EXAMPLE 16. The corresponding Agrobacterium tumefaciens A136 strain harboring pCIBS42 and pCIB1215 is designated strain CGA1403.

EXAMPLE 19: Isolation of wild-type maize AHAS gene

a) Isolation of maize DNA:
Maize DNA is isolated from Zea mays elite inbred line Funk 2717 (Funk Seeds International) using the following procedure: 100 grams of etiolated maize seedlings are frozen in liquid nitrogen and ground to a fine powder using a cold mortar and pestle. The resulting powder is mixed with 200 ml of cold extraction buffer (50 mM Tris-HCl, pH 8.0, 50 mM EDTA, pH 8.0, 50 mM NaCl, 2 % sodium N-lauroylsarcosine [Sigma Chemicals, St. Louis, MO], 400 μg/ml ethidium bromide) and stirred for 15 minutes on ice. The resulting solution is clarified by twice centrifuging for 30 minutes at 10,000 rpm in a GSA rotor (Sorval/DuPont, Wilmington, DE) at 4°C. The resulting supernatant is used to prepare a CsCl/ethidium bromide solution consisting of the following: 32.2 ml of supernatant, 31.13 g of CsCl, and 2.9ml of 10 mg/ml ethidium bromide. The resulting solution is heated at 55°C for 5 minutes to dissolve the CsCl completely. After heating, the solution is loaded into VTi50 polyallomer tubes and centrifuged for 18 hours at 20°C at 45,000 rpm in a VTi50 rotor (Beckman).

DNA bands are identified in the CsCl gradient by ultraviolet fluorescence and are collected from the centrifuge tubes using 3 ml syringes and # 16 gauge needles. DNA containing fractions are pooled and further purified by repetition of the CsCl/EtBr equilibrium centrifugation in the VTi50 rotor. Resulting ultraviolet fluorescing bands are pulled from the tubes. Ethidium bromide is removed by six cycles of extraction using an equal volume of isopropanyl alcohol saturated with 20 X SSC. DNA is precipitated from this cleared gradient solution by the addition in succession of: 2 volumes of distilled water, 0.1 volumes of 3.0 M sodium acetate, pH 5.4, and 2 volumes of absolute ethanol. DNA is spooled from the resulting solution using a pasteur pipette, washed with 70 % ethanol, dissolved in 30 ml of TE buffer and extracted with an equal volume of 1:1 phenol:chloroform. The resulting aqueous phase is further extracted using an equal volume of chloroform, and the DNA is precipitated by adding 0.1 volume of 3 M sodium acetate, pH 5.4, and 2.0 volumes of absolute ethanol. DNA is spooled from the resulting solution, washed once in 70 % ethanol, air-dried for 5 minutes, dissolved in 10 ml of TE buffer and stored at 4°C until use.

## b) Construction of genomic library of maize:

A genomic library of maize DNA is prepared by Stratagene Company (La Jolla, CA) using 300 µg of maize DNA prepared as described above. Preparation of the genomic library is in lambda Dash vector (Stratagene). The library contains at least 1 X 10^6 primary recombinant phages constructed from 15 to 30 kbp inserts derived from a Sau3A partial digest of the maize DNA. Inserts are cloned into the BamHI site of lambda Dash. The phage yield is titered on E. coli strain CES201 (DNA cloning Volume 1: a practical approach, D.M. Glover ed., IRL Press, Oxford and Washington DC, p. 33).

## c) Preparation of a radiolabeled probe from the Arabidopsis AHAS coding sequence:

A radioactive probe is prepared from a DNA fragment containing internal coding sequences of the Arabidopsis AHAS gene of EXAMPLE 15. 50 µg of pCIB1207 plasmid DNA is digested with SacII and BglII, and products of the digest are resolved by electrophoresis on a 0.8 % agarose gel. The 1.7 kb DNA fragment which spans the SacII to BglII restriction sites within the Arabidopsis AHAS coding region (G. Haughn et al., Mol.Gen.Genet. 211: 266-271, 1988) is isolated from the gel using a NA45 DEAE membrane (Schleicher and Schuell Inc., Keene, NH) following procedures recommended by the manufacturer. The radiolabeled probe is synthesized on the day of the hybridization reaction by random priming starting with 0.3 µg of the Arabidopsis AHAS fragment and using the Prime Time kit (International Biotechnologies Inc., New Haven, CT), following the manufacturer's procedures accompanying the kit. The labelling reaction is scaled up to 125 µl and contains 0.3 µg of the fragment and 125 µCi of 3000 Ci/mMol α-$^{32}$P dCTP. At the end of the labeling reaction 50 µg of carrier yeast tRNA is added to the reaction, and the DNA is precipitated with 125 µl of 5 M ammonium acetate and 500 µl of ethanol. The reaction mixture is frozen in dry ice, thawed, and centrifuged for 15 min at top speed in a microfuge (Brinkman). The pellet is dissolved in 200 µl of TE buffer, and unincorporated nucleotides are removed from the radiolabeled DNA probe by a G-50 Sephadex Quick Spin column (Boehringer Mannheim Biochemicals, Indianapolis, IN) chromatography, following manufacturer's recommended procedures. The specific activity of the resulting radiolabelled probe equals or is greater than 1 X 10^9 cpm per µg of input DNA. The DNA probe is denatured by heating for 5 minutes in a boiling water bath, chilled on ice, and added to the hybridization solution.

## d) Screening of the lambda library:

800,000 recombinant phages of the maize library are plated on E. coli strain CES201. Sixteen plates at a density of 50,000 phages per 150 mm Petri plate are plated following procedures recommended by Stratagene. Duplicate membrane lifts of the phage plaques are made using Colony/Plaque Screen membranes (NEN Research Products, Du Pont, Boston, MA) following procedures recommended by the manufacturer. The membranes are incubated overnight in wash buffer (5 X SSC, 2 % SDS). The membranes are then incubated at 42°C with gentle shaking in 400 ml of LS hybridization buffer (see below).

After 16 hours of incubation, the membranes are transferred to 400 ml of fresh LS hybridization buffer containing 0.3 µg of the $^{32}$P-labeled Arabidopsis AHAS gene probe prepared as described above. The membranes are incubated with the probe for 16 hours at 42°C with gentle shaking, washed twice with room temperature 2 X SSC for 15 minutes per wash, washed three times with 42°C LS wash buffer (25 % formamide, 5 X SSC, 1 % SDS) for 2 hours per wash, and washed twice in room temperature rinse buffer (0.1 X SSC, 0.1% SDS) for 30 minutes per wash. Membranes are mounted and fluorographed with Cronex Lightening Plus Sceens (Du Pont, Wilmington, DE) and XAR-5 film (Kodak, Rochester, NY). Plaques from regions of the plates that give positive film signals on both membrane lifts are picked for further study. These are replated at a low density of 300 to 500 plaques per 150 mm plate, and the screening process is repeated twice more until hybridizing single plaques are isolated. Three independently derived recombinant phages are purified to the single plaque stage. Two of these phages, LCIB1200 and LCIB1202, are stable in that they give uniform and identical hybridizing plaques upon replating. The third phage LCIB1201 gives rise to both large-and small-sized plaques upon replating. Hybridization withthe Arabidopsis AHAS probe shows that only the small LCIB1201 plaques contain homologous sequences while the large plaques do not. The LCIB1201 phage is apparently unstable and spontaneously gives rise to derivatives which form large plaques and which have deleted AHAS homologous sequences. LCIB 1200 and LCIB 1202 are deposited in the American Type Culture Collection, see above.

## e) Subcloning of maize AHAS gene sequences onto plasmid vectors:

Minipreps of LCIB1200 and LCIB1202 phage DNA from the plaque-purifed phages are carried out following procedures accompanying the LamdaSorb Phage Adsorbent kit (Promega, Madison, WI). The LCIB1200 phage DNA, designated DCIB1200, is digested with restriction enzyme XbaI. The digest fragments are cloned into the XbaI site of Bluescript SK+ plasmid (Stratagene Cloning Systems). A clone containing an insert of a 4.0 kbp XbaI fragment is identified by hybridization using the SacII-BglII Arabidopsis AHAS fragment. This clone contains maize AHAS gene sequences. The resulting plasmid is designated pCIB1253.

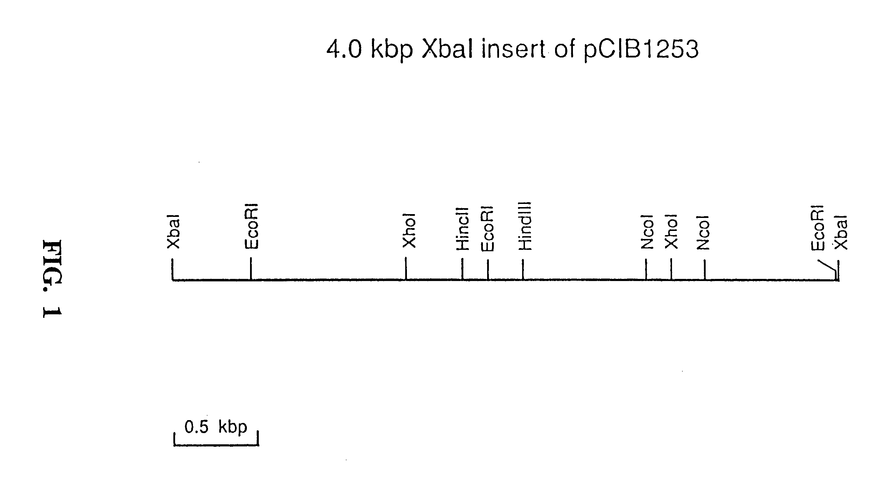

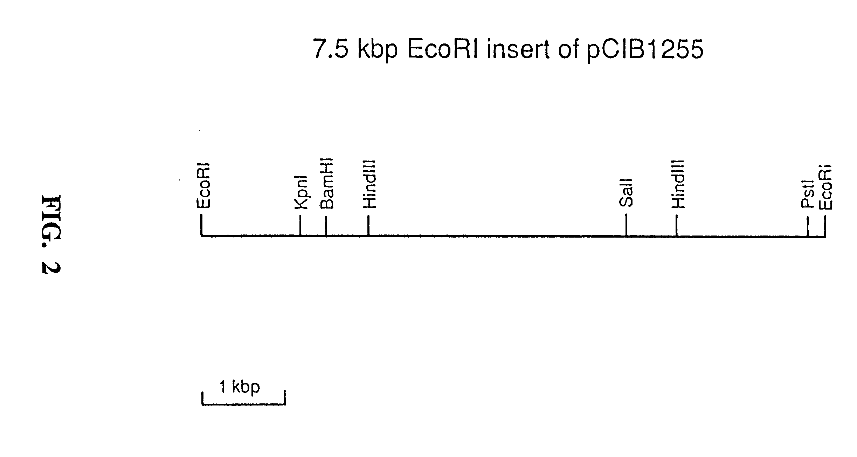

The LCIB1202 phage DNA, designated DCIB1202, is digested with restriction enzyme EcoRI. The digest

27

fragments are cloned into the EcoRI site of Bluescript SK+ plasmid. A clone containing a 7.5 kbp EcoRI fragment insert is identified by hybridization with the SacII-BgIII Arabidopsis AHAS fragment. This clone contains a maize AHAS gene sequence. The plasmid is designated pCIB1255. Plasmid DNA of pCIB1253 and pCIB1255 is digested with various restriction enzymes, analyzed after electrophoresis on agarose gels, and restriction maps are deduced for their inserted DNA. These restriction maps of the DNA insert in pCIB1253 and pCIB1255 are shown in Figures 1 and 2, respectively.

f) Sequencing and identification of the maize AHAS genes in pCIB1253 and pCIB1255:
The DNA sequence of the DNA inserts of pCIB1253 and pCIB1255 are determined with dideoxy sequencing (M. Hattoni and Y. Sakaki, Anal. Biochem. 152: 232-238, 1986).

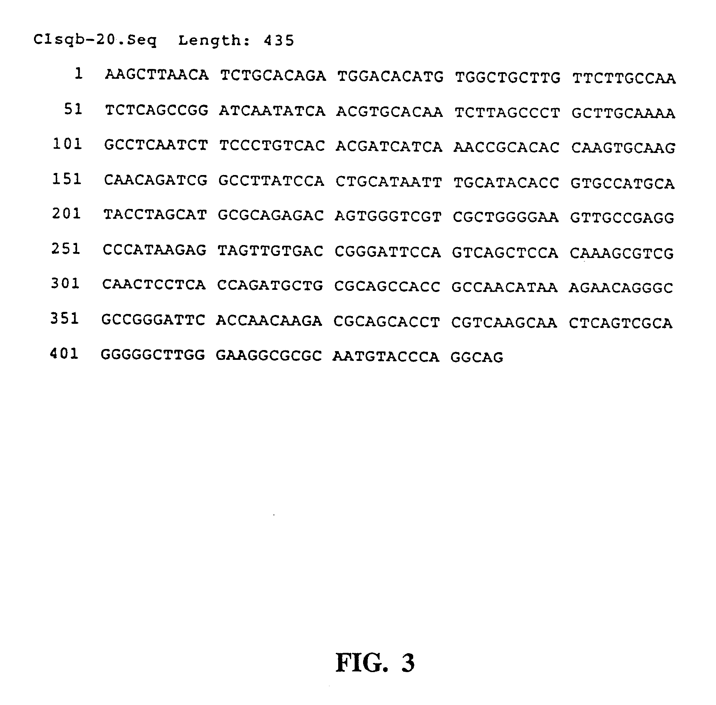

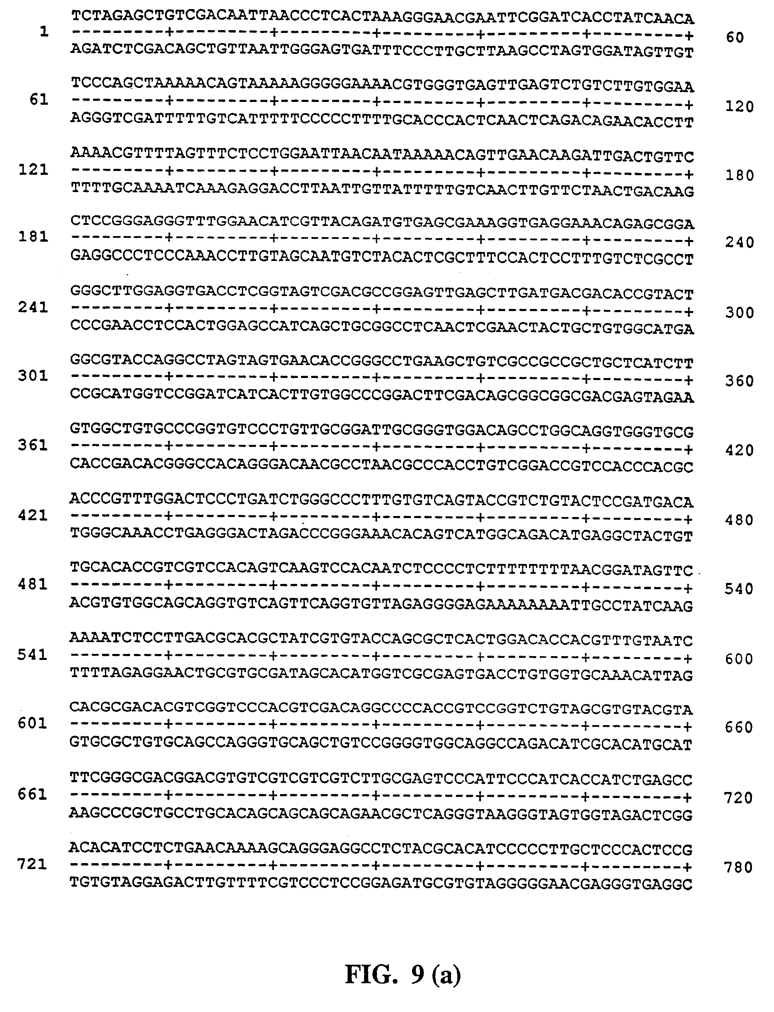

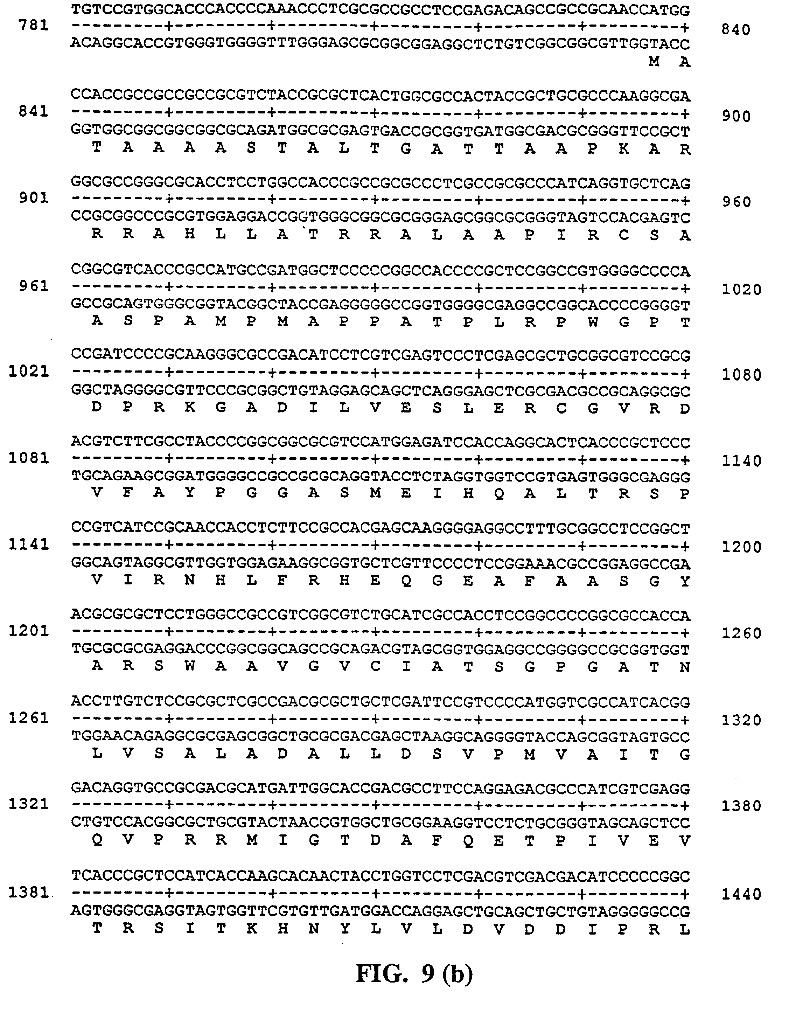

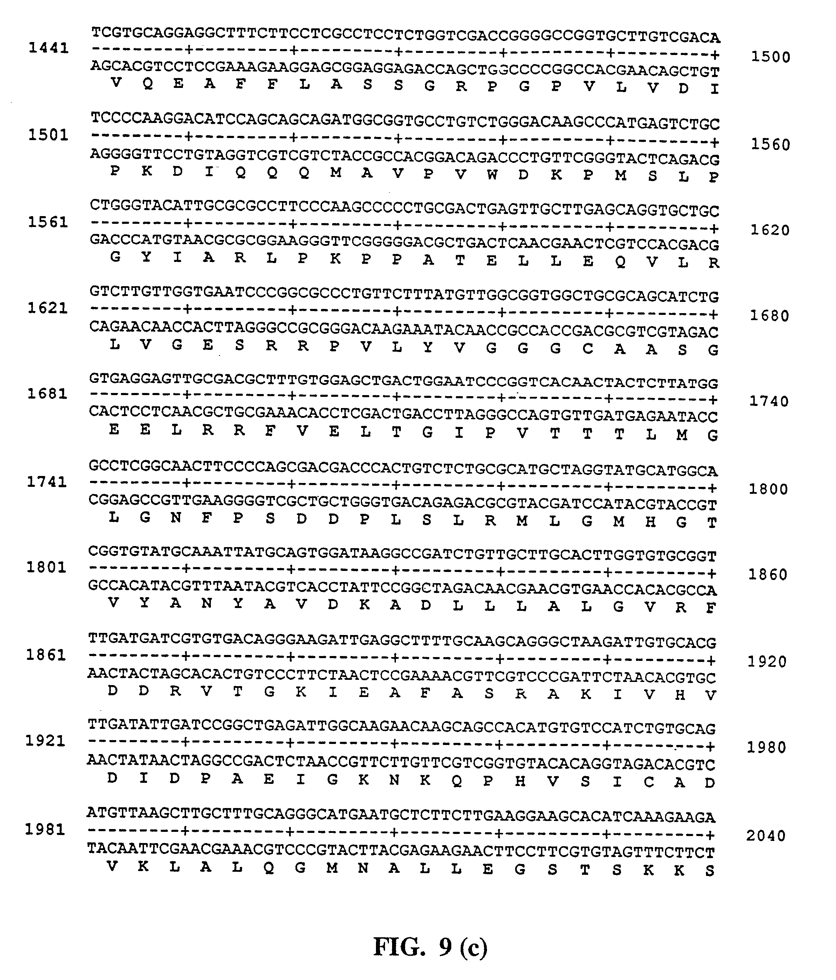

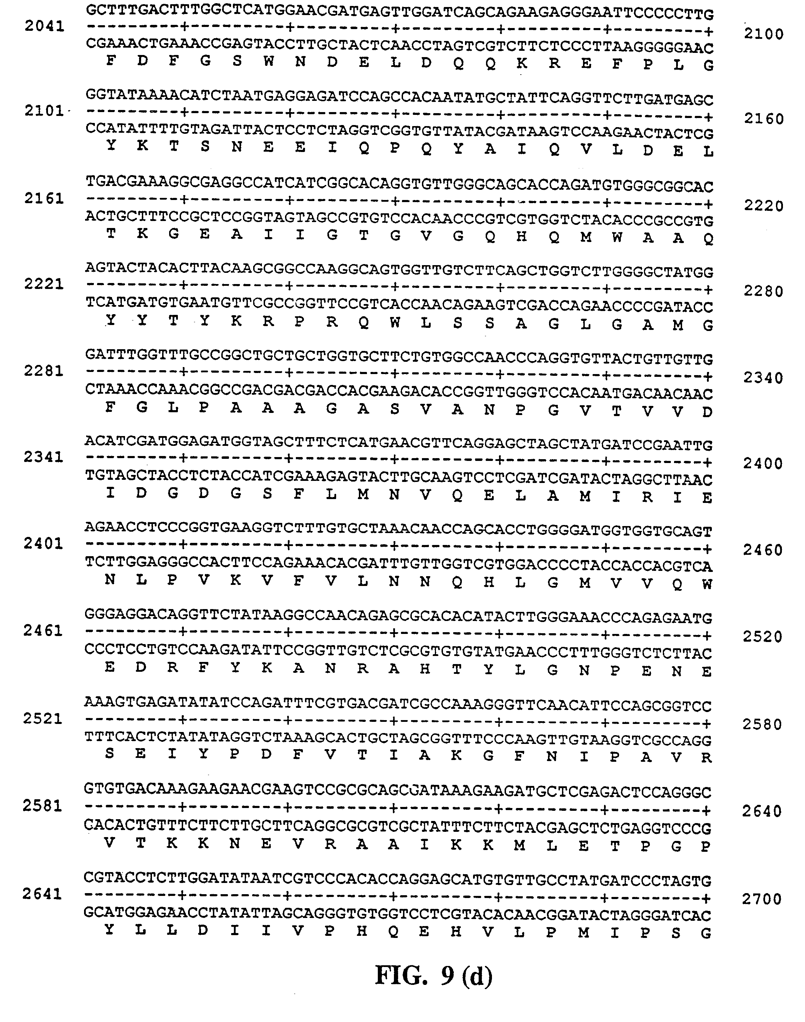

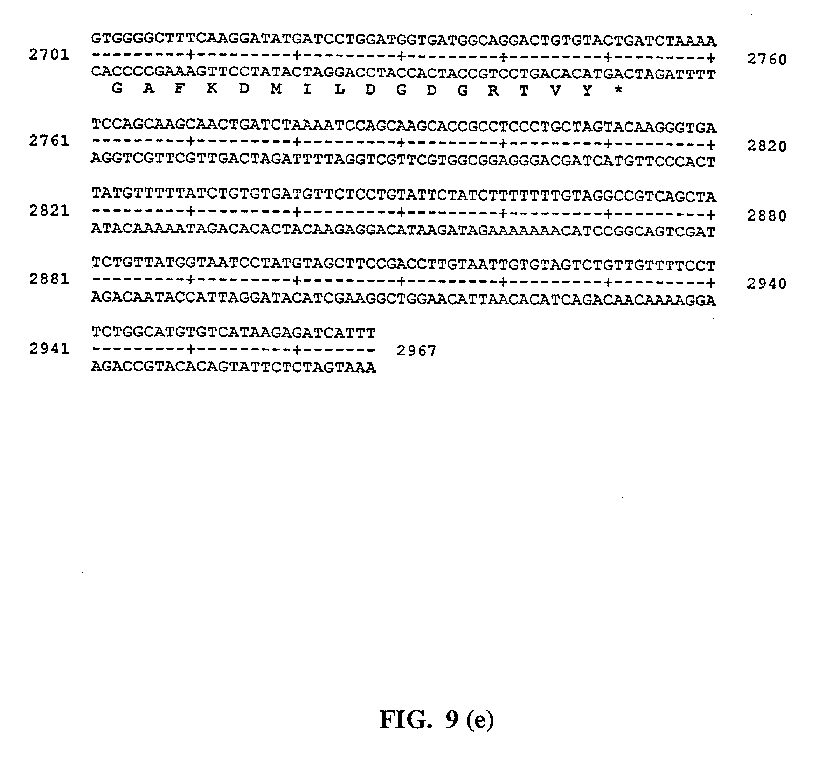

1) Clone pCIB 1253:
The 2.1 kbp HindIII-EcoRI fragment from pCIB1253 is cloned into Bluescript SK+ plasmid (Stratagene). The cloned insert of the of the resulting plasmid DNA is sequenced using the "M13 minus20" primer, 5'GTAAAACGACGGCCAGT3'. The resulting partial sequence, Clsqb-20.Seq, is shown in Figure 3. The deduced amino acid sequence from one reading frame of the sequence complementary to C1sqb-20.Seq is shown in Figure 4. A comparison of this amino acid sequence with the sequence of the Arabidopsis and tobacco B AHAS genes (B. Mazur et al., Plant Physiol. 85: 1110-1117, 1987, and K. Lee et al., EMBO J. 7: 1241-1248, 1988) is shown in Figure 5. The comparison shows this reading frame to have 89 % homology to a specific region within both the Arabidopsis and the tobacco B AHAS polypeptides. Such substantial homology is an indication that the DNA insert in pCIB1253 contains part or all of a maize AHAS gene. The complete maize C1 AHAS gene sequence and its flanking sequences are obtained on further dideoxy sequencing and are shown in Figure 9.

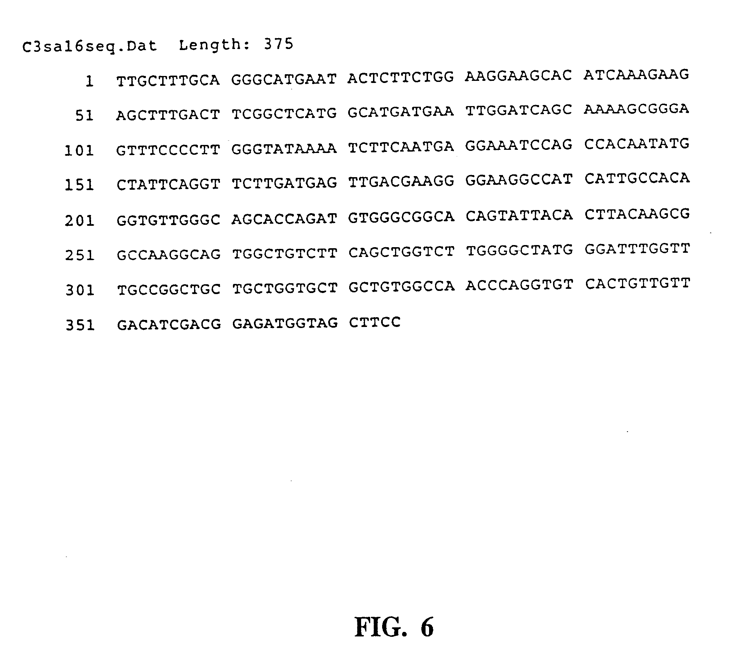

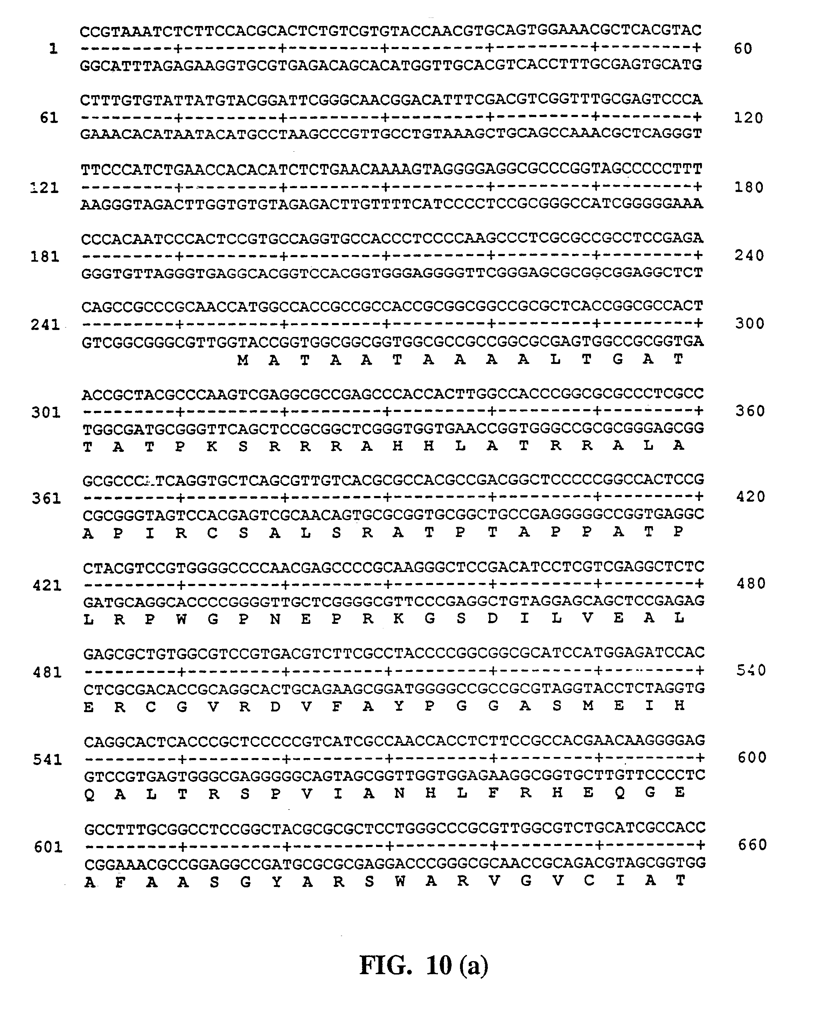

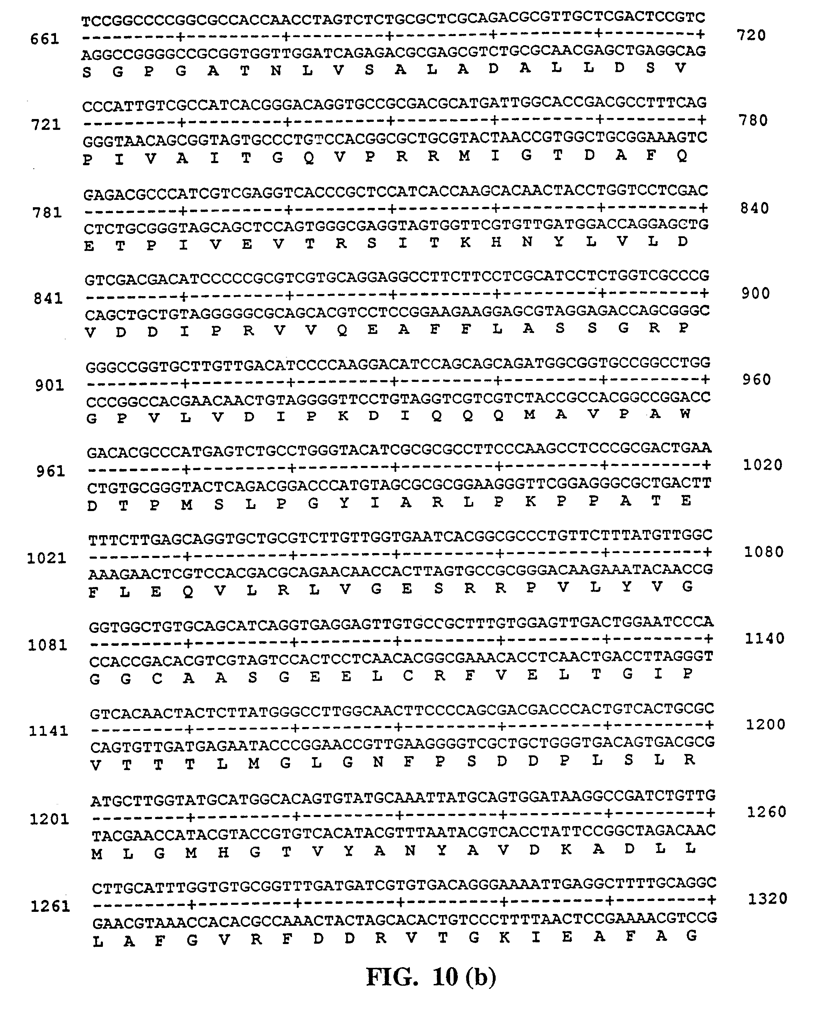

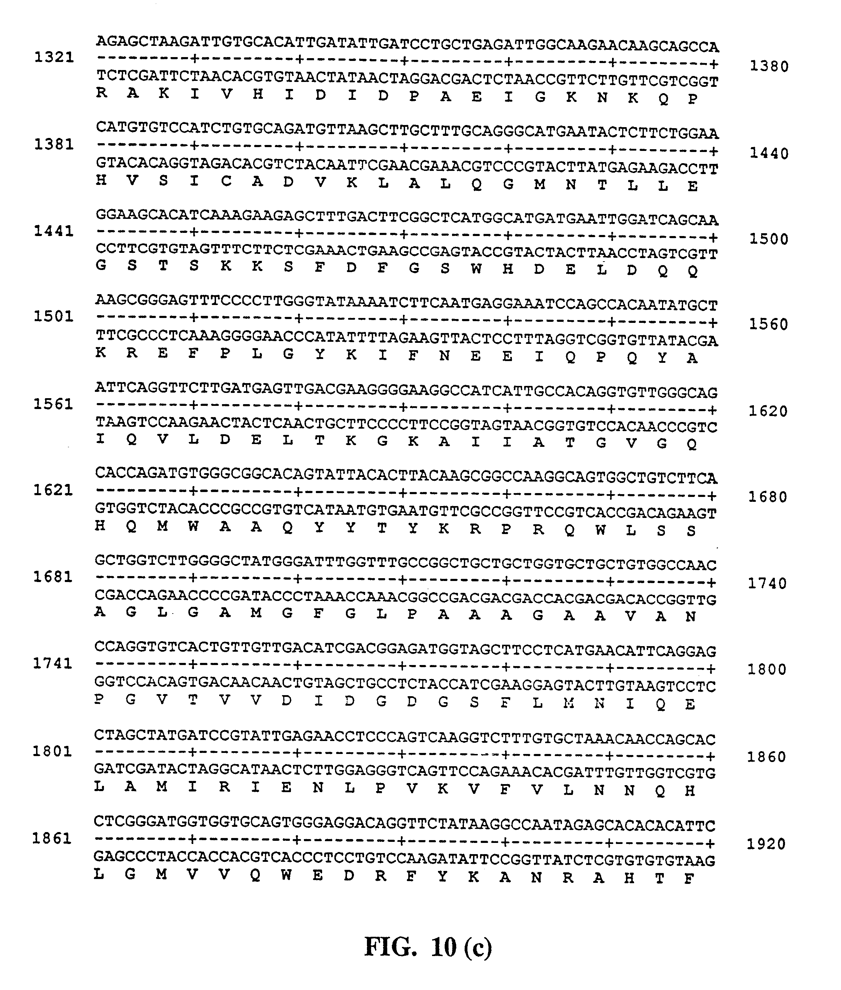

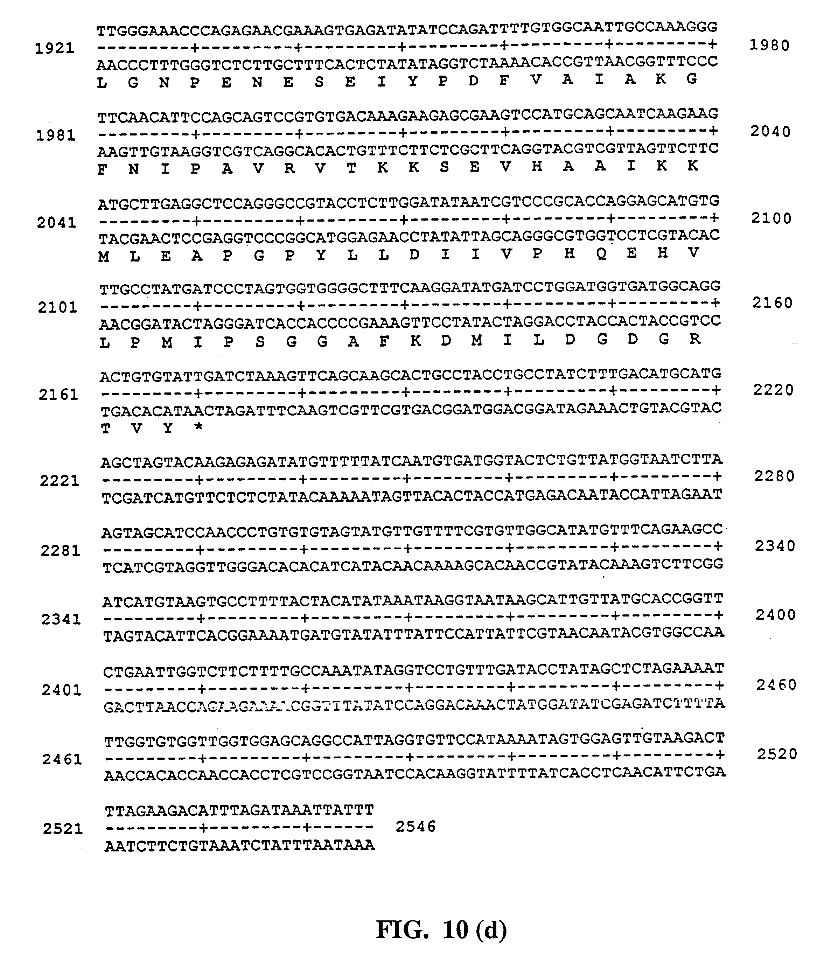

2) Clone pCIB 1255:
The pCIB1255 DNA is sequenced using the following primer: 5'TAAGATTGTTCATATTG3'. This is the sequence of the Arabidopsis AHAS gene from nucleotide position 1166 to 1182. Position #1 is the A of the initiator ATG of the Arabidopsis AHAS coding sequence. The resulting partial sequence of the DNA insert of pCIB 1255, designated C3sa16seq.Dat, is shown in Figure 6. The deduced amino acid sequence from one possible reading frame of C3sa16seq.Dat is shown in Figure 7. A comparison of this amino acid sequence with that of the Arabidopsis and tobacco B AHAS is shown in Figure 8. This comparison indicates that the chosen reading frame has 81 % homolgy to a specific region within both the of Arabidopsis and tobacco B AHAS polypeptides. This substantial homology indicates that the DNA insert of pCIB1255 contains part or all of a second, distinct maize AHAS gene. The complete maize C3 AHAS gene sequence and its flanking sequences are obtained on further dideoxy sequencing and are shown in Figure 10.

EXAMPLE 20: Construction of CaMV 355/wild-type maize AHAS chimeric gene.

a) Transcriptional fusion:
Transcription initiation sites of maize AHAS genes in DCIB1200 and DCIB1202 are mapped by primer extension following procedures described in examples below. Restriction sites are introduced into pCIB1253 and pCIB1255 upstream (in the 5' direction) and within 10 bp of the transcription initiation sites of each AHAS gene. This is done by in vitro mutagenesis using the Muta-Gene kit (BIO-RAD, Richmond, CA) following the manufacturer's instructions.
The maize AHAS genes are excised from modified pCIB1253 and pCIB1255 plasmids by restriction enzyme cutting at the newly created site and at a site located downstream of the termination codons of the two genes. The ends of the excised gene fragments are modified with the attachment of appropriate linkers, and the maize AHAS genes fused to the CaMV35S promoter cassette in pCIB710 following procedures of EXAMPLE 18. The CaMV expression vectors containinig the maize AHAS genes of DCIB1200 and DCIB1202 are designated pCIB1256 and pCIB1258 respectively.

b) Transfer of the CaMV 35S/maize AHAS gene fusions from pCIB1256 and pCIB1258 to the binary vector pCIB200:
The CaMV 35S/maize AHAS gene fusions are excised from pCIB1256 and pCIB1258 by digestions with restriction enzymes that cut outside of the gene fusions. The ends of the released chimeric gene fragments are modified with the appropriate linkers, and the fragments are cloned into the XbaI site of pCIB 200 similarly modified with the same restriction site linkers. The ligation reactions are transformed into E. coli strain HB101 and kanamycin resistant bacteria selected. Miniprep plasmid DNA is prepared from the kanamycin-resistant transformants and analyzed using restriction enzyme digests to identify pCIB200 plasmids containing the two desired chimeric CaMV/maize AHAS gene fusions. The binary vectors containing CaMV/maize AHAS gene fusions from pCIB1256 and pCIB1258 plasmids are designated pCIB1259 and pCIB1261, respectively.

c) Transfer of pCIB1259 and pCIB1261 from E. coli to A. tumefaciens:
Triparental matings are used to transfer pCIB1259 and pCIB1261 from E. coli strain HB101 to A. tumefaciens strain A136 bearing the virulence-helper plasmid pCIB542 according to the procedures described in EXAMPLE 16. The A. tumefaciens A136 strain harboring pCIB542 and pCIB1259 is designated strain CGA1450; and the strain containing pCIB542 and pCIB1261 is designated strain CGA1452.

## EXAMPLE 21: Construction of a tobacco PR-1A promoter regulated Arabidopsis AHAS gene

### a) Preparation of tobchrPR1013

Nuclei are isolated from leaves of Nicotiana tabaccum by first freezing 35 grams of the tissue in liquid nitrogen and grinding to a fine powder with a mortar and pestle. The powder is added to 250 ml of grinding buffer (0.3 M sucrose, 50 mM Tris, pH 8, 5 mM magnesium chloride, 5 mM sodium bisulfite, 0.5% NP40) and stirred on ice for 10 minutes. This mixture is filtered through six layers of cheesecloth, and the liquid is transferred to centrifuge bottles and subjected to centrifugation at 700 x g for 10 minutes. The pellets are resuspended in grinding buffer and recentrifuged. The pellets are again resuspended in grinding buffer and recentrifuged. The pellets are again resuspended in grinding buffer and this suspension is layered over a 20 ml cold sucrose cushion containing 10 mM Tris, pH 8, 1.5 mM magnesium chloride, 140 mM sodium chloride, 24% sucrose, 1% NP40. These tubes are centrifuged at 17,000 x g for 10 minutes. The pellets at this stage contain mostly nuclei and starch granules. High molecular weight DNA is isolated from the nuclei essentially according to Maniatis, T. et al., supra. The DNA is partially digested with MboI and ligated into the BamHI site of the EMBL3 cloning vector. Approximately 300,000 plaques are screened with a labeled PR-1b cDNA probe. Fifty positive clones are isolated and characterized. Positive plaques are purified and characterized by restriction analysis. These clones are classified into eight distinct groups by restriction mapping. One of the clones is identified as tobchrPR1013. A partial restriction map of tobchrPR1013 is shown in Figure 1. Isolation of DNA and DNA manipulations are essentially as described by Maniatis, T. et al., supra.

### b) Preparation of pBS-PR1013Cla

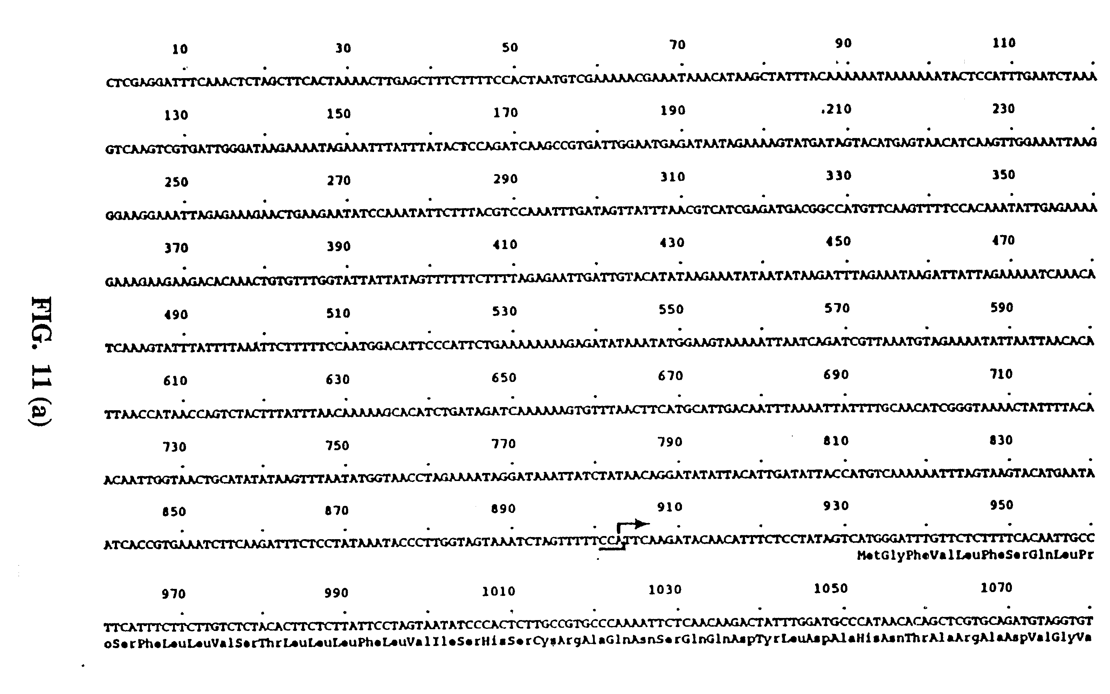

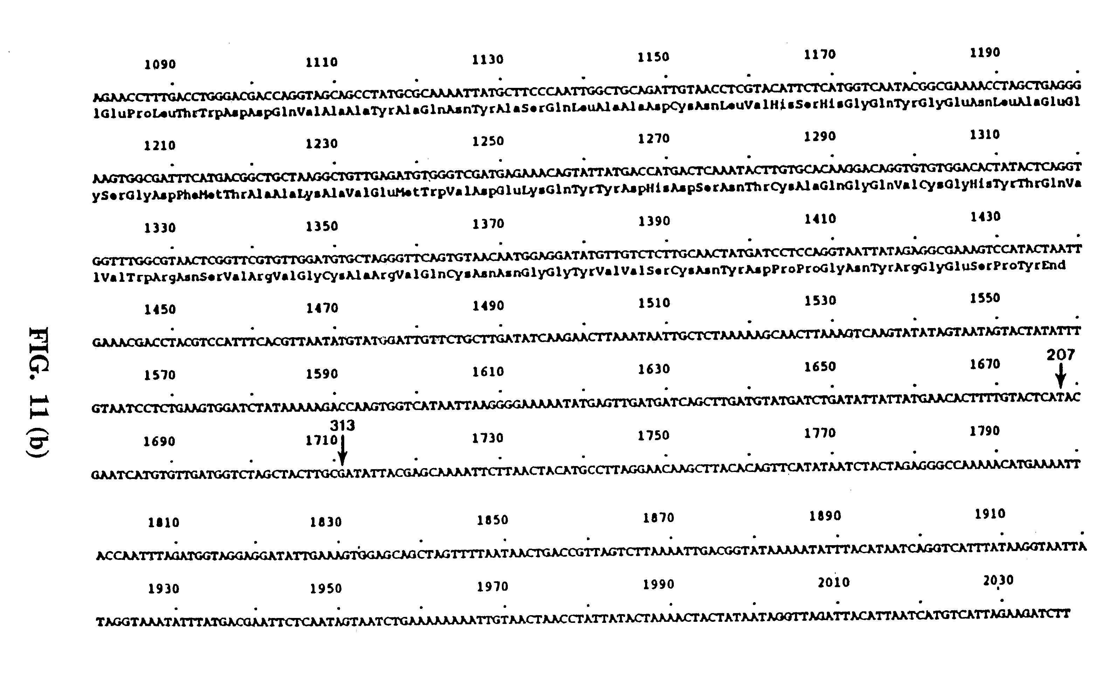

A ClaI fragment from the clone tobchrpR1013 is subcloned into the Bluescript plasmid (Stratagene Cloning Systems), resulting in pBS-PR1013Cla. The 2 kb XhoI-BglII fragment from pBS-PR1013Cla is sequenced and positions 913 to 1711 (Figure 11) are found to match perfectly the sequence of a cDNA clone clone for PR-1a isolated by Pfitzner, U.M. et al., Mol. Gen. Genet., 211: 290-295 (1988). Possible rearrangements of the tobacco DNA during the cloning procedure are ruled out by analyzing predicted restriction fragments from genomic DNA. Based on sequence information and a restriction map of pBS-PR1013Cla, digestion of genomic tobacco DNA with either EcoRI, HindIII, XhoI + BglII, or HindIII + PatI should generate fragments of 3.2 kb, 6.7 kb, 2.0 kb or 6.4 kb, respectively, which contain the PR-1a gene. To test this prediction, 3 µg of tobacco chromosomal DNA is digested with the appropriate enzymes and electrophoresed on a 0.7% agarose gel. The DNA is transferred to a nylon membrane and probed with a labeled XhoI-BstEII restriction fragment from the PR-1a gene. As a control, pBS-PR1013Cla DNA is digested and electrophoresed on the same gel. As predicted, the EcoRI, HindIII, XhoI + BglII, and HindIII + PatI digests produce bands of the expected molecular weight which comigrate with the control bands. Therefore, the DNA contained in the tobchrPR1013 clone represents a contiguous piece of DNA inthe tobacco genome.

### c) Preparation of pBS-PR1013Eco

A. The plasmid pBS-PR1013Eco is constructed by subcloning the 3.6 kb EcoRI fragment containing the PR-1a gene from tobchrPR1013 into the unique EcoRI site of the bluescript plasmid. Bluescript plasmid (catalog no. 21203) is obtained from Stratagene Cloning Systems, La Jolla, California. The structure of pBS-PR1013Eco is confirmed by both restriction mapping and DNA sequencing.

B. Alternatively, the plasmid pBS-PR1013Cla is digested with EcoRI and the 3.6 kb fragment containing the PR-1a gene is isolated. The pBluescript is digested with EcoRI, mixed with and ligated to the previously isolated 3.6 kb EcoRI fragment.

### d) Preparation of pBS-pR1013EcoΔPst

The plasmid pBS-PR1013Eco is digested with PstI and the DNA fragments separated on a 2.5% low-gelling temperature agarose gel. The large fragment containing the bluescript vector and a 3.0 kb fragment of the tobacco DNA are precisely excised and heated to 65. C to melt the agarose. 2 µl are added to 15 µl water and the DNA is ligated in agarose. After overnight incubation, the agarose containing the DNA is then melted by incubating at 65° C for 10 minutes and then transformed from the agarose essentially as described above. A portion of the bacterial culture from each of six putative positive constructions is inoculated into 5 ml of LB medium [Miller, J.H., Experiments in Molecular Genetics, Cold Spring Harbor Laboratory, New York (1972)] containing 100 µg/ml ampicillin, and this culture is incubated at 37. C overnight. Cells are harvested by centrifugation in a clinical centrifuge at full speed for 10 minutes. The supernatant is removed and the cells are resuspended in 100 µl of 50 mM glucose, 25 mM Tris, pH 7.5, 10 mM EDTA, and the suspension is transferred to a 1.5 ml Eppendorf centrifuge tube. 25 µl of a freshly prepared 10 mg/ml lysozyme solution is added and the suspension is left on ice for 10 minutes. 200 µl of 0.2 N sodium hydroxide, 1% sodium dodecyl sulfate is then added and the suspension is mixed and allowed to stand on ice for two minutes. 200 µl of 2 M sodium acetate (pH 4.8) is added and the solution is allowed to sit on ice for 15 minutes. The resulting solution is subjected to centrifugation at full speed for 10 minutes in an Eppendorf centrifuge. The supernatant is removed to a fresh centrifuge tube and extracted with 400 µl phenol/chloroform (1:1). The aqueous phase is removed to a fresh tube and extracted with 400 μl chloroform. The aqueous phase from this extraction is removed to a fresh tube and DNA is precipitated by the addition of two volumes ethanol and storage at -20°C for 30 minutes. The precipitated DNA is collected by centrifugation in an Eppendorf microfuge for 15 minutes at full speed. The ethanol is removed and the pellets are washed with 80% ethanol. The ethanol is again removed and the pellets are allowed to air-dry. The DNA is resuspended in 100 μl of TE buffer. Constructions are verified by digestion with PstI. The parental plasmid pBS-PR1013Eco yields a 6 kb and ~650 bp band, the smaller of which is missing in the new constructions. One of these plasmids is selected as the plasmid pBS-PR1013EcoΔPst. The structure of this subclone is verified by sequence analysis.

e) Preparation of pBS-PR1013EcoΔPstΔXho

A preliminary restriction map of pBS-PR1013Eco is established. An XhoI restriction site is located ~1200 bp upstream from the PstI site. The plasmid pBS-PR1013EcoΔPst is digested with XhoI and the fragments are separated on 0.9% low-gelling temperature agarose gel. Two fragments are visualized with sizes of 3.9 kb and 1.7 kb. The band containing the 3.9 kb fragment is carefully excised from the gel and ligated in agarose essentially as described above. The agarose is melted and the DNA transformed into competent E. coli strain HB101 as described above. Cells are plated onto LB-amp plates and incubated as described. One putative positive colony is inoculated into LB-amp media and DNA is isolated essentially as described. The structure of this new subclone pBS-PR1013EcoΔPstΔXho is verified by restriction analysis and DNA sequencing.

f) Preparation of pCIB270

Plasmid pBI101.3 (Catalog no. 6024.1) is obtained from Clonetech Labs, Palo Alto, California. This plasmid is first digested with BamHI and then with SalI. The plasmid pBS-PR1013EcoΔPstΔXho is digested with PstI. A PstI/BamHI adaptor having the sequence
5'-GGGATCCCTGCA-3'
is synthesized on an Applied Biosystems Synthesizer using B-cyanoethylphosphoramidite chemistry and purified by reverse-phase HPLC and ligated to the PstI- digested pBS-pR1013EcoΔPstΔXho. The resulting material is first digested with BamHI and then with XhoI. The BamHI/XhoI fragment is isolated, mixed with and ligated to the digested pBI101.3, and transformed. A putative positive clone of pCIB270 is isolated and verified by restriction and DNA sequence analysis.

g) Preparation of M13mp18- or mp19-PR1013EcoΔPstΔXho

The plasmid pBS-PR1013EcoΔPstΔXho is digested with PatI and Asp718. The 1.1 kb Asp718/PstI fragment is isolated after electrophoresis on a 1% TAE low-gelling agarose gel. The replicative form (RF) of the coli phage M13mp18 or M13mp19 is prepared according to Messing, J., Meth. Enzymol. 101, 20 (1983). Alternatively, these vectors can be obtained from Bethesda Research Labs., Gaithersburg, Maryland. Either vector is digested first with Asp718 and then with PstI. After removal of the polylinker piece by electrophoresis on 0.7% TAE low-gelling agarose, the resulting vector RF is mixed and ligated in agarose with the 1.1 kb fragment prepared above. The DNA is transformed, putative positive plaques are isolated and their structure is verified by analysis of the RF DNA.

h) Preparation of M13mp18- or mp19- PR1013EcoΔPstΔXho.Nco and pCIB 268

Single stranded M13mp18-PR1013EcoΔPstΔXho phage DNA is isolated according to Messing, supra. An 18 bp oligonucleotide primer of the sequence
5'-AATCCCATGGCTATAGGA-3'
is synthesized as described above. The primer is phosphorylated with T4 polynucleotide kinase (Maniatis, T. et al., supra at p. 125) using unlabeled ATP. After incubation at 37°C for 60 minutes the reaction is heated to 68°C for 15 minutes and then placed on ice. This primer and the M13-40 forward sequencing primer (New England Biolabs #1212) are annealed to single stranded M13mp18-PR1013EcoΔPstΔXho DNA after mixing in a 1:1:1 molar ratio, by slow cooling after heating for 10 minutes at 68°C. The annealed mixture is placed on ice and 1/10 volume of 10 X elongation buffer (20 mM Tris buffer pH 7.5, 6 mM MgCl₂, 1 mM DTT, 1 mM dATP, 1 mM dCTP, 1 mM dGTP, and 1 mM dTTP) is added. 10 units of DNA Polymerase I large fragment (Klenow fragment, New England Biolabs #210) and 0.1 unit of T4 DNA Ligase (NEB # 202) are added and the reaction is allowed to proceed 14 hours at 15°C. At that time an additional aliquot of each enzyme is added and the reaction is carried out for an additional 2 hours at 25°C before the mixture is used to transform competent JM101 E. coli.

To identify the mutated phage, the plaques are lifted to Gene Screen Plus (DuPont) and processed according to the manufacturer's recommendations. The filters are pre-hybridized for four hours, and hybridized overnight at 42°C, in the manufacturer,s hybridization solution containing 0.9 M NaCl. The filters are then sequentially washed at a NaCl concentration of 0.9 M and monitored by autoradiography increasing the wash temperature by 5°C at each step. Phages identified by hybridization to have been mutated are sequenced to confirm the base change.

The replicative form of one such candidate (M13mp18-PR1013EcoΔPstΔXho.Nco) is isolated and digested with PstI and BStEII to produce a 394 bp fragment which is used to replace the corresponding non-mutated 394 bp fragment in pBS-PR1013EcoΔPstΔXho. This results in the preparation of pCIB268 which has an NcoI site at position 930 followed by 217 bp of the coding sequence for the PR-1a gene. The structure of this plasmid is confirmed by sequence analysis.

i) Preparation of tobacco PR-1A promoter cassette

A. Construction of pBS-GUS1.2

pBS-GUS1.2 is created by three part ligation of a 391 bp Sall/Snabl fragment from pRAJ265 (Jefferson, R.A. et al., EMBO J. 6, 3091-3907 (1987) and GUS User Manual, Clonetech Labs., Palo Alto, Ca.) with a 1707 bp Snabl/EcoRI fragment from pBl221 (Jefferson, R.A. et al., EMBO J. supra) and PBS digested with Sall and EcoRI. Transformants are isolated and analyzed by restriction digestion and DNA sequencing. One verified clone is named pBS-GUS1.2.

B. Construction of pCIB269

A fragment of 930 bp is isolated after electrophoresis of XhoI and NcoI digested pCIB268 on a 1.0 % low gelling temperature agarose TAE gel. This fragment is then ligated into PBS-GUS1.2 which had been digested with XhoI and NcoI. Transformants are isolated and analyzed by restriction analysis and DNA sequencing. One positive plasmid is chosen and named pCIB269.

k) Transcriptional fusion of the Arabidopsis AHAS gene to a tobacco PR-1A promoter cassette:

The construction of the plasmid pCIB269 in which the PR-1A promoter controls the expression of a bacterial β-glucuronidase gene is described in the copending U.S. patent application Serial No. 165,667, which is incorporated by reference. To construct a PR-1A regulated AHAS gene, pCIB1207 containing a wild-type Arabidopsis AHAS gene described in EXAMPLE 15 is digested with NcoI and XbaI, and ligated with NcoI and XbaI digested pCIB269 plasmid. The ligation reaction is transformed into E. coli strain JM109. Miniprep plasmid DNA is prepared from ampicillin-resistant transformants and is digested with NcoI and XbaI to identify pCIB269 derivatives that contain a 3.3 kbp fragment in the place of the original 2.0 kbp fragment. One such plasmid is designated pCIB1216.

l) Transfer of the chimeric PR-1A/AHAS gene to a binary transformation vector:

The chimeric PR-1A/AHAS gene is excised from pCIB1216 by digestion with KpnI and XbaI and is ligated to KpnI and XbaI digested pCIB200. The ligation reaction is transformed into E. coli strain HB101. Miniprep DNA is prepared from kanamycin-resistant transformants and digested with KpnI and XbaI to identify pCIB200 derivatives containing the PR-1A/AHAS gene fragment. One such plasmid is designated pCIB1217. This plasmid is transferred from E. coli strain HB101 to A. tumefaciens strain A136 containing the pCIB542 virulence helper plasmid by triparental mating, following procedures described in EXAMPLE 16. One isolate of A. tumefaciens A136 containing pCIB542 and pCIB1217 is designated Strain CGA1404.

EXAMPLE 22: Preparation of DNA for direct gene transfer to protoplasts

Plasmid pCIB1210 (EXAMPLE 17) is propagated in DH5a (BRL) by growing the strain in TB broth (BRL Focus) containing 100 g/ml carbenicillin. Plasmid DNA is isolated by the Triton cleared lysate procedure of D.B. Clewel and D.R. Helinski, J.Bacteriol. 110: 668-672 (1972), modified to use three fold larger volumes of reagents and 2 % Triton X-100 rather than 10 % Triton X-100. The plasmid is collected after banding on a cesium chloride gradient containing ethidium bromide formed by centrifugation for 4 hours at 65,000 rpm or 14 hours at 45,000 rpm in a vertical rotor in a Beckmann L880 ultracentrifuge. The ethidium bromide is removed by repeated extractions with isopropyl alcohol which has been equilibrated with 20 X SSC, and the DNA is precipitated bythe addition of one volume 10 mM Tris-HCl, pH 8.0, and 0.8 volumes isopropanol.

2 mg of this pCIB1210 DNA are completely digested with SpeI to give two fragments of about 4000 bp and 5195 bp which are then separated by electrophoresis at 1 Volt/cm on a 0.7 % gel of low melting temperature agarose (BRL) in TAE buffer. The 4 kbp band is cut from the gel, melted at 65°C for five minutes, diluted with an equal volume of water and held at 37°C for an additional ten minutes. The DNA is then separated from the agarose by two successive extractions with phenol followed by the addition of 0.1 volume of 3 M sodium acetate and two volumes of 95 % ethanol.

The isolated fragment is then dissolved in 10mM Tris-HCl, pH 7.0, at a concentration of 1 mg/ml. 0.1 volume of 10 X ligation salts (0.66 M Tris-HCl, pH 7.5, 0.66 M MgCl$_2$, 0.1 M DTT, 0.66 mM ATP) and 1 unit of T4 DNA ligase are added and the reaction is incubated at 15°C for one hour. The reaction mixture is frozen, and a sample containing 0.5 μg DNA is electrophoresed on a 0.7 % TB agarose gel to determine the extent of ligation that has taken place. Depending on this result, the reaction is continued after the addition of ligase or SpeI and further monitored until the majority of fragments in the mixture are in the range of 12 to 16 kbp. The DNA is then precipitated by the addition of an equal volume of 5 M ammonium acetate and two volumes of ethanol. The precipitated DNA is washed with 70 % ethanol and air dried.

The DNA is dissolved in sterile buffer and used to transform corn protoplasts as described below.

EXAMPLE 23: Leaf disk transformation of tobacco

a) Agrobacterium infection of plant material:

The different genotypes of A. tumefaciens are grown on AB minimal medium (Watson, B. et al., J.Bacteriol. 123, 255 (1975)) plus mannitol or glutamate salts medium for 48 hours at 28°C. Bacteria are pelleted, resuspended in MSBN medium at a twofold dilution, and held for three hours at 25° C. Prior to use the bacterial suspension is diluted with MSBN or glutamate salts to an optical density (OD) between 0.1 and 0.2. 5-7 mm leaf discs are punched aseptically from in vitro cultured Nicotiana tabacum cv. Xanthi, Havana 38, SR1, and Coker 176 plants and are dipped into and kept in the bacterial suspension for 6 to 10 minutes following the method of R. Horsch et al., Science 227: 1229-1231 (1985). Control discs are dipped and incubated in glutamate salts medium for the same period of time. The leaf disks are blotted on filter paper to remove excess bacteria and transferred to MSBN medium. Leaf disks are then transferred to filter paper on MSBN medium. The wrapped plates are incubated for 48 hours at 25-28. C. The leaf discs are then dipped in liquid MSBN medium containing 500 mg/l of carbenicillin and 200 mg/l of vancomycin and placed, lower side up, onto solidified (0.8 % agar) MSBN medium containing 100 mg/l kanamycin, 500 mg/l carbenicillin and 200 mg/l vancomycin for selection of transformed cells. Kanamycin and carbenicillin are added as a filter sterilized solution after autoclaving the rest of the medium. The culture plates are incubated in the light at 25-28° C. Leaf discs are transferred to fresh medium of the same composition at weekly intervals.

b) Plant maturation and self-pollination:

Shoots forming on the leaf discs on MSBN selection medium are excised when approximately 1 cm in length and transferred to MSB medium which is comprised of MS major, minor salts and Fe-EDTA (Gibco # 500-1117; 4.3 g/l), 85 vitamins (Gamborg, O.L. et al., Experimental Cell Research 50: 151-158, 1968), 100 mg/l myo-inositol and 30 g/l sucrose, pH 5.8) and supplemented with 100 mg/l kanamycin, 500 mg/l carbenicillin and 200 mg/l vancomycin. Shoots are excised from different leaf discs or different sides of the same disc so as to insure that they derive from different transformed cells. Plantlet development is allowed to continue until roots are well developed. Plantlets are divided to give replicate rooted shoot cultures. Rooted plantlets are transplanted to a soil-vermiculite mixture and transferred to the greenhouse. Freshly potted plantlets are kept moist and shaded for several days to facilitate their hardening off. Plants are cultivated following common procedures and grown to maturity. At flowering time flowers are induced to selfpollinate. Seeds are harvested following maturation.

EXAMPLE 24: Production of transgenic tobacco callus and plants

Agrobacterium strains CGA1402 and CGA1403 are used to transform tobacco by the leaf disk method according to EXAMPLE 23. Transformed plants derived from selection are grown to maturity. Callus forming from the leaf disks on MSBN selection medium is maintained on a callus growth medium. The callus is used to regenerate transgenic plants by transferring callus pieces to MSBN medium as described above. Callus and suspension cultures derived from callus are used to determine the expression of herbicide tolerance as described in EXAMPLES 11. Callus and suspension cultures are also used to determine the expression of herbicide tolerance after chemical induction as described below.

EXAMPLE 25: Preparation of a suspension culture of Zea mays, elite inbred Funk 2717

The callus described in EXAMPLE 12 is subcultured for a total of at least six months. The type of callus chosen for subculture is relatively non-mucilaginous, granular and very friable, such that it separates into small individual cell aggregates upon placing into liquid medium. Cultures containing aggregates with large, expanded cells are not retained. Approximately 500 mg aliquots of the special callus of Zea mays elite inbred Funk 2717 are placed into 30 ml of N6 medium containing 2 mg/l 2,4-D in 125 ml Delong flasks. After one week of culture at 26° C in the dark on a gyratory shaker (130 rpm, 2.5 cm throw), the medium is replaced with fresh medium. The suspensions are again subcultured in this way after another week. At that time, the cultures are inspected, and those which do not show large numbers of expanded cells are retained. The preferred tissue consists of densely cytoplasmic dividing cell aggregates which has a characteristically smoother surface than the usual type of cell aggregates. The cultures retained have at least 50 % of the cells represented in these small aggregates. These suspensions also have a rapid growth rate, with a doubling time of less than one week. The suspension cultures are subcultured weekly by transferring 0.5 ml PCV (packed cell volume: settled cell volume in a pipette) into 25 ml of fresh medium. After four to six weeks of subculture in this fashion, the cultures increase two- to three-fold per weekly subculture. Cultures in which more than 75 % of the cells are of the desired morphology are retained for further subculture. The lines are maintained by always choosing for subculture the flask whose contents exhibit the best morphology. Periodic filtration through 630 μm pore size stainless steel sieves is used in some cases to increase the dispersion of the cultures, but is not necessary.

A Zea mays suspension culture having the desired morphology is deposited with the American Type Culture Collection (ATCC), see above.

EXAMPLE 26: Preparation of protoplasts from suspension cultures of Zea mays

1-1.5 ml PCV of the suspension culture cells prepared as in EXAMPLE 25 are incubated in 10-15 ml of a filter-sterilized mixture consisting of 4 % (w/v) cellulase RS with 1 % (w/v) Rhozyme in KMC (8.65 g/l KCl, 16.47 g/l MgCl$_2$·6H20, 12.5 g/l CaCl$_2$·2H20, 5 g/l MES, pH 5.6) salt solution. Digestion is carried out at 30° C on a slow rocking table for a period of 3-4 hours. The preparation is monitored under an inverted microscope for protoplast release. The protoplasts which are released are collected as follows: The preparation is filtered through a 100 μm mesh sieve, followed by a 50 μm mesh sieve. The protoplasts are washed through the sieves with a volume of KMC salt solution equal to the original volume of enzyme solution. 10 ml of the protoplast preparation is placed in each of several disposable plastic centrifuge tubes, and 1.5-2 ml of 0.6 M sucrose solution (buffered to pH 5.6 with 0.1 % (w/v) MES and KOH) layered underneath. The tube is centrifuged at 60-100 x g for 10 minutes, and the protoplasts banding at the interface collected using a pipette and placed in a fresh tube. The protoplast preparation is resuspended in 10 ml of fresh KMC salt solution, and centrifuged for five minutes at 60-100 x g. The supernatant is removed and discarded, and the protoplasts resuspended gently in the drop remaining, and then 10 ml of a 13/14 strength KMC solution gradually added. After centrifuging again for five minutes, the supernatant is again removed and the protoplasts resuspended in a 6/7 strength KMC solution. An aliquot is taken for counting, and the protoplasts again sedimented by centrifugation. The protoplasts are resuspended at $10^7$ per ml in KM medium or in 0.5 M mannitol containing 0.1 % (w/v) MES and 6 mM $MgCl_2$ as required for use in transformation described in the following examples.

## EXAMPLE 27: Transformation of Zea mays protoplasts by electroporation

All steps except the heat shock are carried out at room temperature (22-28°C). The resistance of the suspension of the protoplasts of EXAMPLE 26 in 0.5 M mannitol containing 0.1 % (w/v) MES and 6 mM $MgCl_2$ is measured in the chamber of a Dialog Electroporator (DIA-LOG GmbH, D-4000 Duesseldorf 13, Federal Republic of Germany) and adjusted to 1-1.2 Kohm using a 300 mM $MgCl_2$ solution. The protoplasts are heat-shocked by immersing the tube containing the sample in a water bath at 45°C for five minutes, followed by cooling to room temperature on ice. 4 μg of DNA of EXAMPLE 22 or of linearized plasmid containing the CaMV 355/maize AHAS gene construct of EXAMPLE 19, and 20 μg of calf thymus carrier DNA are added to aliquots of 0.25 ml of the protoplast suspension. 0.125 ml of a 24 % (w/v) polyethylene glycol (PEG) solution (MW 8000) in 0.5 M mannitol containing 30 mM $MgCl_2$ are added to the protoplasts. The mixture is mixed well but gently, and incubated for 10 minutes. The sample istransferred to the chamber of the electroporator and samples pulsed three times at 10 second intervals, at initial voltages of 1000, 1200, 1500, 1800, 2300 or 2800 $Vcm^{-1}$, and an exponential decay time of 10 μsec.

The protoplasts are cultured as follows: The samples are plated in 6 cm Petri dishes at room temperature. After a further 5-15 minutes, 3 ml of KM medium containing 1.2 % (w/v) SeaPlaque® agarose and 1 mg/l 2,4-D are added. The agarose and protoplasts are mixed well and the medium allowed to gel.

Alternatively, the protoplasts are taken up in culture medium (KM medium with 0.5 mg/l 2,4-D and 100 mg/l acetyl-salicylic acid in 1.2 % SeaPlaque® agarose) and placed onto nurse culture Durapore filters (GVWP 047 00, 0.22 μm pore size, 47 mm diameter) at a density of 0.5 x $10^6$ per ml. The filters are autoclaved in distilled, deionized water in a GA7 plastic container for 20 minutes and pre-conditioned in liquid KM culture medium as above, but without gelling agent, for 3 hours prior to placing the filters on the nurse cultures.

Nurse cultures are prepared from Black Mexican Sweet (BMS) corn suspension cultures (Green, Hort. Sci. 12: 131, 1977; Smith et al., Plant Sci. Lett. 36: 67, 1984) or the embryogenic suspensions described in EXAMPLE 25. When BMS is used as a nurse, sterile KM medium with 0.5 mg/l 2,4-D and 100 mg/l acetylsalicylic acid (2 mg/ml in 2 % (v/v) DMSO containing 0.1 % (w/v) MES, pH 6.0) is added to sterile SeaPlaque® agarose to give a final concentration of agarose of 1.2 % (w/v). After microwaving to melt the agarose, the medium is cooled to 44°C in a water bath, 1 ml PCV of BMS suspension is added per 10 ml of medium, and 5 ml aliquots are distributed over the surface of 60 mm diameter Petri dishes. When the medium has solidified the mentioned sterile Durapore filters are placed on the surface. 0.5 ml of the protoplast suspension in the mentioned medium, microwaved and cooled to 44°C, are pipetted slowly onto the surface of each of the filters.

When embryogenic suspension cultures are used as nurse, the N6 medium is increased in osmolality using glucose to approximately 530-540 mOs/kg $H_2O$. The pH is re-adjusted to 6.0, and the medium is filter-sterilized through a 0.2 μm filter. 2,4-D and acetylsalicylic acid are added and the procedure followed as described in the preceding paragraph.

## EXAMPLE 28: Transformation of Zea mays protoplasts by treatment with polyethylene glycol

The protoplasts are resuspended in the last step of EXAMPLE26 in a 0.5 M mannitol solution containing 12 mM $MgCl_2$. A heat shock of 45°C for five minutes is given as described in EXAMPLE 27. The protoplasts are distributed in aliquots for transformation in centrifuge tubes, 0.3 ml of suspended protoplasts per tube. During the next 10 minutes the following are added: DNA of EXAMPLES 19 or 22 and 40 % (w/v) PEG solution (MW 6000) containing 0.1 M $Ca(NO_3)_2$ and 0.4 M mannitol, adjusted to pH 8-9 with KOH to give a final concentration of 20 % PEG. The aliquots are incubated for 30 minutes with occasional gentle shaking, and then the protoplasts are placed in Petri dishes (0.3 ml original protoplast suspension per 6 cm diameter dish) and cultured as described in EXAMPLE 27.

## EXAMPLE 29: Regeneration of callus from protoplasts

The plates containing the protoplasts in agarose are placed in the dark at 26°C. Within 14 days, colonies arise from the protoplasts. The agarose containing the colonies is transferred to the surface of a 10 cm diameter Petri dish containing 30 ml of N6 medium containing 2 mg/l 2,4-D, solidified with 0.24 % (w/v) Gelrite®. This medium is referred to as 2N6. The callus is cultured further in the dark at 26°C and callus pieces subcultured every two weeks onto fresh 2N6 medium.

As an alternative, 2N6 medium containing 100 ppb of SU872 is used in order to select for transformed cells.

## EXAMPLE 30: Sample preparation for AHAS assay

The weight of the material to be assayed is determined either by weighing the pellet obtained by refrigerated centrifugation of a cell suspension or by weighing callus, leaf or root tissue placed into liquid nitrogen and macerated to a fine powder. One volume (in ml) of cold homogenization buffer equal to the weight (in g) of the cell suspension pellet or equal to two times the weight (in g) of tissue is added and the mixture is passed through a French pressure cell at 15,000 psi or is ground with sand in a mortar and pestle. All subsequent steps are carried out keeping the material cold (about 4°C). The cells are disrupted at 16,000 psi, collecting the cell exudate into a container on ice. The exudate is centrifuged 5 min at 10,000 rpm to clear the cell debris. The volume of the supernate is measured and enzyme grade ammonium sulfate added to 25 % saturation (144 mg $(NH_4)_2SO_4$ per ml supernate). The mixture is stirred on ice for 15 min, then centrifuged for 5 min at 10,000 rpm. The pellet is discarded and the supernate adjusted to 50 % saturation with ammonium sulfate (158 mg $(NH_4)_2SO_4$ per ml supernate). After stirring on ice for 15 min, the pellet is collected by centrifugation at 10,000 rpm for 5 min. The obtained pellet is dissolved in 1-2 ml of column buffer. The extract is desalted by application to a Sephadex G50 column equilibrated with column buffer. The sample is eluted with column buffer, collecting approximately 2 ml of sample for each ml of sample applied to the column.

Homogenization buffer and column buffer are chosen depending on the source of the AHAS to be assayed: Tobacco homogenization buffer consists of: 50 mM $NaH_2PO_4$ buffer pH 7.0, 0.5 mM EDTA pH 7.0, 0.5 mM $MgCl_2$, 1 mM sodium pyruvate pH 7.0, 10 μM FAD, 1 mM PMSF, 10 % glycerol.
Maize homogenization buffer consists of: 50 mM Tris pH 7.5, 1 mM EDTA pH 7.5, 5 mM $MgCl_2$, 20 mM sodium pyruvate pH 7.0, 10 μM FAD, 1 mM PMSF, 1 mM DTT, 1 mM TPP, 10 % glycerol.
Tobacco column buffer consists of: 50 mM $NaH_2PO_4$ buffer pH 7.0, 0.1 mM EDTA, 0.5 mM $MgCl_2$, 10 μM FAD, 1 mM PMSF, 10 % glycerol.
Maize column buffer consists of: 50 mM Tris pH 7.5, 5 mM $MgCl_2$, 10 μM FAD, 0.1 mM EDTA, 1 mM PMSF, 1 mM DTT, 10 % glycerol.

## EXAMPLE 31: AHAS enzyme assay

### a) Tube assay:

5-50 μl of an extract prepared according to EXAMPLE 30 are dissolved in a total volume of 0.5 ml of reaction mixture (see below). The mixture is incubated for 30 min at 37°C. The reaction is stopped by the addition of 50 μl of 6 N $H_2SO_4$. The mixture is incubated for 15 min at 60°C, then treated with 625 μl of a-naphthol reagent (500 μl 5 % a-naphthol in 2.5 M NaOH and 125 μl 25 mg/ml creatine) and incubated another 15 min at 60°C. If a precipitate has formed, this is spun out, and the absorbance measured at 520 nm. The amount of acetoin formed is calculated from a standard curve developed using 0.4 μg to 10 μg acetoin. The specific activity is expressed as pKat acetoin per mg protein.

### b) ELISA plate assay:

1-20 μl of a sample are placed in a ELISA plate well with a total volume of 0.15 ml of reaction mixture. The mixture is reacted for 30 min at 37°C. The reaction is stopped by the addition of 50 μl of 2.4 M $H_2SO_4$ containing 1 % creatine. The mixture is incubated for 30 min at 60°C. Any precipitate present is separated by centri fugation. 150 μl of the assay mixture are transferred to a new ELISA plate, treated with 100 μl 10 % a-naphthol in 5M NaOH and incubated another 20 min at 60°C. The absorbance is measured at 520 nm, and the specific activity expressed as under a).

### c) Inhibition curves:

Ten point inhibition curves are generated by determining AHAS activity in the presence of sulfonylurea, imidazolinone and triazolopyrimidine herbicides. The sulfonylurea and triazolopyrimidine herbicides are included in the reaction mixture to give a range of final concentrations of the inhibitors between 1 nM and 10,000 nM. The imidazolinone herbicides are included in the concentration ranges of 10 nM to 100,000 nM. Inhibition of AHAS activity by isoleucine, leucine and valine is determined by the inclusion of each amino acid, separately and in all possible combinations, in the assay reaction mixture at final concentrations of 10, 20 or 30 mM.

The reaction mixture depends on the source of the AHAS to be assayed: Tobacco reaction mixture consists of: 20 mM $NaH_2PO_4$ buffer pH 7.0, 20 mM sodium pyruvate pH 7.0, 0.5 mM TPP, 5 mM $MgCl_2$, 10 μM FAD.
Maize reaction mixture consists of: 50 mM Tris pH 7.5, 10 mM $MgCl_2$, 10 μM FAD, 1 mM TPP, 70 mM sodium pyruvate pH 7.5.

TABLE 1: AHAS specific activity of wild-type and AHAS inhibitor tolerant tobacco cell lines[a]

| Strain designation[b] | AHAS specific activity[c] | % AHAS activity remaining with | | | | | |
| --- | --- | --- | --- | --- | --- | --- | --- |
| | | 4.2nM | .21µM SU872 | 10.6µM | 4.8nM | .24µM SU464 | 12.1µM |
| S3- | 61.8 | 80.5 | 17.4 | 14.3 | 92.9 | 44.2 | 24.0 |
| S3+ | 37.8 | 82.5 | 16.9 | | 61.0 | | |
| | | | | | | | |
| SR 102 | 85.6 | 78.8 | 14.4 | 10.8 | | | |
| SR 103 | 70.6 | 61.3 | 24.7 | 22.0 | | | |
| SR 118 | 118.6 | 97.4 | 38.0 | 22.5 | 95.6 | 40.6 | 22.6 |
| SR 126 | 90.3 | 57.3 | 8.6 | 5.8 | | | |
| SR 128 | 65.6 | 63.6 | 27.3 | 20.3 | | | |
| SR 139 | 106.6 | 105.0 | 42.0 | 29.8 | 91.0 | 44.2 | 27.5 |
| SR·143 | 78.8 | 100.0 | 59.2 | 56.5 | 100.0 | 45.3 | 31.7 |
| SR 147 | 104.7 | 61.0 | 15.6 | 12.1 | | | |
| SR 152 | 140.6 | 62.6 | 15.5 | 15.0 | 87.7 | 18.9 | 17.1 |
| SR 154 | 82.0 | 57.8 | 19.3 | 11.6 | 82.4 | 18.3 | 11.9 |
| SR 158 | 76.9 | 67.8 | 17.8 | 14.9 | | 17.9 | 12.0 |
| SR 163 | 69.4 | 71.0 | 32.6 | 27.6 | 79.6 | 32.8 | 23.5 |
| SR 164 | 121.7 | 55.2 | 20.9 | 15.1 | 77.8 | 20.7 | 15.1 |
| SR 168 | 105.3 | 64.6 | 29.3 | 21.5 | 79.0 | 28.0 | 21.9 |
| SR 173 | 86.4 | 51.8 | 25.5 | 18.3 | 85.8 | 32.8 | 18.1 |
| | | | | | | | |
| SR 1011 | 158.9 | 80.1 | 43.2 | 24.9 | 100.0 | 40.8 | 27.6 |
| | | | | | | | |
| SR 2002 | 119.8 | 45.2 | | | | | |
| SR 2003 | 93.3 | 19.8 | | | | | |
| SR 2005 | 97.1 | 35.4 | 29.4 | | | | |
| SR 2006 | 98.4 | 40.8 | 31.7 | | | | |
| SR 2008 | 70.0 | 64.3 | 16.1 | 13.6 | | | |
| SR 2012 | 114.8 | | 15.4 | 5.1 | | | |
| SR 2022 | 112.2 | | 26.9 | 23.0 | | | |
| | | | | | | | |
| Su 6 | 204 | | 27 | 12 | | 36 | 15 |
| Su 8 | 121 | | 32 | 12 | | 38 | 16 |
| Su 10 | 161 | | 39 | 15 | | 37 | 20 |
| Su 20 | 150 | | 18 | 17 | | 32 | 21 |
| Su 25 | 365 | | 20 | 18 | | 32 | 18 |
| Su 27 | 130 | | 36 | 20 | | 39 | 19 |
| Su 41 | 360 | | 15 | 12 | | 32 | 21 |

(Footnotes Table 1)

a The cell lines are subcultured for 2-3 days in MX1 medium containing 100 ppb SU872 (S3+ and SR lines) or 100 ppb

SU464 (Su lines) before assaying following the outline of EXAMPLE 31.

b S3- and S3+: wild type strain. S3- is subcultured without any herbicide before assaying.

SR 102-173: AHAS inhibitor tolerant strains obtained by the selection procedure outlined in EXAMPLE 2 using 100 ppb SU872.

SR 1011: AHAS inhibitor tolerant strain obtained by the selection procedure outlined in EXAMPLE 2 using 1000 ppb SU872.

SR 2002-2022: AHAS inhibitor tolerant strains obtained by the selection procedure outlined in EXAMPLE 2 and 4 using 100 ppb SU872 in the first selection and 1000 ppb SU872 in the second selection.

SU6-41: AHAS inhibitor tolerant strain obtained by the selection procedure outlined in EXAMPLE 3 using 100 ppb SU464.

c specific activity = pKat acetoin/mg protein.

The results presented in TABLE 1 demonstrate that tobacco cell lines can be found using the selection procedure of EXAMPLE 2, 3 and 4 which have elevated levels of AHAS enzyme but an AHAS sensitivity towards increasing levels of herbicidal sulfonylureas SU872 and SU464 resembling the sensitivity of the AHAS in the wild-type strain S3.

TABLE 2: AHAS specific activity, herbicide inhibition and amino acid feedback inhibition of AHAS inhibitor tolerant tobacco cell lines[a]

| Strain desig-nation[b] | AHAS specific activity[c] | % AHAS activity remaining with | | | | | | |
|---|---|---|---|---|---|---|---|---|
| | | SU872 | | SU464 | | Leu | Val | Ile |
| | | 0.21µM | 10.6µM | 0.21µM | 10.6µM | 10mM | 10mM | 10mM |
| S3 | 62 | 17 | 14 | 44 | 24 | 38 | 69 | 80 |
| Su 6 | 204 | 27 | 12 | 36 | 15 | 58 | 82 | 66 |
| Su 8 | 121 | 32 | 12 | 38 | 16 | 61 | 81 | 95 |
| Su 10 | 161 | 39 | 15 | 37 | 20 | 62 | 48 | 102 |
| Su 20 | 150 | 18 | 17 | 32 | 21 | 53 | 75 | 58 |
| Su 25 | 365 | 20 | 18 | 32 | 18 | 84 | 86 | 100 |
| Su 27 | 130 | 36 | 20 | 39 | 19 | 81 | 91 | 92 |
| Su 41 | 360 | 15 | 12 | 32 | 21 | 71 | 83 | 97 |

a The cell lines are subcultured for 2-3 days in MX1 medium conaining 100 ppb SU464 before assaying following the outline of EXAMPLE 31.

b SU 6-41: AHAS inhibitor tolerant strain obtained by the selection proedure outlined in EXAMPLE 3 using 100 ppb SU464.

c specific activity = pKat acetoin/mg protein.

The results presented in TABLE 2 demonstrate that the AHAS in cell lines with increased activity, but comparable sensitivity towards herbicides, show also feedback inhibition by valine, leucine and isoleucine which is comparable to the feedback inhibition of the AHAS enzyme in corresponding wild-type cells.

EXAMPLE 32: Northern analysis of AHAS message level

To determine whether the plants and the plant cell lines containing multiple copies of AHAS gene or chimeric AHAS gene(s) with strong promoters are indeed over-expressing the AHAS gene at the transcriptional level, the levels of AHAS polyA RNA from these plants and cell lines are compared with those of the controls, which are either identical or comparable plants or cell lines without extra copies of the AHAS gene or chimeric AHAS genes.

PolyA RNA is prepared from Arabidopsis plants, tobacco leaves, and tobacco calli. For each source, 25 g of tissue are used in the preparation following the procedures described by E. Back et al., Mol.Gen.Genet. 212: 20-26 (1987), except that the reaction volumes are scaled up to accommodate the larger amounts of tissue used.

1 µg of polyA RNA from each of the cell lines or plants and their controls are resolved on formaldehyde agarose gels and blotted to nitrocellulose membranes following procedures described by Maniatis et al. (supra, p. 202-203). In these experiments the electrophoresis runs are terminated and the gels are processed for blotting when the bromophenol blue dye fronts reach approximately three-quarters way down the length of each gels. The nitrocellulose membranes are wetted in 20 X SSC and prehybridized in LS hybridization buffer at 42°C. After 6 hours the membranes are transferred to fresh LS hybridization buffer containing $4 \times 10^6$ cpm per ml of the Arabidopsis AHAS ScaII-BgIII gene fragment probe of EXAMPLE 17. In these experiments the LS buffer is used at an amount of 0.2 ml per square cm of the membrane. The membranes are incubated in the hybridization solution for 16 hours at 42°C with gentle shaking, washed twice in room temperature 2 X SSC for 15 minutes per wash, washed three times in 42°C LS wash buffer (25 % formamide, 5 X SSC, 1 % SDS) for 2 hours per wash, and washed twice in room temperature Rinse buffer (0.1 X SSC, 0.1 % SDS) for 30 minutes per wash. Membranes are mounted and fluorographed with Cronex Lightening Plus® screens (Du Pont, Wilmington, DE) and XAR-5 film (Kodak, Rochester, NY) for 8 hours, 1 day, 3 days, and 9 days.

The activity for each hybridizing band on the blots is determined as follows: The XAR-5 films are scanned with the Zeineh 50ft Laser Scanning Densitometer Model SL-504-XL (Biomed Instrument Incorporated, Fullerton, CA). For each band, an exposure is chosen such that the peak intensity of the subject band is between 0.1 and 1 optical units (OD). With that exposure the total OD of that band is determined by integration across it. The total OD is multiplied by the exposure time to give the activity.

EXAMPLE 33: Southern analysis of tobacco cell DNA

DNA of the cells to be analyzed is prepared as described in EXAMPLE 17. 20 μg of this DNA are digested with 100 units of the restriction enzyme NcoI for a total of 4 hours. Digested DNA samples are extracted with phenol/chloroform, precipitated with ethanol and dissolved in TE buffer. The sample concentrations are determined with a UV spectrophotometer. 10 μg samples of digested DNA are analyzed on a TAE (40 mM Tris-acetate, pH 8.0, 1 mM EDTA) 0.8 % agarose gel. For calibration the gel contains copy number reconstructions consisting of XhoI-digested pCIB1209 (EXAMPLE 17) at the following levels of pCIB1209 DNA: 13 pg (2 copies), 32.5 pg (5 copies), 130 pg (20 copies). The calibration is based on $15 \times 10^6$ kbp as the size of the Nicotiana tabacum genome (Bennet and Smith, Proc.R.Soc.Lond. B 274: 227-274, 1976), and 9.7 kbp as the size of pCIB1209. The gel is treated with 0.25 N HCl for 15 min and the DNA samples are transferred from the gel to GeneScreen plusR (DuPont) by capillary transfer with 0.4 N NaOH for 60 hours. The membrane is washed with 2 X SSC, allowed to dry, and prehybridized in SW solution (1 % BSA, 0.5 M $NaH_2PO_4$, pH 7.0, 7 % SDS, 1 mM EDTA) at 55°C for 8 hours.

A tobacco AHAS gene probe is synthesized as follows: The 1.42 kbp NcoI-BglII tobacco SuRA fragment is isolated from pCIB1207 as described in EXAMPLE 15 for the isolation of the yeast AHAS gene fragment. 100 ng of this NcoI-BglII fragment are radiolabeled with the random priming reaction using the Prime Time® kit (International Biotechnologies Inc.) following the procedures recommended by the manufacturer. 100 μCi (3000 Ci/mMol) $\alpha$-$^{32}$P dCTP are used in a 100 μl reaction to achieve a specific activity of greater than 109 cpm/μg of DNA. The labeled DNA is separated from the unincorporated nucleotides with a run over a G50 spin column (Quick Spin Column, Boehringer Mannheim) according to procedures recommended by the manufacturer.

The radiolabeled DNA is heated in a boiling waterbath for 5 min, chilled on ice, and added to the prehybridization reaction to a final activity of $10^7$ cpm per ml. The blot is hybridized at 55°C with constant agitation. After 20 hours the blot is rinsed in 2 X SSC for 15 min , washed in 1 liter of 40 mM $NaH_2PO_4$, pH 7.0, 1 mM EDTA, 1 % SDS, 0.125 M NaCl for 90 min, and rinsed in 2 X SSC, all at room temperature. The membrane is mounted wet between two sheets of Saran® wrap (Union Carbide) and fluorographed with a Cronex Lightening Plus® screen (Dupont) on XAR-5 film (Kodak). One hour, 16 hour and 4 day exposures of the blot are taken.

The identities of the bands appearing in the fluorograms are assigned based upon the restriction map of K. Lee et al., EMBO J. 7: 1241-1248 (1988). The tobacco SuRA gene give rises to a band at approximately 4.55 kbp position and the SuRB gene give rise to a band at approximately 5.9 kbp position. The activities of the SuRA and SuRB gene for each sample and the copy number reconstructions are determined in the following way: The XAR-5 films are scanned with the Zeineh Soft Laser Scanning Densitometer Model SL-504-XL (Biomed Instrument Incorporated, Fullerton CA). For each band, an exposure is chosen such that the peak intensity of the subject band is between 0.1 and 1 optical units (OD), and the total OD of that band is determined by integration across it. The total OD is multiplied by the exposure time to give the activity. A standard curve is drawn up based upon the activities of the SuRA gene in the copy number reconstructions and the activity of each gene band is interpolated to determine its copy number of the sample examined.

EXAMPLE 34: Characterization of transgenic tobacco plants containing a CaMV 35S wild-type Arabidopsis AHAS chimeric gene

a) Herbicide tolerance of transgenic tobacco plants:

The herbicide tolerance of the transgenic plants obtained according to EXAMPLE 23 and 24 using CGA1402 are essentially determined as described in EXAMPLE 11a. Leaf discs are cut from different transgenic plants, weighed and then placed, lower side up, onto solidified MSBN medium containing 0, 10, 30, 100 and 300 ppb of SU872. The culture plates are incubated in the light at 25-28°C. At 2, 4, 6 and 8 weeks after plating, the discs are weighed to determine their fresh weight increase. Discs are inspected visually and evaluated for shoot and/or callus formation.

TABLE 3: Herbicide tolerance of transgenic tobacco plants[a]

| Plant type[b] | Plant number[c] | Concentration of SU872 [ppb] | | | |
|---|---|---|---|---|---|
| | | 0 | 10 | 30 | 100 |
| A | A3 | 2700±1130[d] 100 % | 2151±995 80 % | 180±98 6.7 % | 33±29 1.2 % |
| | A4 | 2991±901 100 % | 2847±548 95 % | 333±174 11 % | 19±15 0.6 % |
| B | B2 | 3076±898 | −[e] | − | − |
| C | C2 | 2520±1876 | −[e] | − | − |
| D | D2 | 2656±322 100 % | 70±14[e] 2.6 % | − | − |
| E | | 4338±1662 100 % | 87±19[e] 2 % | − | − |

a Havana 39 tobacco transformants selected on kanamycin and established as sterile shoot cultures. Leaf discs derived from different transgenic plants were plated (seven per plate) on the indicated levels of SU872. The mean fresh weight per disc at the time of plating was 3.14±0.43 mg.

b A Plant transformed with CGA1402, 35S/wild-type AHAS from Arabidopsis thaliana, EXAMPLE 16
B Plant transformed with a genomic clone of the wild type AHAS gene of Arabidopsis (sulfonylurea sensitive)
C Control plant transformed with pCIB200, kanamycin resistant
D Plant transformed with a genomic clone of AHAS from the chlorsulfuron tolerant mutant GH-50, G. Haughn et al., supra. Plants containing this gene are tolerant to chlorsulfuron but not to SU872.
E wild-type tobacco, cv. Havana 38

c 6 plants were regenerated from different transformed plant cells of each type A-D. Plant No. A3 and A4 proved to be herbicide tolerant wheras No. A1, A2, A5 and A6 were not.

d Mean mg fresh weight per disc ± standard deviation (n=7) as measured after 40 days; data also expressed as percent growth of control discs grown at 0 ppb.

e Leaf discs were bleached, had not enlarged nor formed any callus.

The results clearly demonstrate increased tolerance to SU872 of transgenic tobacco plants transformed with the AHAS gene construct comprising the AHAS gene of wild type, sulfonylurea sensitive Arabidopsis under the expression control of the 355 promoter from Cauliflower mosaic virus.

b) Southern analysis of transgenic tobacco plants:

DNA from tobacco upper leaves is isolated following procedures described for isolation of Arabidopsis plant DNA in EXAMPLE 15. DNA from control plants Havana 38 and from transgenic plants of EXAMPLE 23 and 24 prepared using CGA1402 (plant No. A3 and A4, see TABLE 3) is digested with restriction enzyme Ncol and subjected to Southern analysis following procedures described in EXAMPLE 33 with the following modifications: (1) The AHAS gene probe used is the 1.7 kb Scall/BglII Arabidopsis AHAS fragment. Its preparation and radiolabeling are done according to procedures described in EXAMPLES 15 and 17. (2) pCIB1212 plasmid DNA, of EXAMPLE 16, cut with restriction enzyme Ncol is used for the copy number reconstructions. The calculations for copy number reconstructions are done according to EXAMPLE 33 using 6.8 kbp as the size of pCIB1212. (3) 100 ng of the labeled fragment is used in the hybridization reaction. (4) The hybridization is done at 65°C. The washes used are: a) 2 X SSC at room temperature for 30 min. b) 1 liter of 3 X SSC, 1 % SDS for 2 hours at 65°C. c) 0.3 X SSC, 1 % SDS for 1 hour at 65°C. Fluorography of the Southern membrane and the analysis of the fluorograms are done according to procedures described in EXAMPLE 33.

The following results are observed with a 16 hour exposure and a 72 hour exposure of the Southern blot: The control tobacco Havana 38 DNA shows no hybridizing bands. The transformed tobacco plant DNAs (plant No. A3 and A4) show a band at 3 kbp which corresponds to the expected fragment containing the Arabidopsis AHAS coding sequence in the chimeric CaMV 35S Arabidopsis AHAS gene of pCIB1212 and pCIB1213 (EXAMPLE 16). Based upon comparisons of the intensity of the 3 kbp band in the A3 and A4 samples with those of the copy number reconstructions, it is estimated that the transformed plants contain a single copy of the CaMV 35S Arabidopsis AHAS gene per tetraploid genome.

EXAMPLE 35: Western analysis of transgenic tobacco plants containing a chimeric CaMV 35S wild-type Arabidopsis AHAS gene

a) Production of rabbit antisera to Arabidopsis AHAS peptides fragments:

ALS6, ALS8, and ALS9 peptide fragments corresponding to amino acid residues 674-689, 344-356, and 196-211, respectively, of the deduced amino acid sequence of the GH-50 Arabidopsis AHAS gene (B.J. Mazur et al., Plant Physiol. 85: 1110-1117, 1987; G.W. Haughn et al., Mol. Gen. Genet. 211: 266-271, 1988) are obtained from Dr. Dave Klapper (Department of Microbiology and Immunology, University of North Carolina Medical School, Chapel Hill, NC). The sequences of the ALS6, ALS8 and ALS9 peptides are GTFNDVITEGDGRIKY, GLGSYPCDDELSL, and GQVSRRMIGTDAFQET, respectively. The peptides are synthesized on a BioSearch Peptide Synthesizer using BOC-amino acids.

The ALS8 peptide, which contains a cysteine residue, is coupled to carrier proteins with N-succinimidyl 3-(2-pyridyldithio)propionate (SPDP) using a modification of the procedure of J. Carlsson et al., Biochem. J. 173: 723-737 (1978). Peptides coupled to ovalbumin are used as the immunogen while conjugates to bovine serum albumin (BSA) are used to assay the antisera. 100 mg of carrier protein (ovalbumin or BSA) is dissolved in 10 ml 0.01 MN-ethylmorpholine HCl, pH 7.5. 50 mg SPDP (Pierce Chemical Co.) is dissolved in 10 ml ethanol, and 2 ml aliquots are added to the protein solution at 3 min intervals. The reaction mix is rotated between additions. The modified protein is dialyzed against water to remove unreacted SPDP. If necessary 0.1% SDS is added to dissolve precipitated conjugate. The number of attached thiol groups is estimated from the optical density $OD_{343}$ after treatment of a small aliquot with an excess of dithiothreitol. A 1.5 molar excess of peptide over thiol groups is added to the remainder of the modified protein and allowed to react for 12 hours. The amount of bound peptide is estimated from the $OD_{343}$ at the end of the reaction and by amino acid analysis of the conjugate after separation of unbound peptide by gel filtration. Typically 4-10 moles of peptide are coupled to one mole of carrier.

The ALS6 and ALS9 peptides are coupled to KLH at 4°C in phosphate buffered saline (PBS) as described by Avravmeas and Ternyck, Immunochemistry 6: 53 (1969). The peptides are added as a 1 mg/ml suspension using 1 mg peptide per 3.3 mg KLH suspended in 1 ml PBS, which corresponds to a molar ratio of approx. 1:300. An equal volume of 0.2 % glutaraldehyde is added very slowly drop by drop to the constantly stirred peptide/KLH mixture. The reaction mixture is placed on a rotator at 4°C for 24 hours, then dialyzed overnight at 4°C against PBS. The total protein concentration in the mixture is calculated from the absorbance at 280 nm.

For the immunization of rabbits, an equal mixture of the peptide conjugates ALS6-KLH, ALS9-KLH and ALS8-ovalbumin is used. 1.5 mg of this mixture (corresponding to 0.5 mg of each peptide conjugate and approx. 160 μg of each ALS peptide fragment within these conjugates) in 1 ml PBS is emulsified with 1 ml complete Freund's adjuvant. 0.5 ml of the mix is injected subcutaneously into each shoulder, and 0.5 ml intramuscularly into the semi-tendinous/semi-membranous muscles of each hind leg. After 30 days a boost of another 1.5 mg antigen mixture emulsified in incomplete Freund's adjuvant is given subcutaneously into both shoulders. After a total of 37 days the animal is bled, and 30 ml of blood are taken by heart puncture. Serum is prepared in the usual way and frozen. After a further week, additional blood is taken. After three weeks, the animal is again boosted with more antigen and bled twice one and two weeks later.

The serum is tested against each individual antigen peptide fragment ALS6, ALS9 and ALS8 at a series ranging from 0.03 to 31.62 µg/ml free peptide in serum dilutions from 1:300 to 1:30,000, using a standard direct ELISA as described in M.-M. Cordonnier et al., Planta 158: 369-376 (1983). For the particular serum from rabbit No. 1986 (4 different batches from 4 bleedings) $A_{50}$, the half maximum activity expressed as the serum dilution at the peptide concentration of 3.16µg/ml, is found to be in the range of 1:2615 to 1:6000 for ALS6, 1:26,150 to better than 1:30,000 for ALS8, and 1:3000 to 1:5500 for ALS9.

b) Preparation of ammonium sulfate precipitated plant extracts:

Young leaves or roots from transformed tobacco plants No. A3 and A4 (TABLE 3) and from control tobacco Havana 38 are harvested, quick-frozen in liquid nitrogen, and stored at -80°C. The frozen tissue is added to a chilled blender cup containing 2 ml/g tissue of cold extraction buffer (86.4 mM $KH_2PO_4/K_2HPO_4$ pH 6.7, 2.0 mM sodium pyruvate (Boehringer Mannheim Biochemicals [BMB], Indianapolis, IN), 1.0 mM DTT, 10 µM FAD (BMB), 0.5 mM TPP (BMB), 0.5 mM EDTA, 0.5 mM $MgCl_2$, 10 % glycerol. Immediately prior to use, 2.0 mM benzamidine (Sigma Chemical Co., St. Louis, MO), 10.0 mM ε-amino-n-caproic acid (Sigma), 1.0 mM leupeptin (Sigma) and 1.0 mM PMSF (Sigma) are added). The mixture is ground to a slushy paste. The paste is filtered through Miracloth (Calbiochem-Behring Diagnostics, La Jolla, CA), the extract is collected, and the volume determined. The extract is brought to 20 % ammonium sulfate saturation by adding 114.0 mg of ultra pure ammonium sulfate (Schwarz/Mann Biotech, ICN Biomedicals, Cleveland, OH) per ml of extract. The solution is stirred on ice for 15 minutes to dissolve the ammonium sulfate during which time the pH is monitored and maintained at 6.7 by adding small amounts of a 2.5 M Tris solution. The extract is centrifuged at 20,000 x g (14,000 rpm in a Sorvall SA-600 rotor) for 15 minutes at 4°C. The supernatant is transferred to a fresh tube and the ammonium sulfate saturation increased to 30 % by adding 59.0 mg/ml ammonium sulfate. The ammonium sulfate is dissolved and the solution is centrifuged as described above. The supernatant is discarded and the pellet dried by inverting the tube in a 4°C cabinet. Any remaining supernatant is carefully removed with tissue paper. The pellet is resuspended in 1.5 ml 400 mM $KH_2PO_4/K_2PO_4$ buffer (pH 6.7) per 10 ml of original extract. The protein content is quantified with the BCA Protein Assay Reagent (Pierce Chemicals, Rockford, IL) using BSA as the concentration standard. The ammonium sulfate precipitate extract is stored at -80°C until assayed.

c) SDS polyacrylamide gel electrophoresis (SDS-PAGE) analysis of the plant extracts:

An SDS-PAGE sample is prepared containing 20 µg of protein in 10 µl of 400 mM $KH_2PO_4/K_2PO_4$ buffer (pH 6.7) from each extract. Each sample is mixed with an equal volume of 2 X sample buffer (0.1 M Tris-HCl pH 6.8, 20 % sucrose, 2.0 % SDS, 4.0% 2-mercaptoethanol (Sigma), 0.012 % bromophenol blue (Sigma)). The mixture is boiled for 5 minutes and loaded on a 4-20 % SDS-polyacrylamide gradient gel (84x70xl mm; Daiichi Pure Chemicals Co., Ltd., Tokyo, Japan). Prestained protein "Rainbow Markers" (Amersham) are included to estimate molecular weight. The gel is run in the SE200 miniature slab gel unit (Hoefer Scientific Instruments, San Francisco, CA) in 1 X PAGE buffer (0.025 M Tris-HCl pH 8.3, 0.192 M glycine, 0.1 M SDS). Electrophoresis is at 90 volts for 30 minutes, then at 120 volts for 1 hour or until the bromophenol blue dye reaches the end of the gel.

d) Western blotting:

The gel is blotted onto a nitrocellulose filter (0.45 µm pore size; Schleicher & Schuell, Inc., Keene, NH) using the LKB Multiphor II Nova Blot unit (Pharmacia LKB/Biotechnology, Uppsala, Sweden). Blotting is as per the manufacturer's instructions, using the continuous buffer system, for 90 minutes. Upon completion, transfer is verified by staining the filter with Ponceau S (Sigma Diagnostics, St. Louis, MO) as per the supplier's instructions.

e) Immunostaining:

The filter is preblocked by incubating at room temperature for 1 hour with shaking in 50 ml of dot blot blocking solution (0.14 M NaCl, 8 mM $Na_2HPO_4$ and 2mM $NaH_2PO_4$, pH to 7.3, 1.0 % BSA (blot-qualified, Promega, Madison, WI), 0.02 % $NaN_3$ (Sigma), 2.0% lamb serum (Gibco Laboratories Life Technologies Inc., Grand Island, NY)). The filter is washed twice for 10 minutes in 50 ml dot blot wash solution (10.0 mM Tris, 0.05 % Tween 20 (Promega), 0.02 % $NaN_3$ (Sigma), adjusted to pH 8.0, lamb serum (Gibco) to 1.0 % and BSA (Promega) to 0.25 %). For primary antibody binding, the filter is incubated for 45 minutes at room temperature in 10 ml of dot blot diluent containing 5 µl of the anti-AHAS antibody of serum No. 1986 (1:2000 dilution). Dot blot diluent is 0.01 M NaCl, 5.6 mM $Na_2HPO_4$ and 1.4 mM $NaH_2PO_4$ pH7.3, 1.0 %BSA (blot-qualified, Promega),0.05 % Tween 20 (Promega), 0.02 % $NaN_3$ Sigma), 10% lamb serum (Gibco), adjusted to pH 7.4 with 10 M NaOH. The filter is then washed twice as above. For secondary antibody binding, the filter is incubated 45 minutes at room temperature in 15 ml dot blot diluent (see above) containing 15 µl (dilution 1:1000) of anti-Rabbit IgG (whole molecule) Alkaline Phosphatase Conjugate (Sigma Chemical Co., St. Louis, MO). The filter is washed twice as above, then soaked for 5 minutes in alkaline phosphatase buffer (0.1 M Tris-HCl pH 9.5, 0.1 M NaCl, 5.0 mM $MgCl_2$). For the color development reaction, 48 µl of the BCIP substrate and 99 µl of the NBT substrate (Protoblot Immunoscreening Kit, Promega, Madison, WI) are added to 15 ml of alkaline phosphatase buffer (see above). The filter is placed in this solution and the reaction is allowed to proceed in a dark container, at room temperature, with gentle shaking. Color development is seen in approximately 5 minutes. The filter is rinsed with alkaline phosphatase buffer and photographed.

41

The transformed A3 and A4 root samples each yield a 65 kD (kilo-Dalton) band. The control tobacco Havana 38 does not. In addition to the 65 kD band all samples show a number of cross-reacting bands of less than 55 kD. The 65 kD band is approximately the expected size for the Arabidopsis AHAS protein.

EXAMPLE 36: Chemical induction in transgenic callus cultures and plants

Callus is assayed for chemical induction by applying with a sterile paint brush filter sterilized aqueous solutions of salicylic acid, benzoic acid, polyacrylic acid, and 1,2,3-benzothiazole-7-carboxylic acid, which have been adjusted to a pH between 6.0 and 8.0 by the addition of NaOH. The following concentrations are tested: 0.1 mM, 0.5 mM, 1 mM, 5 mM, 10 mM, and 50 mM. The callus is allowed to incubate for two days and then harvested, frozen in liquid nitrogen and stored at -80°C until assayed for gene induction as described in EXAMPLE 37.

Chemical induction in plants is obtained by applying the mentioned solutions as a fine spray to the leaves using a plant mister. The plants are allowed to continue to grow for two days and are then harvested, frozen in liquid nitrogen and stored at -80°C until assayed.

EXAMPLE 37: Assay for chemically inducible DNA sequences

Callus or plant material induced and harvested as described in EXAMPLE 36 is assayed for the induction of mRNA species by the isolation of RNA and the primer extension assay as described in EXAMPLE 38. In parallel callus or cell cultures obtained from the plant material are plated on solid MX1 medium containing 100 ppb SU872 or 100 ppb SU464 to check the tolerance to these herbicides.

EXAMPLE 38: Primer extension mapping

a) Synthesis and 5' end labeling of primers for primer extension:

For mapping AHAS transcripts 18-base oligonucleotides are synthesized, using an Applied Biosystems Synthesizer and b-cyanoethylphosphoramidite chemistry. The primer sequences are:
(a) Arabidopsis gene: 5'AGGAGATGGTTTGGTGGA3'
(b) Tobacco SuRA gene: 5'ATGTGGAGGAGGAAGGCG3'
(c) Tobacco SuRB gene: 5'GATTTGGAGGAGGAGGAC3'. The oligonucleotides are purified by reverse-phase high pressure liquid chromatography (HPLC). 5 pMol of either oligonucleotide are kinased (Maniatis et al., supra, p. 125) using 200 μCi of γ-$^{32}$P ATP (6000 Ci/mMol, 10 μCi/μl). After incubation at 37°C for 30 minutes, the reaction is diluted to 100 μl, the oligonucleotide extracted with phenol/chloroform and then precipitated three times with 50 μg carrier RNA. The final precipitate is resuspended in reverse transcriptase buffer (50 mM Tris-HCl, pH 7.5, 40 mM KCl, 3 mM MgCl) at a concentration of 2 nM. The specific activity of the labeled oligonucleotide is determined to be about 3 X 10$^6$ cpm/pMol.

b) Total RNA preparation:

Total RNA is prepared essentially as described by L.M. Lagrimini et al., Proc.Nat. Acad.Sci. USA 84: 7542 (1987). Tissue is ground under liquid nitrogen in a mortar and pestle. The ground tissue is added to grinding buffer (Lagrimini et al., supra) using 2.5 ml per gram tissue. An equal volume of phenol is then added and the emulsion is homogenized in a Brinkman polytron. A one-half volume of chloroform is added and the emulsion is gently mixed for 15 minutes. The phases are separated by centrifugation and the aqueous phase is removed. RNA is precipitated by the addition of sodium acetate to 0.3 M and 2.5 volumes ethanol. The precipitate is collected by centrifugation and resuspended in 2 ml sterile water. Lithium chloride is added to a final concentration of 3 M and left at 4°C overnight. The precipitate is collected by centrifugation and the pellet is washed with ice-cold 80% ethanol. The pellet is dried and resuspended in 500 μl sterile water. The concentration of this total RNA preparation is determined spectrophotometrically. Alternatively, RNA is extracted from callus as described above except that the callus tissue is cut into cubes approximately 3 mm in size, and added to pre-chilled mortars and pestles for grinding in liquid nitrogen prior to the polytron step.

c) Primer extension:

50 μg of total RNA is lyophilized in a 500 μl Eppendorf tube. The RNA is resuspended in 30 μl of radiolabeled oligonucleotide probe solution and heated to 70°C for 10 minutes. The tube is slowly cooled to 37°C and allowed to incubate overnight. Without removing the tube from the 37°C water bath, 2 μl of 10 X reverse transcriptase buffer (500 mM Tris-HCl, pH 7.5, 400 mM KCl, 30 mM MgCl$_2$), 1 μl 5 mg/ml bovine serum albumin, 5 μl 100 mM dithiothreitol, 5 μl 10 X dNTPs (10 mM of each dNTP in H$_2$O), 3 μl H$_2$O, 2 μl RNasin (80 units), and 2 μl reverse transcriptase (400 units) are added and the reaction is incubated at 37°C for 30 minutes. To stop the reaction, 5 μl of 3 M sodium acetate, pH 5, and 150 μl absolute ethanol are added. The tube is left at -20°C for 30 minutes, the precipitate is collected by centrifugation, washed with 80% ethanol and allowed to air-dry. The precipitate is resuspended in 10-20 μl of loading dye (90% formamide, 0.05% bromophenol blue, 0.05% xylene cyanol, 1 mM EDTA) and the extension products are separated on a 6% sequencing gel (Maniatis et al., supra). Extension products are visualized by autoradiography.

EXAMPLE 39: S1 nuclease mapping

Plasmids pCIB1209 and pCIB1210 (EXAMPLE 17) containing the tobacco SuRA and SuRB gene, respectively, are digested with restriction endonuclease Ncol, dephosphorylated with calf intestinal phosphatase and kinased with $\gamma^{-32}P$ ATP. Following phenol extraction and ethanol precipitation, the DNA is digested with BamHI and EcoRI, respectiviely, and the fragments of 534 bp and 484 bp, respectively, are isolated after electrophoresis on a low gelling temperature agarose gel. The probe is resuspended in formamide hybridization buffer (A.J. Berk et al., Cell 12: 721, 1977) at a concentration of about 2 nM. The specific activity of the probe is about 5 X 10 cpm/pMol. Lyophilized total RNA (50 µg) is dissolved in 30 µl of the probe solution, and the tubes are heated to 65°C for 10 minutes, then allowed to hybridize overnight at 48°C. S1 nuclease treatment and gel electro phoresis are essentially as described, using an S1 concentration of 400 units/ml and an incubation temperature of 30°C. The appropriate S1 nuclease concentration is determined- in pilot experiments.

The 3, ends of the SuRA and SuRB genes are also mapped accord ing to this procedure using Bcli/Xhol and Bglll/BamHI fragments, respectively.

EXAMPLE 40: Mapping the transcriptional start site

The transcriptional start site for the AHAS gene is determined by a combination of S1 nuclease mapping and primer extension analysis. An autoradiogram of a primer extension experiment using RNA isolated from leaves of CaMV 35S and Arabidopsis AHAS transformed plants and the Arabidopsis primer demonstrates the presence of a 157 bp band. The primer itself is labeled at the 5' phosphate, therefore the size of the extension product indicates that the mRNA is initiated at the expected site in the 35S promoter and contains 89 bp derived from the promoter and 68 bp derived from the Arabidopsis gene. Similarly the predicted size fragments are detected when the tobacco SuRA and SuRB primers are used.

However, primer extension analysis alone cannot be used to identify the 5' end of a mRNA. For instance, the mRNA may contain a 5' end that has been spliced from an upstream location. To determine conclusively the 5' end, high resolution S1 nuclease mapping is used in conjunction with primer extension. A Ncol fragment is labeled at the 5, end and digested with BamHI or EcoRI, respectively, to yield strand-specific probes for the tobacco SuRA and SuRB genes, respectively. These probes are used to map the 5, end of AHAS transcripts in RNA isolated from transgenic tobacco and corn leaves.

## Claims

1. A plant cell which is tolerant to a herbicidal acetohydroxyacid synthase (AHAS) inhibitor wherein said tolerance is caused by an increased level of AHAS enzyme.

2. The plant cell of claim 1 wherein said tolerance is caused by an increased level of AHAS enzyme and said AHAS essentially has the properties of a wild-type AHAS.

3. The plant cell of claim 2 wherein said AHAS essentially has the properties of a wild-type AHAS derived from a plant cell which is sensitive to said herbicidal AHAS inhibitor.

4. The plant cell of claim 1 wherein said increased level of AHAS enzyme is at least twofold higher than the level of AHAS enzyme in otherwise identical, naturally occurring plant cells sensitive to said herbicidal AHAS inhibitor.

5. The plant cell of claim 1 wherein said increased level of AHAS enzyme is at least tenfold higher than the level of AHAS enzyme in otherwise identical, naturally occurring plant cells sensitive to said herbicidal AHAS inhibitor.

6. The plant cell of claim 1 wherein said increased level of AHAS enzyme is due to increased expression of an endogenous AHAS coding sequence.

7. The plant cell of claim 1 wherein said increased level of AHAS enzyme is due to multiple copies of an endogenous AHAS gene.

8. The plant cell of claim 1 wherein said increased level of AHAS enzyme is due to a mutation in the non-coding sequence of an endogenous AHAS gene.

9. The plant cell of claim 1 wherein said increased level of AHAS enzyme is due to an exogenous AHAS gene.

10. The plant cell of claim 9 wherein said exogenous AHAS gene codes for an AHAS which essentially has the properties of a wild-type AHAS.

11. The plant cell of claim 10 wherein the coding sequence of said exogenous AHAS gene is derived from a plant cell which is sensitive to said herbicidal AHAS inhibitor.

12. The plant cell of claim 1 having stably incorporated a chimeric DNA construct comprising a DNA sequence coding for an AHAS functional in said plant cell operably linked to a promoter providing increased expression of said AHAS.

13. The plant cell of claim 1 having stably incorporated a chimeric DNA construct comprising a DNA sequence coding for an AHAS functional in said plant cell and a DNA sequence coding for a transit peptide, said coding sequences being operably linked to a promoter providing increased expression of said AHAS.

14. The plant cell of claim 12 wherein the promoter is a chemically regulatable promoter.

15. The plant cell of claim 1 which is a monocotyledonous plant cell.

16. The plant cell of claim 1 which is a dicotyledonous plant cell.

17. The plant cell of claim 1 which is selected from the group consisting of corn, wheat, barley, rice, sorghum, sugarcane, sugarbeet, soybean, Brassica, sunflower, carrot, tobacco, lettuce, cucumber, tomato, potato, cotton, Arabidopsis, Lolium, Festuca, Dactylis, and poplar plant cells.

18. The plant cell of claim 1 which is tolerant to a sulfonylurea herbicide.

19. The plant cell of claim 18 which is tolerant to a herbicide selected from the group consisting of N-o-methoxy-and ethoxycarbonylphenylsulfonyl-N'-(difluoromethoxy substituted pyrimidinyl)-ureas, N-o-(2-methoxy- and 2-ethoxyethoxy)-phenylsulfonyl-N'-(methyl and/or methoxy substituted triazinyl)-ureas, and N-o-(2-chloroethoxy)-phenylsulfonyl-N'-(methyl and/or methoxy substituted triazinyl)-ureas.

20. The plant cell of claim 1 which is tolerant to a imidazolinone herbicide.

21. The plant cell of claim 1 which is tolerant to a triazolopyrimidine herbicide.

22. A plant which is tolerant to a herbicidal acetohydroxyacid synthase (AHAS) inhibitor wherein said plant comprises a plant cell of claim 1.

23. The plant of claim 22 which comprises a plant cell of claim 14.

24. Seed and other propagules of a plant of claim 22.

25. A chimeric DNA construct comprising a DNA sequence coding for a wild-type AHAS operably linked to a promoter, wherein said promoter is different from the promoter linked to said AHAS in the wild-type gene.

26. The chimeric DNA construct of claim 25 wherein said promoter is functional in plant cells.

27. The chimeric DNA construct of claim 26 comprising a DNA sequence coding for a wild-type AHAS and a DNA sequence coding for a transit peptide operably linked to a promoter, wherein said promoter is different from the promoter linked to said AHAS in the wild-type gene.

28. The chimeric DNA construct of claim 26 wherein said promoter is regulatable.

29. The chimeric DNA construct of claim 28 wherein said promoter is chemically regulatable.

30. The chimeric DNA construct of claim 28 wherein said promoter is regulatable by light.

31. The chimeric DNA construct of claim 28 wherein said promoter is regulatable by heat.

32. The chimeric DNA construct of claim 26 wherein said promoter is a cauliflower mosaic virus (CaMV) 355 promoter.

33. The chimeric DNA construct of claim 29 wherein said promoter is a pathogenesis-related (PR) protein promoter.

34. The chimeric DNA construct of claim 27 wherein said transit peptide is a chloroplast transit peptide.

35. The chimeric DNA construct of claim 25 comprising a DNA sequence coding for a wild-type AHAS derived from tobacco (Nicotiana tabacum) which is sensitive to a herbicidal AHAS inhibitor.

36. The chimeric DNA construct of claim 25 comprising a DNA sequence coding for a wild-type AHAS derived from Arabidopsis thaliana which is sensitive to a herbicidal AHAS inhibitor.

37. The chimeric DNA construct of claim 25 comprising a DNA sequence coding for a wild-type AHAS derived from corn (Zea mays) which is sensitive to a herbicidal AHAS inhibitor.

38. The chimeric DNA construct of claim 27 comprising a DNA sequence coding for a wild-type AHAS and a DNA sequence coding for a chloroplast transit peptide wherein said DNA sequences are derived from tobacco (Nicotiana tabacum) which is sensitive to a herbicidal AHAS inhibitor.

39. The chimeric DNA construct of claim 25 comprising a DNA sequence coding for a selectable marker.

40. The chimeric DNA construct of claim 39 wherein the selectable marker is hygromycin resistance.

41. A recombinant DNA comprising multiple copies of a wild-type AHAS coding sequence.

42. The recombinant DNA of claim 41 comprising multiple copies of a wild-type AHAS gene.

43. The recombinant DNA of claim 41 wherein the AHAS coding sequence is derived from tobacco (Nicotiana tabacum) which is sensitive to a herbicidal AHAS inhibitor.

44. The recombinant DNA of claim 41 comprising a DNA sequence coding for a selectable marker.

45. The recombinant DNA of claim 44 wherein the selectable marker is hygromycin resistance.

46. A recombinant DNA comprising a chimeric DNA construct of claim 26 which is a vector for the transformation of plant cells.

47. The recombinant DNA of claim 46 comprising an origin of replication functional in a microorganism.

48. The recombinant DNA of claim 47 comprising an origin of replication functional in E. coli.

49. The recombinant DNA of claim 47 comprising an origin of replication functional in Agrobacterium.

50. A recombinant DNA comprising a DNA of claim 41 which is a vector for the transformation of plant cells.

51. A recombinant DNA comprising a DNA sequence coding for a wild-type AHAS of corn (Zea mays).

52. The recombinant DNA of claim 51 comprising a wild-type AHAS gene of corn.

53. The recombinant DNA of claim 51 which is the plasmid pCIB1253.

54. The recombinant DNA of claim 51 which is the plasmid pCIB1255.

55. The recombinant DNA of claim 51 comprising the coding sequence of the DNA sequence of Figure 9.

56. The recombinant DNA of claim 51 comprising the coding sequence of the DNA sequence of Figure 10.

57. A method of preparation of a plant cell of claim 1 wherein

(a) wild-type plant cells are cultured in the presence of increasing amounts of a herbicidal AHAS

44

inhibitor,

(b) surviving plant cells from step (a) are subcultured in the presence of said herbicidal AHAS inhibitor,

(c) the plant cells obtained in step (b) are analyzed for the presence of AHAS having essentially wild-type AHAS characteristics and for increased level of AHAS enzyme, and

(d) the plant cells having the desired properties mentioned in step (c) are selected and cultured.

58. A method of preparation of a plant cell of claim 1 wherein a plant cell is transformed with a recombinant DNA of claim 46.

59. A method of preparation of a plant cell of claim 1 wherein a plant cell is transformed with a recombinant DNA of claim 50.

60. A method of selecting plant cells representing a desired genotype wherein a mixture of plant cells comprising cells of claim 1 are cultured in the presence of a herbicidal AHAS inhibitor and surviving cells are isolated.

61. A method of controlling weed in a plantation comprising plants of claim 22 tolerant to a herbicidal acetohydroxyacid synthase (AHAS) inhibitor wherein all plants in said plantation are contacted with a plant controlling amount of said herbicidal AHAS inhibitor.

62. The method of claim 61 of controlling weed in a plantation comprising plants of claim 23 in which the tolerance to a herbicidal AHAS inhibitor is regulatable by a chemical regulator, wherein all plants in said plantation are contacted with said chemical regulator and simultaneously or consecutively with a plant controlling amount of said herbicidal AHAS inhibitor.

63. Process for producing a chimeric DNA construct capable of conferring tolerance to a herbicidal acetohydroxyacid synthase (AHAS) inhibitor to a plant, which process comprises the following steps:

(a) isolating a first DNA component sequence coding for a wild-type AHAS from its natural source or from a genomic or cDNA library; and

(b) operably linking the DNA component sequence of step (a) to a promoter which is different from the promoter linked to said AHAS in the wild-type gene but is functional in plants, by ligating them concurrently or consecutively.

64. Process for producing a chimeric DNA construct capable of conferring tolerance to a herbicidal acetohydroxyacid synthase (AHAS) inhibitor to a plant, which process comprises the following steps:

(a) isolating a first DNA component sequence coding for a wild-type AHAS from its natural source or from a genomic or cDNA library;

(b) isolating a second DNA component sequence conding for a transit peptide;

(c) ligating the component sequences of step (a) and (b)

(d) operably linking the resulting DNA sequence of step (c) to a promoter which is different from the promoter linked to said AHAS in the wild-type gene but is functional in plants, by ligating them concurrently or consecutively.

4.0 kbp XbaI insert of pCIB1253

FIG. 1

7.5 kbp EcoRI insert of pCIB1255

EcoRI — Psti

HindIII —

SaII —

HindIII —

BamHI —

KpnI —

1 kbp

EcoRI —

FIG. 2

CIsqb-20.Seq  Length: 435

```
  1  AAGCTTAACA TCTGCACAGA TGGACACATG TGGCTGCTTG TTCTTGCCAA
 51  TCTCAGCCGG ATCAATATCA ACGTGCACAA TCTTAGCCCT GCTTGCAAAA
101  GCCTCAATCT TCCCTGTCAC ACGATCATCA AACCGCACAC CAAGTGCAAG
151  CAACAGATCG GCCTTATCCA CTGCATAATT TGCATACACC GTGCCATGCA
201  TACCTAGCAT GCGCAGAGAC AGTGGGTCGT CGCTGGGGAA GTTGCCGAGG
251  CCCATAAGAG TAGTTGTGAC CGGGATTCCA GTCAGCTCCA CAAAGCGTCG
301  CAACTCCTCA CCAGATGCTG CGCAGCCACC GCCAACATAA AGAACAGGGC
351  GCCGGGATTC ACCAACAAGA CGCAGCACCT CGTCAAGCAA CTCAGTCGCA
401  GGGGGCTTGG GAAGGCGCGC AATGTACCCA GGCAG
```

# FIG. 3

```
     AAGCTTAACATCTGCACAGATGGACACATGTGGCTGCTTGTTCTTGCCAATCTCAGCCGG
  1  ---------+---------+---------+---------+---------+---------+ 60
     TTCGAATTGTAGACGTGTCTACCTGTGTACACCGACGAACAAGAACGGTTAGAGTCGGCC

      L   K   V   D   A   C   I   S   V   H   P   Q   K   N   K   G   I   E   A   P   -

     ATCAATATCAACGTGCACAATCTTAGCCCTGCTTGCAAAAGCCTCAATCTTCCCTGTCAC
 61  ---------+---------+---------+---------+---------+---------+ 120
     TAGTTATAGTTGCACGTGTTAGAATCGGGACGAACGTTTTCGGAGTTAGAAGGGACAGTG

      D   I   D   V   H   V   I   K   A   R   S   A   F   A   E   I   K   G   T   V   -

     ACGATCATCAAACCGCACACCAAGTGCAAGCAACAGATCGGCCTTATCCACTGCATAATT
121  ---------+---------+---------+---------+---------+---------+ 180
     TGCTAGTAGTTTGGCGTGTGGTTCACGTTCGTTGTCTAGCCGGAATAGGTGACGTATTAA

      R   D   D   F   R   V   G   L   A   L   L   L   D   A   K   D   V   A   Y   N   -

     TGCATACACCGTGCCATGCATACCTAGCATGCGCAGAGACAGTGGGTCGTCGCTGGGGAA
181  ---------+---------+---------+---------+---------+---------+ 240
     ACGTATGTGGCACGGTACGTATGGATCGTACGCGTCTCTGTCACCCAGCAGCGACCCCTT

      A   Y   V   T   G   H   M   G   L   M   R   L   S   L   P   D   D   S   P   F   -

     GTTGCCGAGGCCCATAAGAGTAGTTGTGACCGGGATTCCAGTCAGCTCCACAAAGCGTCG
241  ---------+---------+---------+---------+---------+---------+ 300
     CAACGGCTCCGGGTATTCTCATCAACACTGGCCCTAAGGTCAGTCGAGGTGTTTCGCAGC

      N   G   L   G   M   L   T   T   T   V   P   I   G   T   L   E   V   F   R   R   -

     CAACTCCTCACCAGATGCTGCGCAGCCACCGCCAACATAAAGAACAGGGCGCCGGGATTC
301  ---------+---------+---------+---------+---------+---------+ 360
     GTTGAGGAGTGGTCTACGACGCGTCGGTGGCGGTTGTATTTCTTGTCCCGCGGCCCTAAG

      L   E   E   G   S   A   A   C   G   G   G   V   Y   L   V   P   R   R   S   E   -

     ACCAACAAGACGCAGCACCTCGTCAAGCAACTCAGTCGCAGGGGGCTTGGGAAGGCGCGC
361  ---------+---------+---------+---------+---------+---------+ 420
     TGGTTGTTCTGCGTCGTGGAGCAGTTCGTTGAGTCAGCGTCCCCCGAACCCTTCCGCGCG

      G   V   L   R   L   V   E   D   L   L   E   T   A   P   P   K   P   L   R   A   -

     AATGTACCCAGGCAG
421  ---------+----- 435
     TTACATGGGTCCGTC

      I   Y   G   P   L   -
```

# FIG. 4

```
267 LPGYMSRLPKLPNEMLLEQIVRLISESKKPVLYVGGGCSQSSEELRRFVE 316
    |||||  ||||  |        |||||||||||||||||||||||||  ||||||||||
  1 LPGYIARLPKPPATELLDEVLRLVGESRRPVLYVGGGCAASGEELRRFVE 50
    |||||  ||||||         |||||||||||||||||||||||||  ||||| ||||
273 LPGYMSRMPKPPEDSHLEQIVRLISESKKPVLYVGGGCLNSSDELGRFVE 322
```

```
317 LTGIPVASTLMGLGAFPTGDELSLSMLGMHGTVYANYAVDSSDLLLAFGV 366
    ||||||  ||||||  ||  ||  |||  |||||||||||||||  |||||||||
 51 LTGIPVTTTLMGLGNFPSDDPLSLRMLGMHGTVYANYAVDKADLLLALGV 100
    ||||||  ||||||  |||||  ||||||||||||||||||  |||||||||
323 LTGIPVASTLMGLGSYPCDDELSLHMLGMHGTVYANYAVEHSDLLLAFGV 372
```

```
367 RFDDRVTGKLEAFASRAKIVHIDIDSAEIGKNKQPHVSICADIKL 411
    |||||||||||||||||||||||||||||  |||||||||||||||||||||||
101 RFDDRVTGKIEAFASRAKIVHVDIDPAEIGKNKQPHVSICADVKL 145
    ||||||||||||||||||||||||||||  |||||||  |||||||||||
373 RFDDRVTGKLEAFASRAKIVHIDIDSAEIGKNKTPHVSVCGDVKL 417
```

# FIG. 5

C3sa16seq.Dat   Length: 375

```
  1   TTGCTTTGCA GGGCATGAAT ACTCTTCTGG AAGGAAGCAC ATCAAAGAAG

 51   AGCTTTGACT TCGGCTCATG GCATGATGAA TTGGATCAGC AAAAGCGGGA

101   GTTTCCCCTT GGGTATAAAA TCTTCAATGA GGAAATCCAG CCACAATATG

151   CTATTCAGGT TCTTGATGAG TTGACGAAGG GGAAGGCCAT CATTGCCACA

201   GGTGTTGGGC AGCACCAGAT GTGGGCGGCA CAGTATTACA CTTACAAGCG

251   GCCAAGGCAG TGGCTGTCTT CAGCTGGTCT TGGGGCTATG GGATTTGGTT

301   TGCCGGCTGC TGCTGGTGCT GCTGTGGCCA ACCCAGGTGT CACTGTTGTT

351   GACATCGACG GAGATGGTAG CTTCC
```

# FIG. 6

```
     TTGCTTTGCAGGGCATGAATACTCTTCTGGAAGGAAGCACATCAAAGAAGAGCTTTGACT
  1  ---------+---------+---------+---------+---------+---------+  60
     AACGAAACGTCCCGTACTTATGAGAAGACCTTCCTTCGTGTAGTTTCTTCTCGAAACTGA

      A   L   Q   G   M   N   T   L   L   E   G   S   T   S   K   K   S   F   D   F   -

     TCGGCTCATGGCATGATGAATTGGATCAGCAAAAGCGGGAGTTTCCCCTTGGGTATAAAA
 61  ---------+---------+---------+---------+---------+---------+  120
     AGCCGAGTACCGTACTACTTAACCTAGTCGTTTTCGCCCTCAAAGGGGAACCCATATTTT

      G   S   W   H   D   E   L   D   Q   Q   K   R   E   F   P   L   G   Y   K   I   -

     TCTTCAATGAGGAAATCCAGCCACAATATGCTATTCAGGTTCTTGATGAGTTGACGAAGG
121  ---------+---------+---------+---------+---------+---------+  180
     AGAAGTTACTCCTTTAGGTCGGTGTTATACGATAAGTCCAAGAACTACTCAACTGCTTCC

      F   N   E   E   I   Q   P   Q   Y   A   I   Q   V   L   D   E   L   T   K   G   -

     GGAAGGCCATCATTGCCACAGGTGTTGGGCAGCACCAGATGTGGGCGGCACAGTATTACA
181  ---------+---------+---------+---------+---------+---------+  240
     CCTTCCGGTAGTAACGGTGTCCACAACCCGTCGTGGTCTACACCCGCCGTGTCATAATGT

      K   A   I   I   A   T   G   V   G   Q   H   Q   M   W   A   A   Q   Y   Y   T   -

     CTTACAAGCGGCCAAGGCAGTGGCTGTCTTCAGCTGGTCTTGGGGCTATGGGATTTGGTT
241  ---------+---------+---------+---------+---------+---------+  300
     GAATGTTCGCCGGTTCCGTCACCGACAGAAGTCGACCAGAACCCCGATACCCTAAACCAA

      Y   K   R   P   R   Q   W   L   S   S   A   G   L   G   A   M   G   F   G   L   -

     TGCCGGCTGCTGCTGGTGCTGCTGTGGCCAACCCAGGTGTCACTGTTGTTGACATCGACG
301  ---------+---------+---------+---------+---------+---------+  360
     ACGGCCGACGACGACCACGACGACACCGGTTGGGTCCACAGTGACAACAACTGTAGCTGC

      P   A   A   A   G   A   A   V   A   N   P   G   V   T   V   V   D   I   D   G   -

     GAGATGGTAGCT
361  ---------+--
     CTCTACCATCGA

      D   G   S   F
```

## FIG. 7

```
412 ALQGLNSILESKEGKLKLDFSAWRQELTVQKVKYPLNFKTFGDAIPPQYA 461
    ||||||  ||||   ||    ||||  |||||   ||   |||  || | | | ||||
  1 ALQGMNTLLEGSTSKKSFDFGSWHDELDQQKREFPLGYKIFNEEIQPQYA 50
    ||||||  |||        ||||  ||||||   ||   |||||| | | | ||||
418 ALQGMNKVLENRAEELKLDFGVWRNELNVQKQKFPLSFKTFGEAIPPQYA 467
```

```
462 IQVLDELTNGSAIISTGVGQHQMWAAQYYKYRKPRQWLTSGGLGAMGFGL 511
    ||||||||  |  |||  |||||||||||||||  ||||||||  |||||||||||
 51 IQVLDELTKGKAIIATGVGQHQMWAAQYYTYKRPRQWLSSAGLGAMGFGL 100
    |  ||||||  |||||  |||||||||||||||  |||||||||||||||||||||
468 IKVLDELTDGKAIISTGVGQHQMWAAQFYNYKKPRQWLSSGGLGAMGFGL 517
```

```
512 PAAIGAAVGRPDEVVVDIDGDGSF 535
    |||  ||||||  ||    ||||||||||
101 PAAAGAAVANPGVTVVDIDGDGSF 124
    |||  ||  |||||     ||||||||||
518 PAAIGASVANPDAIVVDIDGDGSF 541
```

# FIG. 8

```
     TCTAGAGCTGTCGACAATTAACCCTCACTAAAGGGAACGAATTCGGATCACCTATCAACA
1    ---------+---------+---------+---------+---------+---------+    60
     AGATCTCGACAGCTGTTAATTGGGAGTGATTTCCCTTGCTTAAGCCTAGTGGATAGTTGT

     TCCCAGCTAAAAACAGTAAAAAGGGGGAAAACGTGGGTGAGTTGAGTCTGTCTTGTGGAA
61   ---------+---------+---------+---------+---------+---------+    120
     AGGGTCGATTTTTGTCATTTTTCCCCCTTTTGCACCCACTCAACTCAGACAGAACACCTT

     AAAACGTTTTAGTTTCTCCTGGAATTAACAATAAAAACAGTTGAACAAGATTGACTGTTC
121  ---------+---------+---------+---------+---------+---------+    180
     TTTTGCAAAATCAAAGAGGACCTTAATTGTTATTTTTGTCAACTTGTTCTAACTGACAAG

     CTCCGGGAGGGTTTGGAACATCGTTACAGATGTGAGCGAAAGGTGAGGAAACAGAGCGGA
181  ---------+---------+---------+---------+---------+---------+    240
     GAGGCCCTCCCAAACCTTGTAGCAATGTCTACACTCGCTTTCCACTCCTTTGTCTCGCCT

     GGGCTTGGAGGTGACCTCGGTAGTCGACGCCGGAGTTGAGCTTGATGACGACACCGTACT
241  ---------+---------+---------+---------+---------+---------+    300
     CCCGAACCTCCACTGGAGCCATCAGCTGCGGCCTCAACTCGAACTACTGCTGTGGCATGA

     GGCGTACCAGGCCTAGTAGTGAACACCGGGCCTGAAGCTGTCGCCGCCGCTGCTCATCTT
301  ---------+---------+---------+---------+---------+---------+    360
     CCGCATGGTCCGGATCATCACTTGTGGCCCGGACTTCGACAGCGGCGGCGACGAGTAGAA

     GTGGCTGTGCCCGGTGTCCCTGTTGCGGATTGCGGGTGGACAGCCTGGCAGGTGGGTGCG
361  ---------+---------+---------+---------+---------+---------+    420
     CACCGACACGGGCCACAGGGACAACGCCTAACGCCCACCTGTCGGACCGTCCACCCACGC

     ACCCGTTTGGACTCCCTGATCTGGGCCCTTTGTGTCAGTACCGTCTGTACTCCGATGACA
421  ---------+---------+---------+---------+---------+---------+    480
     TGGGCAAACCTGAGGGACTAGACCCGGGAAACACAGTCATGGCAGACATGAGGCTACTGT

     TGCACACCGTCGTCCACAGTCAAGTCCACAATCTCCCCTCTTTTTTTTAACGGATAGTTC .
481  ---------+---------+---------+---------+---------+---------+    540
     ACGTGTGGCAGCAGGTGTCAGTTCAGGTGTTAGAGGGGAGAAAAAAAATTGCCTATCAAG

     AAAATCTCCTTGACGCACGCTATCGTGTACCAGCGCTCACTGGACACCACGTTTGTAATC
541  ---------+---------+---------+---------+---------+---------+    600
     TTTTAGAGGAACTGCGTGCGATAGCACATGGTCGCGAGTGACCTGTGGTGCAAACATTAG

     CACGCGACACGTCGGTCCCACGTCGACAGGCCCCACCGTCCGGTCTGTAGCGTGTACGTA
601  ---------+---------+---------+---------+---------+---------+    660
     GTGCGCTGTGCAGCCAGGGTGCAGCTGTCCGGGGTGGCAGGCCAGACATCGCACATGCAT

     TTCGGGCGACGGACGTGTCGTCGTCGTCTTGCGAGTCCCATTCCCATCACCATCTGAGCC
661  ---------+---------+---------+---------+---------+---------+    720
     AAGCCCGCTGCCTGCACAGCAGCAGCAGAACGCTCAGGGTAAGGGTAGTGGTAGACTCGG

     ACACATCCTCTGAACAAAAGCAGGGAGGCCTCTACGCACATCCCCCTTGCTCCCACTCCG
721  ---------+---------+---------+---------+---------+---------+    780
     TGTGTAGGAGACTTGTTTTCGTCCCTCCGGAGATGCGTGTAGGGGGAACGAGGGTGAGGC
```

# FIG. 9 (a)

```
      TGTCCGTGGCACCCACCCCAAACCCTCGCGCCGCCTCCGAGACAGCCGCCGCAACCATGG
781   ---------+---------+---------+---------+---------+---------+      840
      ACAGGCACCGTGGGTGGGGTTTGGGAGCGCGGCGGAGGCTCTGTCGGCGGCGTTGGTACC
                                                                  M  A

      CCACCGCCGCCGCCGCGTCTACCGCGCTCACTGGCGCCACTACCGCTGCGCCCAAGGCGA
841   ---------+---------+---------+---------+---------+---------+      900
      GGTGGCGGCGGCGGCGCAGATGGCGCGAGTGACCGCGGTGATGGCGACGCGGGTTCCGCT
       T  A  A  A  A  S  T  A  L  T  G  A  T  T  A  A  P  K  A  R

      GGCGCCGGGCGCACCTCCTGGCCACCCGCCGCGCCCTCGCCGCGCCCATCAGGTGCTCAG
901   ---------+---------+---------+---------+---------+---------+      960
      CCGCGGCCCGCGTGGAGGACCGGTGGGCGGCGCGGGAGCGGCGCGGGTAGTCCACGAGTC
        R  R  A  H  L  L  A  T  R  R  A  L  A  A  P  I  R  C  S  A

      CGGCGTCACCCGCCATGCCGATGGCTCCCCCGGCCACCCCGCTCCGGCCGTGGGGCCCCA
961   ---------+---------+---------+---------+---------+---------+     1020
      GCCGCAGTGGGCGGTACGGCTACCGAGGGGGCCGGTGGGGCGAGGCCGGCACCCCGGGGT
        A  S  P  A  M  P  M  A  P  P  A  T  P  L  R  P  W  G  P  T

      CCGATCCCCGCAAGGGCGCCGACATCCTCGTCGAGTCCCTCGAGCGCTGCGGCGTCCGCG
1021  ---------+---------+---------+---------+---------+---------+     1080
      GGCTAGGGGCGTTCCCGCGGCTGTAGGAGCAGCTCAGGGAGCTCGCGACGCCGCAGGCGC
        D  P  R  K  G  A  D  I  L  V  E  S  L  E  R  C  G  V  R  D

      ACGTCTTCGCCTACCCCGGCGGCGCGTCCATGGAGATCCACCAGGCACTCACCCGCTCCC
1081  ---------+---------+---------+---------+---------+---------+     1140
      TGCAGAAGCGGATGGGGCCGCCGCGCAGGTACCTCTAGGTGGTCCGTGAGTGGGCGAGGG
        V  F  A  Y  P  G  G  A  S  M  E  I  H  Q  A  L  T  R  S  P

      CCGTCATCCGCAACCACCTCTTCCGCCACGAGCAAGGGGAGGCCTTTGCGGCCTCCGGCT
1141  ---------+---------+---------+---------+---------+---------+     1200
      GGCAGTAGGCGTTGGTGGAGAAGGCGGTGCTCGTTCCCCTCCGGAAACGCCGGAGGCCGA
        V  I  R  N  H  L  F  R  H  E  Q  G  E  A  F  A  A  S  G  Y

      ACGCGCGCTCCTGGGCCGCCGTCGGCGTCTGCATCGCCACCTCCGGCCCCGGCGCCACCA
1201  ---------+---------+---------+---------+---------+---------+     1260
      TGCGCGCGAGGACCCGGCGGCAGCCGCAGACGTAGCGGTGGAGGCCGGGGCCGCGGTGGT
        A  R  S  W  A  A  V  G  V  C  I  A  T  S  G  P  G  A  T  N

      ACCTTGTCTCCGCGCTCGCCGACGCGCTGCTCGATTCCGTCCCCATGGTCGCCATCACGG
1261  ---------+---------+---------+---------+---------+---------+     1320
      TGGAACAGAGGCGCGAGCGGCTGCGCGACGAGCTAAGGCAGGGGTACCAGCGGTAGTGCC
        L  V  S  A  L  A  D  A  L  L  D  S  V  P  M  V  A  I  T  G

      GACAGGTGCCGCGACGCATGATTGGCACCGACGCCTTCCAGGAGACGCCCATCGTCGAGG
1321  ---------+---------+---------+---------+---------+---------+     1380
      CTGTCCACGGCGCTGCGTACTAACCGTGGCTGCGGAAGGTCCTCTGCGGGTAGCAGCTCC
        Q  V  P  R  R  M  I  G  T  D  A  F  Q  E  T  P  I  V  E  V

      TCACCCGCTCCATCACCAAGCACAACTACCTGGTCCTCGACGTCGACGACATCCCCCGGC
1381  ---------+---------+---------+---------+---------+---------+     1440
      AGTGGGCGAGGTAGTGGTTCGTGTTGATGGACCAGGAGCTGCAGCTGCTGTAGGGGGCCG
        T  R  S  I  T  K  H  N  Y  L  V  L  D  V  D  D  I  P  R  L
```

## FIG. 9 (b)

```
      TCGTGCAGGAGGCTTTCTTCCTCGCCTCCTCTGGTCGACCGGGGCCGGTGCTTGTCGACA
1441  ---------+---------+---------+---------+---------+---------+  1500
      AGCACGTCCTCCGAAAGAAGGAGCGGAGGAGACCAGCTGGCCCCGGCCACGAACAGCTGT
       V  Q  E  A  F  F  L  A  S  S  G  R  P  G  P  V  L  V  D  I

      TCCCCAAGGACATCCAGCAGCAGATGGCGGTGCCTGTCTGGGACAAGCCCATGAGTCTGC
1501  ---------+---------+---------+---------+---------+---------+  1560
      AGGGGTTCCTGTAGGTCGTCGTCTACCGCCACGGACAGACCCTGTTCGGGTACTCAGACG
        P  K  D  I  Q  Q  Q  M  A  V  P  V  W  D  K  P  M  S  L

      CTGGGTACATTGCGCGCCTTCCCAAGCCCCCTGCGACTGAGTTGCTTGAGCAGGTGCTGC
1561  ---------+---------+---------+---------+---------+---------+  1620
      GACCCATGTAACGCGCGGAAGGGTTCGGGGGACGCTGACTCAACGAACTCGTCCACGACG
       G  Y  I  A  R  L  P  K  P  P  A  T  E  L  L  E  Q  V  L  R

      GTCTTGTTGGTGAATCCCGGCGCCCTGTTCTTTATGTTGGCGGTGGCTGCGCAGCATCTG
1621  ---------+---------+---------+---------+---------+---------+  1680
      CAGAACAACCACTTAGGGCCGCGGGACAAGAAATACAACCGCCACCGACGCGTCGTAGAC
       L  V  G  E  S  R  R  P  V  L  Y  V  G  G  C  A  A  S  G

      GTGAGGAGTTGCGACGCTTTGTGGAGCTGACTGGAATCCCGGTCACAACTACTCTTATGG
1681  ---------+---------+---------+---------+--------+---------+  1740
      CACTCCTCAACGCTGCGAAACACCTCGACTGACCTTAGGGCCAGTGTTGATGAGAATACC
       E  E  L  R  R  F  V  E  L  T  G  I  P  V  T  T  T  L  M  G

      GCCTCGGCAACTTCCCCAGCGACGACCCACTGTCTCTGCGCATGCTAGGTATGCATGGCA
1741  ---------+---------+---------+---------+---------+---------+  1800
      CGGAGCCGTTGAAGGGGTCGCTGCTGGGTGACAGAGACGCGTACGATCCATACGTACCGT
       L  G  N  F  P  S  D  D  P  L  S  L  R  M  L  G  M  H  G  T

      CGGTGTATGCAAATTATGCAGTGGATAAGGCCGATCTGTTGCTTGCACTTGGTGTGCGGT
1801  ---------+---------+---------+---------+---------+---------+  1860
      GCCACATACGTTTAATACGTCACCTATTCCGGCTAGACAACGAACGTGAACCACACGCCA
        V  Y  A  N  Y  A  V  D  K  A  D  L  L  L  A  L  G  V  R  F

      TTGATGATCGTGTGACAGGGAAGATTGAGGCTTTTGCAAGCAGGGCTAAGATTGTGCACG
1861  ---------+---------+---------+---------+---------+---------+  1920
      AACTACTAGCACACTGTCCCTTCTAACTCCGAAAACGTTCGTCCCGATTCTAACACGTGC
       D  D  R  V  T  G  K  I  E  A  F  A  S  R  A  K  I  V  H  V

      TTGATATTGATCCGGCTGAGATTGGCAAGAACAAGCAGCCACATGTGTCCATCTGTGCAG
1921  ---------+---------+---------+---------+---------+--------.--+  1980
      AACTATAACTAGGCCGACTCTAACCGTTCTTGTTCGTCGGTGTACACAGGTAGACACGTC
        D  I  D  P  A  E  I  G  K  N  K  Q  P  H  V  S  I  C  A  D

      ATGTTAAGCTTGCTTTGCAGGGCATGAATGCTCTTCTTGAAGGAAGCACATCAAAGAAGA
1981  ---------+---------+---------+---------+---------+--------+  2040
      TACAATTCGAACGAAACGTCCCGTACTTACGAGAAGAACTTCCTTCGTGTAGTTTCTTCT
       V  K  L  A  L  Q  G  M  N  A  L  L  E  G  S  T  S  K  K  S
```

## FIG. 9 (c)

```
        GCTTTGACTTTGGCTCATGGAACGATGAGTTGGATCAGCAGAAGAGGGAATTCCCCCTTG
        ---------+---------+---------+---------+---------+---------+
        CGAAACTGAAACCGAGTACCTTGCTACTCAACCTAGTCGTCTTCTCCCTTAAGGGGGAAC
         F  D  F  G  S  W  N  D  E  L  D  Q  Q  K  R  E  F  P  L  G

        GGTATAAACATCTAATGAGGAGATCCAGCCACAATATGCTATTCAGGTTCTTGATGAGC
        ---------+---------+---------+---------+---------+---------+
        CCATATTTGTAGATTACTCCTCTAGGTCGGTGTTATACGATAAGTCCAAGAACTACTCG
         Y  K  T  S  N  E  E  I  Q  P  Q  Y  A  I  Q  V  L  D  E  L

        TGACGAAAGGCGAGGCCATCATCGGCACAGGTGTTGGGCAGCACCAGATGTGGGCGGCAC
        ---------+---------+---------+---------+---------+---------+
        ACTGCTTTCCGCTCCGGTAGTAGCCGTGTCCACAACCCGTCGTGGTCTACACCCGCCGTG
         T  K  G  E  A  I  I  G  T  G  V  G  Q  H  Q  M  W  A  A  Q

        AGTACTACACTTACAAGCGGCCAAGGCAGTGGTTGTCTTCAGCTGGTCTTGGGGCTATGG
        ---------+---------+---------+---------+---------+---------+
        TCATGATGTGAATGTTCGCCGGTTCCGTCACCAACAGAAGTCGACCAGAACCCCGATACC
         Y  Y  T  Y  K  R  P  R  Q  W  L  S  S  A  G  L  G  A  M  G

        GATTTGGTTTGCCGGCTGCTGCTGGTGCTTCTGTGGCCAACCCAGGTGTTACTGTTGTTG
        ---------+---------+---------+---------+---------+---------+
        CTAAACCAAACGGCCGACGACGACCACGAAGACACCGGTTGGGTCCACAATGACAACAAC
         F  G  L  P  A  A  A  G  A  S  V  A  N  P  G  V  T  V  V  D

        ACATCGATGGAGATGGTAGCTTTCTCATGAACGTTCAGGAGCTAGCTATGATCCGAATTG
        ---------+---------+---------+---------+---------+---------+
        TGTAGCTACCTCTACCATCGAAAGAGTACTTGCAAGTCCTCGATCGATACTAGGCTTAAC
         I  D  G  D  G  S  F  L  M  N  V  Q  E  L  A  M  I  R  I  E

        AGAACCTCCCGGTGAAGGTCTTTGTGCTAAACAACCAGCACCTGGGGATGGTGGTGCAGT
        ---------+---------+---------+---------+---------+---------+
        TCTTGGAGGGCCACTTCCAGAAACACGATTTGTTGGTCGTGGACCCCTACCACCACGTCA
         N  L  P  V  K  V  F  V  L  N  N  Q  H  L  G  M  V  V  Q  W

        GGGAGGACAGGTTCTATAAGGCCAACAGAGCGCACACATACTTGGGAAACCCAGAGAATG
        ---------+---------+---------+---------+---------+---------+
        CCCTCCTGTCCAAGATATTCCGGTTGTCTCGCGTGTGTATGAACCCTTTGGGTCTCTTAC
         E  D  R  F  Y  K  A  N  R  A  H  T  Y  L  G  N  P  E  N  E

        AAAGTGAGATATATCCAGATTTCGTGACGATCGCCAAAGGGTTCAACATTCCAGCGGTCC
        ---------+---------+---------+---------+---------+---------+
        TTTCACTCTATATAGGTCTAAAGCACTGCTAGCGGTTTCCCAAGTTGTAAGGTCGCCAGG
         S  E  I  Y  P  D  F  V  T  I  A  K  G  F  N  I  P  A  V  R

        GTGTGACAAAGAAGAACGAAGTCCGCGCAGCGATAAAGAAGATGCTCGAGACTCCAGGGC
        ---------+---------+---------+---------+---------+---------+
        CACACTGTTTCTTCTTGCTTCAGGCGCGTCGCTATTTCTTCTACGAGCTCTGAGGTCCCG
         V  T  K  K  N  E  V  R  A  A  I  K  K  M  L  E  T  P  G  P

        CGTACCTCTTGGATATAATCGTCCCACACCAGGAGCATGTGTTGCCTATGATCCCTAGTG
        ---------+---------+---------+---------+---------+---------+
        GCATGGAGAACCTATATTAGCAGGGTGTGGTCCTCGTACACAACGGATACTAGGGATCAC
         Y  L  L  D  I  I  V  P  H  Q  E  H  V  L  P  M  I  P  S  G
```

<div align="center">

## FIG. 9 (d)

</div>

```
      GTGGGGCTTTCAAGGATATGATCCTGGATGGTGATGGCAGGACTGTGTACTGATCTAAAA
2701  ---------+---------+---------+---------+---------+---------+  2760
      CACCCCGAAAGTTCCTATACTAGGACCTACCACTACCGTCCTGACACATGACTAGATTTT
       G   A   F   K   D   M   I   L   D   G   D   G   R   T   V   Y   *

      TCCAGCAAGCAACTGATCTAAAATCCAGCAAGCACCGCCTCCCTGCTAGTACAAGGGTGA
2761  ---------+---------+---------+---------+---------+---------+  2820
      AGGTCGTTCGTTGACTAGATTTTAGGTCGTTCGTGGCGGAGGGACGATCATGTTCCCACT

      TATGTTTTTATCTGTGTGATGTTCTCCTGTATTCTATCTTTTTTTGTAGGCCGTCAGCTA
2821  ---------+---------+---------+---------+---------+---------+  2880
      ATACAAAAATAGACACACTACAAGAGGACATAAGATAGAAAAAAACATCCGGCAGTCGAT

      TCTGTTATGGTAATCCTATGTAGCTTCCGACCTTGTAATTGTGTAGTCTGTTGTTTTCCT
2881  ---------+---------+---------+---------+---------+---------+  2940
      AGACAATACCATTAGGATACATCGAAGGCTGGAACATTAACACATCAGACAACAAAAGGA

      TCTGGCATGTGTCATAAGAGATCATTT
2941  ---------+---------+-------  2967
      AGACCGTACACAGTATTCTCTAGTAAA
```

# FIG. 9 (e)

```
      CCGTAAATCTCTTCCACGCACTCTGTCGTGTACCAACGTGCAGTGGAAACGCTCACGTAC
   1  ---------+---------+---------+---------+---------+---------+   60
      GGCATTTAGAGAAGGTGCGTGAGACAGCACATGGTTGCACGTCACCTTTGCGAGTGCATG

      CTTTGTGTATTATGTACGGATTCGGGCAACGGACATTTCGACGTCGGTTTGCGAGTCCCA
  61  ---------+---------+---------+---------+---------+---------+   120
      GAAACACATAATACATGCCTAAGCCCGTTGCCTGTAAAGCTGCAGCCAAACGCTCAGGGT

      TTCCCATCTGAACCACACATCTCTGAACAAAAGTAGGGGAGGCGCCCGGTAGCCCCCTTT
 121  ---------+---------+---------+---------+---------+---------+   180
      AAGGGTAGACTTGGTGTGTAGAGACTTGTTTTCATCCCCTCCGCGGGCCATCGGGGGAAA

      CCCACAATCCCACTCCGTGCCAGGTGCCACCCTCCCCAAGCCCTCGCGCCGCCTCCGAGA
 181  ---------+---------+---------+---------+---------+---------+   240
      GGGTGTTAGGGTGAGGCACGGTCCACGGTGGGAGGGGTTCGGGAGCGCGGCGGAGGCTCT

      CAGCCGCCCGCAACCATGGCCACCGCCGCCACCGCGGCGGCCGCGCTCACCGGCGCCACT
 241  ---------+---------+---------+---------+---------+---------+   300
      GTCGGCGGGCGTTGGTACCGGTGGCGGCGGTGGCGCCGCCGGCGCGAGTGGCCGCGGTGA
                   M   A   T   A   A   T   A   A   A   A   L   T   G   A   T

      ACCGCTACGCCCAAGTCGAGGCGCCGAGCCCACCACTTGGCCACCCGGCGCGCCCTCGCC
 301  ---------+---------+---------+---------+---------+---------+   360
      TGGCGATGCGGGTTCAGCTCCGCGGCTCGGGTGGTGAACCGGTGGGCCGCGCGGGAGCGG
       T   A   T   P   K   S   R   R   R   A   H   H   L   A   T   R   R   A   L   A

      GCGCCC;TCAGGTGCTCAGCGTTGTCACGCGCCACGCCGACGGCTCCCCCGGCCACTCCG
 361  ---------+---------+---------+---------+---------+---------+   420
      CGCGGGTAGTCCACGAGTCGCAACAGTGCGCGGTGCGGCTGCCGAGGGGGCCGGTGAGGC
       A   P   I   R   C   S   A   L   S   R   A   T   P   T   A   P   P   A   T   P

      CTACGTCCGTGGGGCCCCAACGAGCCCCGCAAGGGCTCCGACATCCTCGTCGAGGCTCTC
 421  ---------+---------+---------+---------+---------+---------+   480
      GATGCAGGCACCCCGGGGTTGCTCGGGGCGTTCCCGAGGCTGTAGGAGCAGCTCCGAGAG
       L   R   P   W   G   P   N   E   P   R   K   G   S   D   I   L   V   E   A   L

      GAGCGCTGTGGCGTCCGTGACGTCTTCGCCTACCCCGGCGGCGCATCCATGGAGATCCAC
 481  ---------+---------+---------+---------+---------+---------+   540
      CTCGCGACACCGCAGGCACTGCAGAAGCGGATGGGGCCGCCGCGTAGGTACCTCTAGGTG
       E   R   C   G   V   R   D   V   F   A   Y   P   G   G   A   S   M   E   I   H

      CAGGCACTCACCCGCTCCCCCGTCATCGCCAACCACCTCTTCCGCCACGAACAAGGGGAG
 541  ---------+---------+---------+---------+---------+---------+   600
      GTCCGTGAGTGGGCGAGGGGGCAGTAGCGGTTGGTGGAGAAGGCGGTGCTTGTTCCCCTC
       Q   A   L   T   R   S   P   V   I   A   N   H   L   F   R   H   E   Q   G   E

      GCCTTTGCGGCCTCCGGCTACGCGCGCTCCTGGGCCCGCGTTGGCGTCTGCATCGCCACC
 601  ---------+---------+---------+---------+---------+---------+   660
      CGGAAACGCCGGAGGCCGATGCGCGCGAGGACCCGGGCGCAACCGCAGACGTAGCGGTGG
       A   F   A   A   S   G   Y   A   R   S   W   A   R   V   G   V   C   I   A   T
```

# FIG. 10 (a)

```
          TCCGGCCCCGGCGCCACCAACCTAGTCTCTGCGCTCGCAGACGCGTTGCTCGACTCCGTC
          ---------+---------+---------+---------+---------+---------+          720
          AGGCCGGGGCCGCGGTGGTTGGATCAGAGACGCGAGCGTCTGCGCAACGAGCTGAGGCAG
           S  G  P  G  A  T  N  L  V  S  A  L  A  D  A  L  L  D  S  V

          CCCATTGTCGCCATCACGGGACAGGTGCCGCGACGCATGATTGGCACCGACGCCTTTCAG
          ---------+---------+---------+---------+---------+---------+          780
          GGGTAACAGCGGTAGTGCCCTGTCCACGGCGCTGCGTACTAACCGTGGCTGCGGAAAGTC
           P  I  V  A  I  T  G  Q  V  P  R  R  M  I  G  T  D  A  F  Q

          GAGACGCCCATCGTCGAGGTCACCCGCTCCATCACCAAGCACAACTACCTGGTCCTCGAC
          ---------+---------+---------+---------+---------+---------+          840
          CTCTGCGGGTAGCAGCTCCAGTGGGCGAGGTAGTGGTTCGTGTTGATGGACCAGGAGCTG
           E  T  P  I  V  E  V  T  R  S  I  T  K  H  N  Y  L  V  L  D

          GTCGACGACATCCCCCGCGTCGTGCAGGAGGCCTTCTTCCTCGCATCCTCTGGTCGCCCG
          ---------+---------+---------+---------+---------+---------+          900
          CAGCTGCTGTAGGGGGCGCAGCACGTCCTCCGGAAGAAGGAGCGTAGGAGACCAGCGGGC
           V  D  D  I  P  R  V  V  Q  E  A  F  F  L  A  S  S  G  R  P

          GGGCCGGTGCTTGTTGACATCCCCAAGGACATCCAGCAGCAGATGGCGGTGCCGGCCTGG
          ---------+---------+---------+---------+---------+---------+          960
          CCCGGCCACGAACAACTGTAGGGGTTCCTGTAGGTCGTCGTCTACCGCCACGGCCGGACC
           G  P  V  L  V  D  I  P  K  D  I  Q  Q  Q  M  A  V  P  A  W

          GACACGCCCATGAGTCTGCCTGGGTACATCGCGCGCCTTCCCAAGCCTCCCGCGACTGAA
          ---------+---------+---------+---------+---------+---------+          1020
          CTGTGCGGGTACTCAGACGGACCCATGTAGCGCGCGGAAGGGTTCGGAGGGCGCTGACTT
           D  T  P  M  S  L  P  G  Y  I  A  R  L  P  K  P  P  A  T  E

          TTTCTTGAGCAGGTGCTGCGTCTTGTTGGTGAATCACGGCGCCCTGTTCTTTATGTTGGC
          ---------+---------+---------+---------+---------+---------+          1080
          AAAGAACTCGTCCACGACGCAGAACAACCACTTAGTGCCGCGGGACAAGAAATACAACCG
           F  L  E  Q  V  L  R  L  V  G  E  S  R  R  P  V  L  Y  V  G

          GGTGGCTGTGCAGCATCAGGTGAGGAGTTGTGCCGCTTTGTGGAGTTGACTGGAATCCCA
          ---------+---------+---------+---------+---------+---------+          1140
          CCACCGACACGTCGTAGTCCACTCCTCAACACGGCGAAACACCTCAACTGACCTTAGGGT
           G  G  C  A  A  S  G  E  E  L  C  R  F  V  E  L  T  G  I  P

          GTCACAACTACTCTTATGGGCCTTGGCAACTTCCCCAGCGACGACCCACTGTCACTGCGC
          ---------+---------+---------+---------+---------+---------+          1200
          CAGTGTTGATGAGAATACCCGGAACCGTTGAAGGGGTCGCTGCTGGGTGACAGTGACGCG
           V  T  T  T  L  M  G  L  G  N  F  P  S  D  D  P  L  S  L  R

          ATGCTTGGTATGCATGGCACAGTGTATGCAAATTATGCAGTGGATAAGGCCGATCTGTTG
          ---------+---------+---------+---------+---------+---------+          1260
          TACGAACCATACGTACCGTGTCACATACGTTTAATACGTCACCTATTCCGGCTAGACAAC
           M  L  G  M  H  G  T  V  Y  A  N  Y  A  V  D  K  A  D  L  L

          CTTGCATTTGGTGTGCGGTTTGATGATCGTGTGACAGGGAAAATTGAGGCTTTTGCAGGC
          ---------+---------+---------+---------+---------+---------+          1320
          GAACGTAAACCACACGCCAAACTACTAGCACACTGTCCCTTTTAACTCCGAAAACGTCCG
           L  A  F  G  V  R  F  D  D  R  V  T  G  K  I  E  A  F  A  G
```

## FIG. 10 (b)

```
      AGAGCTAAGATTGTGCACATTGATATTGATCCTGCTGAGATTGGCAAGAACAAGCAGCCA
      ---------+---------+---------+---------+---------+---------+
      TCTCGATTCTAACACGTGTAACTATAACTAGGACGACTCTAACCGTTCTTGTTCGTCGGT
       R  A  K  I  V  H  I  D  I  D  P  A  E  I  G  K  N  K  Q  P

      CATGTGTCCATCTGTGCAGATGTTAAGCTTGCTTTGCAGGGCATGAATACTCTTCTGGAA
      ---------+---------+---------+---------+---------+---------+
      GTACACAGGTAGACACGTCTACAATTCGAACGAAACGTCCCGTACTTATGAGAAGACCTT
       H  V  S  I  C  A  D  V  K  L  A  L  Q  G  M  N  T  L  L  E

      GGAAGCACATCAAAGAAGAGCTTTGACTTCGGCTCATGGCATGATGAATTGGATCAGCAA
      ---------+---------+---------+---------+---------+---------+
      CCTTCGTGTAGTTTCTTCTCGAAACTGAAGCCGAGTACCGTACTACTTAACCTAGTCGTT
       G  S  T  S  K  K  S  F  D  F  G  S  W  H  D  E  L  D  Q  Q

      AAGCGGGAGTTTCCCCTTGGGTATAAAATCTTCAATGAGGAAATCCAGCCACAATATGCT
      ---------+---------+---------+---------+---------+---------+
      TTCGCCCTCAAAGGGGAACCCATATTTTAGAAGTTACTCCTTTAGGTCGGTGTTATACGA
       K  R  E  F  P  L  G  Y  K  I  F  N  E  E  I  Q  P  Q  Y  A

      ATTCAGGTTCTTGATGAGTTGACGAAGGGGAAGGCCATCATTGCCACAGGTGTTGGGCAG
      ---------+---------+---------+---------+---------+---------+
      TAAGTCCAAGAACTACTCAACTGCTTCCCCTTCCGGTAGTAACGGTGTCCACAACCCGTC
       I  Q  V  L  D  E  L  T  K  G  K  A  I  I  A  T  G  V  G  Q

      CACCAGATGTGGGCGGCACAGTATTACACTTACAAGCGGCCAAGGCAGTGGCTGTCTTCA
      ---------+---------+---------+---------+---------+---------+
      GTGGTCTACACCCGCCGTGTCATAATGTGAATGTTCGCCGGTTCCGTCACCGACAGAAGT
       H  Q  M  W  A  A  Q  Y  Y  T  Y  K  R  P  R  Q  W  L  S  S

      GCTGGTCTTGGGGCTATGGGATTTGGTTTGCCGGCTGCTGCTGGTGCTGCTGTGGCCAAC
      ---------+---------+---------+---------+---------+---------+
      CGACCAGAACCCCGATACCCTAAACCAAACGGCCGACGACGACCACGACGACACCGGTTG
       A  G  L  G  A  M  G  F  G  L  P  A  A  A  G  A  A  V  A  N

      CCAGGTGTCACTGTTGTTGACATCGACGGAGATGGTAGCTTCCTCATGAACATTCAGGAG
      ---------+---------+---------+---------+---------+---------+
      GGTCCACAGTGACAACAACTGTAGCTGCCTCTACCATCGAAGGAGTACTTGTAAGTCCTC
       P  G  V  T  V  V  D  I  D  G  D  G  S  F  L  M  N  I  Q  E

      CTAGCTATGATCCGTATTGAGAACCTCCCAGTCAAGGTCTTTGTGCTAAACAACCAGCAC
      ---------+---------+---------+---------+---------+---------+
      GATCGATACTAGGCATAACTCTTGGAGGGTCAGTTCCAGAAACACGATTTGTTGGTCGTG
       L  A  M  I  R  I  E  N  L  P  V  K  V  F  V  L  N  N  Q  H

      CTCGGGATGGTGGTGCAGTGGGAGGACAGGTTCTATAAGGCCAATAGAGCACACACATTC
      ---------+---------+---------+---------+---------+---------+
      GAGCCCTACCACCACGTCACCCTCCTGTCCAAGATATTCCGGTTATCTCGTGTGTGTAAG
       L  G  M  V  V  Q  W  E  D  R  F  Y  K  A  N  R  A  H  T  F
```

## FIG. 10 (c)

```
        TTGGGAAACCCAGAGAACGAAAGTGAGATATATCCAGATTTTGTGGCAATTGCCAAAGGG
        ---------+---------+---------+---------+---------+---------+         1980
        AACCCTTTGGGTCTCTTGCTTTCACTCTATATAGGTCTAAAACACCGTTAACGGTTTCCC
        L  G  N  P  E  N  E  S  E  I  Y  P  D  F  V  A  I  A  K  G

        TTCAACATTCCAGCAGTCCGTGTGACAAAGAAGAGCGAAGTCCATGCAGCAATCAAGAAG
        ---------+---------+---------+---------+---------+---------+         2040
        AAGTTGTAAGGTCGTCAGGCACACTGTTTCTTCTCGCTTCAGGTACGTCGTTAGTTCTTC
        F  N  I  P  A  V  R  V  T  K  K  S  E  V  H  A  A  I  K  K

        ATGCTTGAGGCTCCAGGGCCGTACCTCTTGGATATAATCGTCCCGCACCAGGAGCATGTG
        ---------+---------+---------+---------+---------+---------+         2100
        TACGAACTCCGAGGTCCCGGCATGGAGAACCTATATTAGCAGGGCGTGGTCCTCGTACAC
        M  L  E  A  P  G  P  Y  L  L  D  I  I  V  P  H  Q  E  H  V

        TTGCCTATGATCCCTAGTGGTGGGGCTTTCAAGGATATGATCCTGGATGGTGATGGCAGG
        ---------+---------+---------+---------+---------+---------+         2160
        AACGGATACTAGGGATCACCACCCCGAAAGTTCCTATACTAGGACCTACCACTACCGTCC
        L  P  M  I  P  S  G  G  A  F  K  D  M  I  L  D  G  D  G  R

        ACTGTGTATTGATCTAAAGTTCAGCAAGCACTGCCTACCTGCCTATCTTTGACATGCATG
        ---------+---------+---------+---------+---------+---------+         2220
        TGACACATAACTAGATTTCAAGTCGTTCGTGACGGATGGACGGATAGAAACTGTACGTAC
        T  V  Y  *

        AGCTAGTACAAGAGAGATATGTTTTTATCAATGTGATGGTACTCTGTTATGGTAATCTTA
        ---------+---------+---------+---------+---------+---------+         2280
        TCGATCATGTTCTCTCTATACAAAAATAGTTACACTACCATGAGACAATACCATTAGAAT

        AGTAGCATCCAACCCTGTGTGTAGTATGTTGTTTTCGTGTTGGCATATGTTTCAGAAGCC
        ---------+---------+---------+---------+---------+---------+         2340
        TCATCGTAGGTTGGGACACACATCATACAACAAAAGCACAACCGTATACAAAGTCTTCGG

        ATCATGTAAGTGCCTTTTACTACATATAAATAAGGTAATAAGCATTGTTATGCACCGGTT
        ---------+---------+---------+---------+---------+---------+         2400
        TAGTACATTCACGGAAAATGATGTATATTTATTCCATTATTCGTAACAATACGTGGCCAA

        CTGAATTGGTCTTCTTTTGCCAAATATAGGTCCTGTTTGATACCTATAGCTCTAGAAAAT
        ---------+---------+---------+---------+---------+---------+         2460
        GACTTAACCAGAAGAAACGGTTTATATCCAGGACAAACTATGGATATCGAGATCTTTTA

        TTGGTGTGGTTGGTGGAGCAGGCCATTAGGTGTTCCATAAAATAGTGGAGTTGTAAGACT
        ---------+---------+---------+---------+---------+---------+         2520
        AACCACACCAACCACCTCGTCCGGTAATCCACAAGGTATTTATCACCTCAACATTCTGA

        TTAGAAGACATTTAGATAAATTATTT
        ---------+---------+------   2546
        AATCTTCTGTAAATCTATTTAATAAA
```

## FIG. 10 (d)

```
          10            30            50            70            90           110
CTCGAGGAATTCAAACTCTTAGCTTCACTAAACTTGAGCTTCTTTTCCACTAAGTCGAAATAAACATAAGCTATTTACAAAAATAAAAAAATACTCCATTGAATCTAAA

         130           150           170           190          .210           230
GTCAAGTCGTGATTGGAATAGAAAATAGAAATTTATTTATATCCAGATCAAGCCGTGATTGGAATGAAAGTATGATAGTAGTAACATCAAGTTGGAAAATTAAG

         250           270           290           310           330           350
GGAAAGGAAATTAGAGAAAGAAACTGAAGAATATCCAAATATCTTTACGTCCAAATTGATAGTTATTTAACGTCATCGAGATGACGGCCATGTTCAAGTTTCCACAAATATTGAGAAAA

         370           390           410           430           450           470
GAAAGAAGAAGACAAACTGTGTTGGTATTATTATATAGTGTTTTTCTTTAGAGAATTGATTGTACATATAGAAATATATAAGATTTAGAAATAGAATTATTAGAAAAATCAAACA

         490           510           530           550           570           590
TCAAAGTAATTATTTTAAATCTTTTTCCAAATGGACAATTCCCATTCTGAAAAAAAGAGATATAAATAATCAGATCGTTAAAGTGAAAATGTAAAATATTAATTAACACA

         610           630           650           670           690           710
TTAACCATAACCAGTCTACTTATTTAACAAAAGCACATCTGATAGATCAAAAAGTGTTTAACTTCATGCATTGACAATTTAAAATTATTTGCAACATCGGGTAAAACTATTTTACA

         730           750           770           790           810           830
ACAATTGGTAACTGCATATATAAGTTTAATATGGTAACCTAGAAAAATAGGATAAATTATCTATAACAGGATATATTACCATTGATATTTAGTAGTAACATGAAATA

         850           870           890           910           930           950
ATCACCGTGAAATCTTCAAGATTCTCCATAAATACCCTTGGTAGTAAATCTAGTTTTTCCTTCAAGATAACAACATTCTCCTATAGTCATGGATTGTTCTCTTTCACAATTGCC
                                                                      MetGlyPheValLeuPheSerGlnLeuPr

         970           990          1010          1030          1050          1070
TTCATTCTCTGTCTCTACACTTCTTATTCCTAGTAATATCCTAGTAATATCCAACAAATTCTCAACAAGACTATTGGATGCCCATAACAGCTGCTGTGCGAGTAGGTGT
oSerPheLeuLeuValSerThrLeuLeuPheLeuValIleSerHisSerCysArgAlaHisAsnThrAlaArgAlaAspValGlyVa
```

## FIG. 11 (a)

AGAACCTTTGACCTGGAACGACCAGGTAGCAGCCTATGCGCCAAAATTATGCTTCCCAATTGGCTGCGAGATTGTAACCTGTACATTCTCGTACATTCTCATGGTCAATACGGCGCAAAACCTAGCTGAGGG
lGluProLeuThrTrpAspAspGlnValAlaAlaTyrAlaGlnAsnTyrAlaSerGlnLeuValHisSerHisGlyGlnTyrGlyGluAsnLeuAlaGlu Gl

AAGTGGCGATTTCATGACGGCTGCTAGGGCTGTTGAGAATGGGTGAGATGAAACAGTATTATGACCATGACTCAAATACTGTGCAAGGACAGTGGTGTTGGACACTATACTCAGT
ySerGlyAspPheMetThrAlaAlaArgAlaValAlaLysAlaValGluMetTrpProValAspGluLysGlnTyrTyrAspHisAspSerAsnThrCysAlaGlnGlyGlnValCysGlyHisTyrThrGlnVa

GGTTGGCGTAACTCGGTTCGTGTTGGAATGTGCTAGGTGTCAGTGTAACAATGGAGGATATGTTGTCTGTCTTGCAACTATGATCCTCCAGTAATTATGAGGGCGAAGTCCATACTAATT
lValTrpArgAsnSerValArgValGlyCysAlaArgValGlyTyrValValSerCysAsnAsnGlyGlyTyrValValCysLeuAlaThrMetIleLeuGlnSerProTyrEnd

GAAACGACCTAGTGTCCATTCAGTTAATATGTATGGAATGTTCTGCTGAATCAGAACTAAATAATGCTCTAAAAGCAACTAAAGTCAAGTATACAGTTAATAGTACTATATTT

GTAATCCCTCGAAGTGGAATCTATAAAAAGACCAAGTGTGTCATTAATTAAGGGGGAAAAATATGAGTTGATGATCTGATGTATGAATCTGATAATTATTGAAACACTTTGTACTCATAC

GAATCAGTGTGTGAATGGTCTACTGCTCTGGCGATATTACGAGCAAAATTCTTAACTACACTACATGCCTTAGGAACAAGCTTACACAGTTCATATAATAATCTACTGAGGGCCAAAACATGAAAATT

ACCAATTTAGAGGTAGGAGGAGGGATTGAAAGTGGGAGCAGCTAGTTTTAAAAACTGACCGTAGTTAAAATTATTTACATAATCAGGTCATTTATATGGTAATTA

TAGGTAAATATTTATGACGAAATTCTCAATGTAATCTGAAAAAAAAATTGTAACTAACCTATATACTAAAACTACTATAATAGGTTAGATTACATTAATCATGTCATTAGAAGATCTT

FIG. 11 (b)